# EUROPEAN PATENT APPLICATION

(11) **EP 4 810 033 A1**
(43) Date of publication of application: **23.09.2026**
(21) Application number: 26166512.9
(22) Date of filing: 20.03.2026
(51) Int. Cl.: A61K 31/341, A23L 33/10, A61K 31/357, A61K 31/443, A61P 31/00, A61P 31/04, C07D 307/52

(54) **PHARMACEUTICAL COMPOSITION FOR PREVENTING OR TREATING SEPSIS CONTAINING AN OLFACTORY RECEPTOR OLFR164 MODULATOR AS AN ACTIVE INGREDIENT**

(30) Priority: 20.03.2025 KR 20250036141; 24.02.2026 KR 20260034036
(71) Applicant: Daegu Gyeongbuk Institute of Science and Technology, Daegu 42988 (KR); Research & Business Foundation Sungkyunkwan University, Gyeonggi-do 16419 (KR); Korea University Research and Business Foundation, Sejong Campus, Sejong-si 30019 (KR)
(72) Inventor: BAE, Yoe Sik, 13597 Seongnam-si (KR); JEONG, Yu Sun, 35246 Daejeon (KR); PARK, Ji Ye, 16418 Suwon-si (KR); KOO, Jaehyung, 42988 Hyeonpung-eup (KR); CHOI, JiWoo, 42988 Hyeonpung-eup (KR); BYUN, Youngjoo, 34049 Daejeon (KR); SHIN, Myeongsu, 30019 Sejong-si (KR); LYU, Kanghyun, 18478 Hwaseong-si (KR); JO, Hwangi, 30021 Sejong-si (KR)
(74) Representative: Mewburn Ellis LLP

(57) **Abstract**

The present disclosure relates to a pharmaceutical composition for preventing or treating sepsis comprising an odorant receptor Olfr164 modulator as an active ingredient, and by synthesizing a derivative that modulates the odorant receptor Olfr164 and confirming the activity of the derivative in cell lines stably expressing the target odorant receptor Olfr164 and in a sepsis animal model, the potential for use as a therapeutic agent for sepsis is demonstrated.

## Description

### [Technical Field]

The present disclosure relates to a pharmaceutical composition for preventing or treating sepsis including an odorant receptor Olfr164 modulator as an active ingredient.

### [Background of the Invention]

Sepsis is a severe acute infectious inflammatory disease in which an immune response is initiated by pathogens invading the body, leading to the excessive secretion of inflammatory cytokines and the induction of a systemic inflammatory response, which ultimately results in the paralysis of the body's defense ability against infectious agents due to tissue and organ damage and loss of leukocyte function. Among these, *Pseudomonas aeruginosa* is one of the representative pathogens that induce sepsis, and infection with *Pseudomonas aeruginosa* may cause pneumonia, intra-abdominal infections, postoperative wound infections, osteoarthritis, and soft tissue infections. *Pseudomonas aeruginosa* is also a representative opportunistic pathogen, and infection occurs when a patient's immunity is reduced.

Treatment for *Pseudomonas aeruginosa* known to date involves the administration of antibiotics based on the site of infection. While antibiotics such as polymyxin, levofloxacin, ciprofloxacin, and ceftazidime are commonly used, severe infections caused by *Pseudomonas aeruginosa* are difficult to treat. Furthermore, due to the increase in multidrug-resistant *Pseudomonas aeruginosa* strains resistant to many antibiotic classes (carbapenems, aminoglycosides, and fluoroquinolones), there is a growing need for control methods other than antibiotic treatment. As of 2017, multidrug-resistant *Pseudomonas aeruginosa* was reported to account for 14.7% of specimens collected from general hospitals and nursing hospitals nationwide. Due to the nature of *Pseudomonas aeruginosa*, there are limitations to antibiotic treatment because it rapidly acquires resistance even to susceptible antibiotics.

One of the most representative characteristics of *Pseudomonas aeruginosa* is its opportunistic nature, meaning it infects immunocompromised patients. Therefore, enhancing and supplementing immune function may be an effective strategy to combat *Pseudomonas aeruginosa* infection. Immune cells include innate immune cells such as neutrophils, macrophages, and dendritic cells, as well as adaptive immune cells such as B cells and T cells, however, innate immune cells play the most crucial role in the defense against *Pseudomonas aeruginosa.* Immune cells exhibit a defensive response against pathogens by recognizing various substances derived from the pathogens, and in addition to pattern-recognition receptors (PRRs) capable of recognizing pathogens, they may also recognize the external inflammatory environment and pathogens through G protein-coupled receptors (GPCRs), which primarily recognize small molecules. However, little is known about which GPCRs are activated and what functions are regulated by micrometabolites derived from pathogens.

In mammals, the odorant receptor (OR) is the largest receptor among GPCRs, accounting for more than 50% of the total. Recently, it has been reported that the various physiological functions are regulated by the odorant receptors in extra-nasal tissues. Furthermore, as there are reports that odorant receptors may recognize micrometabolites produced by various pathogens, odorant receptors could be a novel therapeutic targets for future drug development.

### [Prior-Art Documents]

### [Patent Document]

Patent Document 1. Korean Patent Registration No. 10-1616872.

### [Summary of the Invention]

### [Problem to be Solved by the Invention]

An object of the present disclosure is to provide a pharmaceutical composition for preventing or treating sepsis.

Another object of the present disclosure is to provide a health functional food composition for preventing or improving sepsis.

Another object of the present disclosure is to provide a reagent composition for modulating an odorant receptor Olfr164.

### [Means for Solving the Problem]

In order to achieve the above objects, the present disclosure provides a pharmaceutical composition for preventing or treating sepsis including, as an active ingredient, a compound selected from a compound represented by Chemical Formula 1 below, a pharmaceutically acceptable salt thereof, a solvate thereof, or a stereoisomer thereof: 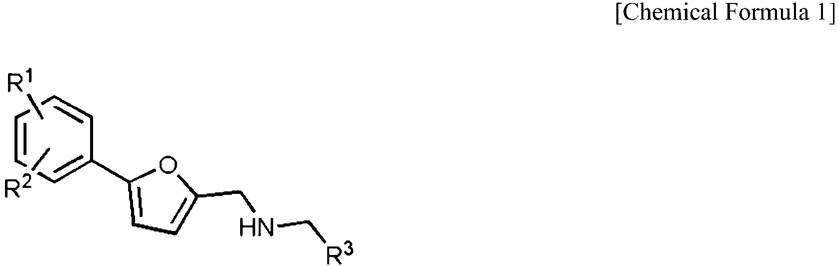 wherein, in Chemical Formula 1,
R¹ and R² may be the same or different, and each independently selected from the group consisting of hydrogen, hydroxy (OH), halogen, (C1~C4)alkyl, (C1~C4)alkoxy, acetyl, 1-hydroxyethyl (-CH(OH)CH₃) and trifluoromethyl (CF₃),
R³ is selected from the group consisting of 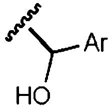 and substituted or unsubstituted phenyl, wherein the substituted phenyl may be substituted with any one selected from the group consisting of halogen, hydroxy (OH), (C1~C4)alkyl, (C1~C4)alkoxy, and trifluoromethyl (CF₃), and

Ar is selected from substituted or unsubstituted phenyl, pyridinyl, 1,3-benzodioxol, or naphthyl, wherein the substituted phenyl may be substituted with one or two substituents selected from the group consisting of halogen, (C1~C4)alkyl, (C1~C4)alkoxy, and trifluoromethyl.

In addition, the present disclosure provides a health functional food composition for preventing or improving sepsis including, as an active ingredient, a compound selected from a compound represented by Chemical Formula 1 below, a pharmaceutically acceptable salt thereof, a solvate thereof, or a stereoisomer thereof: 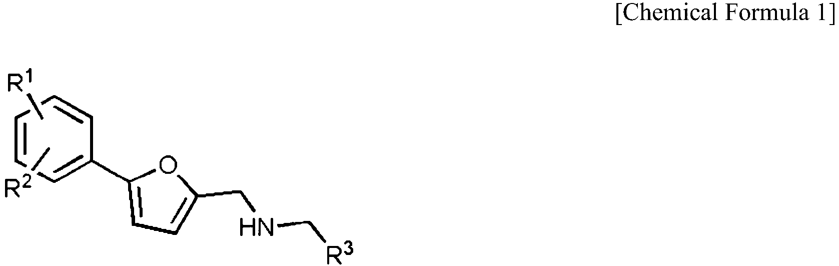 wherein, in Chemical Formula 1,
R¹ and R² may be the same or different, and each independently selected from the group consisting of hydrogen, hydroxy (OH), halogen, (C1~C4)alkyl, (C1~C4)alkoxy, acetyl, 1-hydroxyethyl (-CH(OH)CH₃) and trifluoromethyl (CF₃),
R³ is selected from the group consisting of 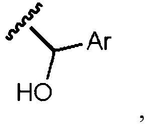 and substituted or unsubstituted phenyl, wherein the substituted phenyl may be substituted with any one selected from the group consisting of halogen, hydroxy (OH), (C1~C4)alkyl, (C1~C4)alkoxy, and trifluoromethyl (CF₃), and

Ar is selected from substituted or unsubstituted phenyl, pyridinyl, 1,3-benzodioxol, or naphthyl, wherein the substituted phenyl may be substituted with one or two substituents selected from the group consisting of halogen, (C1~C4)alkyl, (C1~C4)alkoxy, and trifluoromethyl.

In addition, the present disclosure provides a reagent composition for modulating an odorant receptor Olfr164 including, as an active ingredient, a compound selected from a compound represented by Chemical Formula 1 below, a pharmaceutically acceptable salt thereof, a solvate thereof, or a stereoisomer thereof: 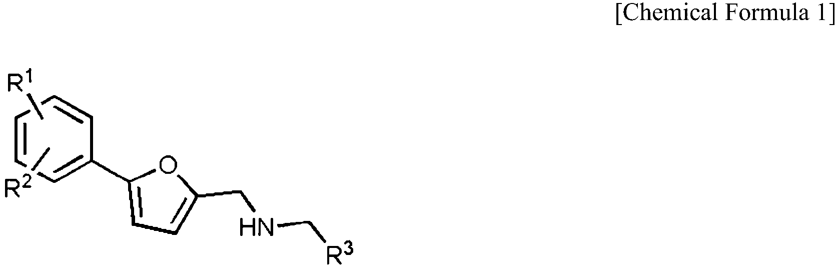 wherein, in Chemical Formula 1,
R¹ and R² may be the same or different, and each independently selected from the group consisting of hydrogen, hydroxy (OH), halogen, (C1~C4)alkyl, (C1~C4)alkoxy, acetyl, 1-hydroxyethyl (-CH(OH)CH₃) and trifluoromethyl (CF₃),
R³ is selected from the group consisting of 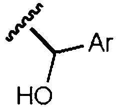 and substituted or unsubstituted phenyl, wherein the substituted phenyl may be substituted with any one selected from the group consisting of halogen, hydroxy (OH), (C1~C4)alkyl, (C1~C4)alkoxy, and trifluoromethyl (CF₃), and

Ar is selected from substituted or unsubstituted phenyl, pyridinyl, 1,3-benzodioxol, or naphthyl, wherein the substituted phenyl may be substituted with one or two substituents selected from the group consisting of halogen, (C1~C4)alkyl, (C1~C4)alkoxy, and trifluoromethyl.

### [Effects of the Invention]

The present disclosure relates to a pharmaceutical composition for preventing or treating sepsis including an odorant receptor Olfr164 modulator as an active ingredient, and by synthesizing a derivative that modulates the odorant receptor Olfr164 and confirming the activity of the derivative in cell lines stably expressing the target odorant receptor Olfr164 and in a sepsis animal model, the potential for use as a therapeutic agent for sepsis is demonstrated.

### [Brief Description of the Drawings]

FIG. 1 is a diagram illustrating an experimental process for establishing a cell line that stably expresses a target odorant receptor.
FIG. 2 shows target receptor expression levels identified through FACS.
FIG. 3 shows the activities of a cAMP sensor obtained through Fold change (Fsk/DMSO) values of selected cell lines.
FIG. 4 shows the features of selected cell lines and the representative images of surface expression via immunofluorescence staining.
FIG. 5 shows a process of screening a target odorant receptor's ligand and the results of primary screening.
FIG. 6 shows the results of a second screening via cAMP assay
FIG. 7 shows compounds that exhibit a target odorant receptor-specific response after third screening.
FIG. 8 is a set of graphs showing the activity of target odorant receptor Olfr164 against to KB45XX derivatives.
FIG. 9 is a set of graphs comparing activity of KB45XX derivatives and compound 55H05 selected through screening.
FIG. 10 shows results of measuring whether calcium ions in neutrophils increased due to KB45XX derivatives.
FIG. 11 shows results of measuring calcium ions by KB45XX derivatives in neutrophils and low-density bone marrow cells isolated from mouse bone marrow.
FIG. 12 is a set of graphs analyzing an effect of KB4501 on neutrophil chemotaxis.
FIG. 13 is a set of graphs analyzing an inflammatory cytokine inhibitory effect.
FIG. 14 is a set of graphs analyzing an inflammatory cytokine inhibitory effect of KB4501.
FIG. 15 is a set of graphs analyzing an inhibitory effect of KB45XX derivatives (KB4501, KB4502 and KB4528) on LPS-induced inflammatory cytokines and chemokines.
FIG. 16 is a set of graphs showing a plasmacytoid dendritic cells (pDC) cytokine production modulatory effect of KB45XX derivatives (KB4527, KB4528 and KB4533).
FIG. 17 is a set of graphs analyzing an effect of KB45XX derivatives (KB4501, KB4502 and KB4528) on neutrophil antigen phagocytosis.
FIG. 18 is a set of graphs analyzing a modulatory effect of KB45XX derivatives (KB4501, KB4502 and KB4528) on reactive oxygen species generation.
FIG. 19 shows results confirming a modulatory effect of KB45XX derivatives (KB4501, KB4502 and KB4528) on NET formation.
FIG. 20 is a set of graphs analyzing a neutrophil degranulation effect of KB45XX derivatives (KB4501, KB4502 and KB4528).
FIG. 21 is a set of graphs showing a modulatory effect of KB45XX derivatives (KB4501, KB4502, KB4527, KB4528 and KB4533) on the expression of pDC surface proteins and transcription factors.
FIG. 22 is a set of graphs showing a sepsis treatment effect of KB4528 in a sepsis animal model.
FIG. 23 shows an organ damage inhibitory effect of KB4528 in a sepsis animal model.
FIG. 24 shows the results of measuring CFU by collecting intraperitoneal bacteria in a sepsis animal model administered with KB4528, confirming a bactericidal activity.
FIG. 25 is a set of graphs analyzing a modulatory effect of KB4528 on inflammatory cytokine production in a sepsis animal model.
FIG. 26 is a set of graphs analyzing an effect of KB4528 on neutrophil reactive oxygen species generation and mechanism thereof.
FIG. 27 shows a modulatory effect of KB4528 on neutrophil NET formation.
FIG. 28 shows the results of analyzing a neutrophil-increasing effect of KB4528 in a sepsis animal model.
FIG. 29 shows the results of analyzing an effect of KB4528 on increasing neutrophil maturation.
FIG. 30 is a set of graphs analyzing a modulatory effect of KB4528 on chemokine receptor expression in neutrophils in a sepsis animal model.
FIG. 31 shows analysis of activation of neutrophil autophagy by KB4528 in a sepsis animal model.
FIG. 32 shows the results of a toxicity evaluation through an analysis of body weight changes of KB45XX derivatives (KB4501, KB4528 and KB4529).
FIG. 33 shows the results of a toxicity assessment through an analysis of changes in major organs of KB45XX derivatives (KB4501, KB4528 and KB4529).

### [Detailed Description]

Hereinafter, the present disclosure will be described in more detail.

The present disclosure provides a pharmaceutical composition for preventing or treating sepsis including, as an active ingredient, a compound selected from a compound represented by Chemical Formula 1 below, a pharmaceutically acceptable salt thereof, a solvate thereof, or a stereoisomer thereof: 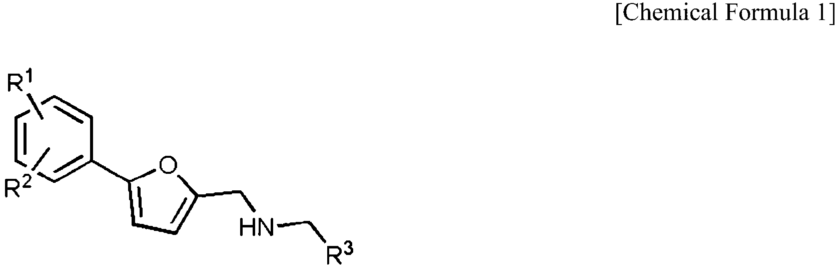 wherein, in Chemical Formula 1,
R¹ and R² may be the same or different, and each independently selected from the group consisting of hydrogen, hydroxy (OH), halogen, (C1~C4)alkyl, (C1~C4)alkoxy, acetyl, 1-hydroxyethyl (-CH(OH)CH₃) and trifluoromethyl (CF₃),
R³ is selected from the group consisting of 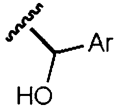 and substituted or unsubstituted phenyl, wherein the substituted phenyl may be substituted with any one selected from the group consisting of halogen, hydroxy (OH), (C1~C4)alkyl, (C1~C4)alkoxy, and trifluoromethyl (CF₃), and

Ar is selected from substituted or unsubstituted phenyl, pyridinyl, 1,3-benzodioxol, or naphthyl, wherein the substituted phenyl may be substituted with one or two substituents selected from the group consisting of halogen, (C1~C4)alkyl, (C1~C4)alkoxy, and trifluoromethyl.

The compound may be selected from the group consisting of:
2-(((5-(4-methoxyphenyl)furan-2-yl)methyl)amino)-1-phenylethanol (Compound 2a);
4-(5-(((2-hydroxy-2-phenylethyl)amino)methyl)furan-2-yl)phenol (Compound 3a);
1-phenyl-2-(((5-(4-(trifluoromethyl)phenyl)furan-2-yl)methyl)amino)ethanol (Compound 4a);
2-(((5-(4-fluorophenyl)furan-2-yl)methyl)amino)-1-phenylethanol (Compound 5a);
1-phenyl-2-(((5-(p-tolyl)furan-2-yl)methyl)amino)ethanol (Compound 6a);
1-(4-(5-(((2-hydroxy-2-phenylethyl)amino)methyl)furan-2-yl)phenyl)ethenone (Compound 7a);
2-(((5-(4-(1-hydroxyethyl)phenyl)furan-2-yl)methyl)amino)-1-phenylethanol (Compound 7b);
2-(((5-(3-chlorophenyl)furan-2-yl)methyl)amino)-1-phenylethanol (Compound 8a);
3-(5-(((2-hydroxy-2-phenylethyl)amino)methyl)furan-2-yl)phenol (Compound 9a);
1-phenyl-2-(((5-(3-(trifluoromethyl)phenyl)furan-2-yl)methyl)amino)ethanol (Compound 10a);
2-(((5-(3-fluorophenyl)furan-2-yl)methyl)amino)-1-phenylethanol (Compound 11a);
1-(3-(5-(((2-hydroxy-2-phenylethyl)amino)methyl)furan-2-yl)phenyl)ethenone (Compound 12a);
2-(((5-(3-(1-hydroxyethyl)phenyl)furan-2-yl)methyl)amino)-1-phenylethanol (Compound 12b);
2-(((5-(3,4-difluorophenyl)furan-2-yl)methyl)amino)-1-phenylethanol (Compound 13a);
2-(((5-(3,4-dichlorophenyl)furan-2-yl)methyl)amino)-1-phenylethanol (Compound 14a);
2-(((5-(3,4-dimethoxyphenyl)furan-2-yl)methyl)amino)-1-phenylethanol (Compound 15a);
2-(((5-(3-fluoro-4-methoxyphenyl)furan-2-yl)methyl)amino)-1-phenylethanol (Compound 16a);
2-(((5-(4-methoxy-3-methylphenyl)furan-2-yl)methyl)amino)-1-phenylethanol (Compound 17a);
(R)-2-(((5-(3-chloro-4-methoxyphenyl)furan-2-yl)methyl)amino)-1-phenylethanol (Compound 27);
(S)-2-(((5-(3-chloro-4-methoxyphenyl)furan-2-yl)methyl)amino)-1-phenylethanol (Compound 28);
2-(((5-(3-chloro-4-methoxyphenyl)furan-2-yl)methyl)amino)-1-(4-methoxyphenyl)ethanol (Compound 29);
2-(((5-(3-chloro-4-methoxyphenyl)furan-2-yl)methyl)amino)-1-(pyridin-3-yl)ethanol (Compound 30);
2-(((5-(3-chloro-4-methoxyphenyl)furan-2-yl)methyl)amino)-1-(p-tolyl)ethanol (Compound 31);
2-(((5-(3-chloro-4-methoxyphenyl)furan-2-yl)methyl)amino)-1-(4-(trifluoromethyl)phenyl)ethanol (Compound 32);
2-(((5-(3-chloro-4-methoxyphenyl)furan-2-yl)methyl)amino)-1-(4-fluoro-3-methoxyphenyl)ethanol (Compound 33);
2-(((5-(3-chloro-4-methoxyphenyl)furan-2-yl)methyl)amino)-1-(3,4-dimethoxyphenyl)ethanol (Compound 34);
1-(benzo[d][1,3]dioxol-5-yl)-2-(((5-(3-chloro-4-methoxyphenyl)furan-2-yl)methyl)amino)ethanol (Compound 35);
2-(((5-(3-chloro-4-methoxyphenyl)furan-2-yl)methyl)amino)-1-(3,4-difluorophenyl)ethanol (Compound 36);
2-(((5-(3-chloro-4-methoxyphenyl)furan-2-yl)methyl)amino)-1-(naphthalen-2-yl)ethanol (Compound 37);
1-(5-(2-fluorophenyl)furan-2-yl)-N-(4-methylbenzyl)methanamine (Compound 41);
1-(5-(2-fluorophenyl)furan-2-yl)-N-(4-methylbenzyl)methanamine hydrochloride (Compound 41a);
1-(5-(4-chlorophenyl)furan-2-yl)-N-(4-methoxybenzyl)methanamine (Compound 42);
N-(3-chloro-4-methoxybenzyl)-1-(5-(4-chlorophenyl)furan-2-yl)methanamine (Compound 43);
1-(5-(3-chloro-4-methoxyphenyl)furan-2-yl)-N-(4-methoxybenzyl)methanamine (Compound 44); and
1-(5-(4-chloro-3-methoxyphenyl)furan-2-yl)-N-(4-methoxybenzyl)methanamine (Compound 45), but is not limited thereto.

The compound may bind to an odorant receptor 164 (Olfr164).

The sepsis may be induced by infection with *Pseudomonas aeruginosa*.

The composition may modulate production of one or more inflammatory cytokines selected from the group consisting of IL-1β, IL-6, TNF-α, and IFN-γ, but is not limited thereto.

In the present specification, the term "pharmaceutical composition" refers to a composition administered for the purpose of preventing or treating a specific disease, and for the purposes of the present disclosure, to be administered for the treatment of sepsis.

The pharmaceutical composition according to the present disclosure may be prepared according to conventional methods in the pharmaceutical field. The pharmaceutical composition may be combined with a suitable pharmaceutically acceptable carrier according to the formulation, and may be prepared by further including excipients, diluents, dispersants, emulsifiers, buffers, stabilizers, binders, disintegrants, solvents, etc., as needed. The suitable carrier or the like may be selected differently depending on the form of administration and formulation, as it does not impair the activity and properties of the compound or a salt thereof according to the present disclosure.

Examples of carriers, excipients, diluents, etc., that may be included in the pharmaceutical composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, mineral oil, etc.

The pharmaceutical composition may be applied in any formulation, and more specifically, may be formulated and used as an oral formulation, topical preparation, suppository, and parenteral formulation of sterile injectable solution according to conventional methods, but is not limited thereto.

Among the oral formulations, solid formulations may be in the form of tablets, pills, powders, granules, capsules, etc., and may be prepared by mixing at least one excipient, such as starch, calcium carbonate, sucrose, lactose, sorbitol, mannitol, cellulose, gelatin, etc., and may also include lubricants such as magnesium stearate and talc in addition to simple excipients. In addition, in the case of capsule formulations, in addition to the substances mentioned above, a liquid carrier such as a fatty oil may be further included. Among the oral formulations, liquid formulations include suspensions, oral liquids, emulsions, syrups, etc., and may include various excipients, such as humectants, sweeteners, flavorings, and preservatives, in addition to commonly used simple diluents such as water and liquid paraffin.

The parenteral formulations may include sterile aqueous solutions, non-aqueous solvents, suspensions, emulsions, lyophilized preparations, and suppositories. As non-aqueous solvents and suspensions, propylene glycol, polyethylene glycol, vegetable oils such as olive oil, and injectable esters such as ethyl oleate may be used. As bases for suppositories, witepsol, macrogol, Tween 61, cacao oil, laurin oil, glycerogelatin, etc., may be used. Not limited thereto, any suitable preparation known in the art may be used.

The pharmaceutical composition according to the present disclosure may be administered in a pharmaceutically effective amount.

In the present specification, "pharmaceutically effective amount" refers to an amount sufficient to treat a disease with a reasonable benefit/risk ratio applicable to medical treatment and that does not cause adverse effects.

The effective dose level of the pharmaceutical composition may be determined differently depending on factors including the purpose of use, the patient's age, gender, weight and health status, type and severity of the disease, drug activity, sensitivity to the drug, method of administration, time of administration, route of administration and elimination rate, duration of treatment, drugs used in combination or concurrently, and other factors well known in the medical field. For example, although not constant, it may generally be administered at a dose of 0.001 to 1000 mg/kg, preferably 0.01 to 100 mg/kg, once or several times daily. The dosage does not limit the scope of the present disclosure in any way.

The pharmaceutical composition may be administered to any animal susceptible to sepsis, and said animals may include, for example, humans and primates, as well as livestock such as cattle, pigs, horses, and dogs.

The pharmaceutical composition may be administered via a suitable route of administration depending on the preparation form, and may be administered via various routes, oral or parenteral, as long as it reaches the target tissue. The method of administration does not need to be particularly limited and may be administered by conventional methods, such as, for example, oral, rectal or intravenous, intramuscular, topical application, respiratory inhalation, intradural or intracere-broventricular injection.

The pharmaceutical composition may be used alone for the prevention or treatment of sepsis, or in combination with surgery or other drug treatments.

The present disclosure provides a health functional food composition for preventing or improving sepsis including, as an active ingredient, a compound selected from a compound represented by Chemical Formula 1 below, a pharmaceutically acceptable salt thereof, a solvate thereof, or a stereoisomer thereof: 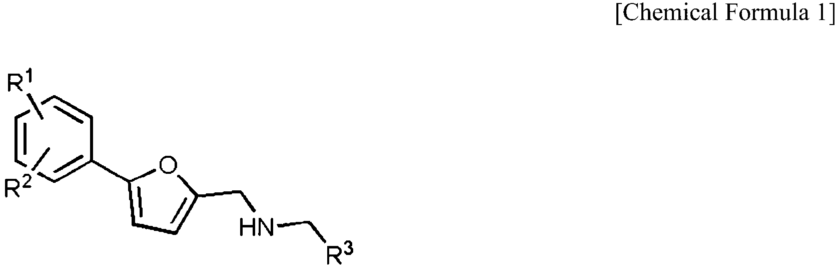 wherein, in Chemical Formula 1,
R¹ and R² may be the same or different, and each independently selected from the group consisting of hydrogen, hydroxy (OH), halogen, (C1~C4)alkyl, (C1~C4)alkoxy, acetyl, 1-hydroxyethyl (-CH(OH)CH₃) and trifluoromethyl (CF₃),
R³ is selected from the group consisting of 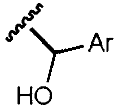 and substituted or unsubstituted phenyl, wherein the substituted phenyl may be substituted with any one selected from the group consisting of halogen, hydroxy (OH), (C1~C4)alkyl, (C1~C4)alkoxy, and trifluoromethyl (CF₃), and

Ar is selected from substituted or unsubstituted phenyl, pyridinyl, 1,3-benzodioxol, or naphthyl, wherein the substituted phenyl may be substituted with one or two substituents selected from the group consisting of halogen, (C1~C4)alkyl, (C1~C4)alkoxy, and trifluoromethyl.

The compound may bind to an odorant receptor 164 (Olfr164).

The sepsis may be induced by infection with *Pseudomonas aeruginosa*.

In the present specification, the term "health functional food" refers to a food manufactured and processed using raw materials or ingredients having functional properties useful to the human body, and means a food with high medical effects that is processed to efficiently exhibit biological regulatory functions in addition to nutritional supply, and may be used interchangeably with terms known in the art, such as functional food.

The health functional food according to the present disclosure may be manufactured in the form of powder, granules, tablets, capsules, syrups, or beverages for the purpose of preventing or improving sepsis, and there are no restrictions on the form that the health functional food may take, and it may include all foods in the conventional sense. For example, beverages and various drinks, fruits and processed foods thereof (canned fruit, jam, etc.), fish, meat and processed foods thereof (ham, bacon, etc.), breads and noodles, cookies and snacks, dairy products (butter, cheese, etc.), etc., are possible, and it may include all functional foods in the conventional sense. In addition, it may also include foods used as animal feed.

The health functional food composition may be manufactured by further including food-grade acceptable food additives and appropriate other auxiliary ingredients commonly used in the art. Unless otherwise stipulated, suitability as a food additive may be determined in accordance with the specifications and standards for the relevant item, based on the general provisions and general test methods of the Food Additives Codex approved by the Ministry of Food and Drug Safety. Items listed in the 'Food Additives Codex' may include, for example, chemically synthesized compounds such as ketones, glycine, calcium citrate, nicotinic acid, and cinnamon acid; natural additives such as persimmon dye, licorice extract, crystalline cellulose, sorghum dye, and guar gum; and mixed preparations such as L-sodium glutamate preparations, alkaline noodle additives, preservative preparations, and tar dye preparations.

The other auxiliary ingredients may additionally contain, for example, flavoring agents, natural carbohydrates, sweeteners, vitamins, electrolytes, coloring agents, pectic acid, alginic acid, organic acids, protective colloidal thickeners, pH adjusters, stabilizers, preservatives, glycerin, alcohol, carbonating agents, etc. In particular, the natural carbohydrates may include monosaccharides such as glucose and fructose, disaccharides such as maltose and sucrose, polysaccharides such as dextrin and cyclodextrin, and sugar alcohols such as xylitol, sorbitol, and erythritol, and as sweeteners, natural sweeteners such as taumatin and stevia extract, or synthetic sweeteners such as saccharin and aspartame may be used.

The effective dose of the compound or the salt thereof contained in the health functional food composition may be appropriately adjusted according to the purpose of use, such as the prevention or improvement of sepsis.

The present disclosure provides a reagent composition for modulating an odorant receptor Olfr164 including, as an active ingredient, a compound selected from a compound represented by Chemical Formula 1 below, a pharmaceutically acceptable salt thereof, a solvate thereof, or a stereoisomer thereof: 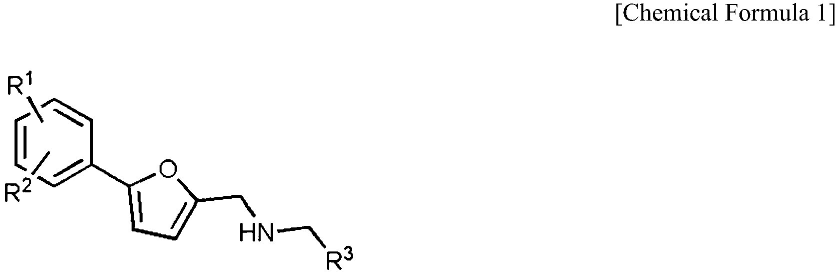 wherein, in Chemical Formula 1,
R¹ and R² may be the same or different, and each independently selected from the group consisting of hydrogen, hydroxy (OH), halogen, (C1~C4)alkyl, (C1~C4)alkoxy, acetyl, 1-hydroxyethyl (-CH(OH)CH₃) and trifluoromethyl (CF₃),
R³ is selected from the group consisting of 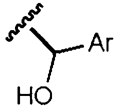 and substituted or unsubstituted phenyl, wherein the substituted phenyl may be substituted with any one selected from the group consisting of halogen, hydroxy (OH), (C1~C4)alkyl, (C1~C4)alkoxy, and trifluoromethyl (CF₃), and

Ar is selected from substituted or unsubstituted phenyl, pyridinyl, 1,3-benzodioxol, or naphthyl, wherein the substituted phenyl may be substituted with one or two substituents selected from the group consisting of halogen, (C1~C4)alkyl, (C1~C4)alkoxy, and trifluoromethyl.

Hereinafter, example embodiments of the present disclosure will be described in detail. Examples described below are merely for illustrating the present disclosure in detail, and the scope of the present disclosure is not limited by the examples. The embodiments of the present disclosure are provided to more completely explain the invention to those skilled in the art.

### <Synthesis Example 1> Synthesis of β-amino alcohol derivatives (1)

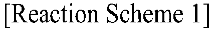

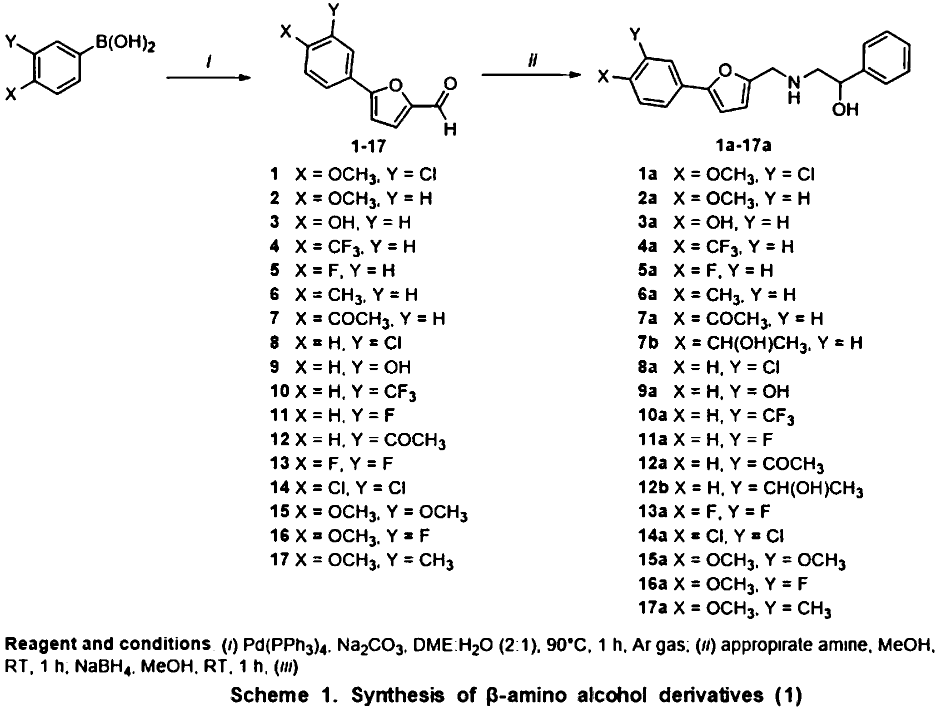

### 1-1. General synthesis method A: Suzuki coupling reaction

5-bromofuran-2-carbaldehyde, sodium carbonate (2.0 equivalents), and tetrakis(triphenylphosphine)palladium (0) (0.02 equivalents) were placed in a mixed solvent of dimethoxyethane (10 mL) and water (15 mL) to prepare a reaction mixture, which was then purged with argon. The reaction mixture was sonicated at room temperature for 5 minutes, after which a solution of appropriate arylboronic acid (1.25 equivalents) dissolved in ethanol (2 mL) was added. Subsequently, the reaction mixture was heated to 90 °C and stirred for 1 hour. After cooling the reaction mixture to room temperature, the residue was diluted by adding ethyl acetate (EtOAc), and the organic layer was washed with a saturated sodium bicarbonate aqueous solution and then washed with brine. This was dried with magnesium sulfate (MgSO₄) and concentrated under reduced pressure. The obtained solid was purified by silica gel column chromatography (hexane/EtOAc) to obtain the desired pure product.

### (1) 5-(3-chloro-4-methoxyphenyl)furan-2-carbaldehyde; (Intermediate 1)

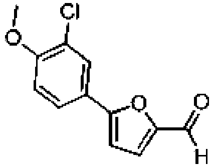

Intermediate 1 was prepared as a yellow solid in 80% yield using 5-bromofuran-2-carbaldehyde (150 mg, 0.86 mmol) and 3-chloro-4-methoxyphenylboronic acid (200 mg, 1.07 mmol) in the same manner as general synthesis method A. The crude product was purified using silica gel column chromatography (hexane/EtOAc).

*R*_{f} = 0.4 (hexane/EtOAc = 7:3). ¹H NMR (600 MHz, CDCl₃) δ 9.62 (s, 1H), 7.83 (d, J = 2.1 Hz, 1H), 7.71 (dd, J = 8.6, 2.1 Hz, 1H), 7.30 (d, J = 3.7 Hz, 1H), 6.99 (d, J = 8.6 Hz, 1H), 6.74 (d, J = 3.7 Hz, 1H), 3.96 (s, 3H).

### (2) 5-(4-methoxyphenyl)furan-2-carbaldehyde; (Intermediate 2)

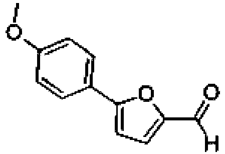

Intermediate 2 was prepared as a yellow solid in 86% yield using 5-bromofuran-2-carbaldehyde (300 mg, 1.71 mmol) and 4-methoxyphenylboronic acid (325 mg, 2.14 mmol) in the same manner as general synthesis method A. The crude product was purified using silica gel column chromatography (hexane/EtOAc).

*R*_{f} = 0.42 (hexane/EtOAc = 7:3); ¹H NMR (600 MHz, CDCl₃) δ 9.59 (s, 1H), 7.76 (d, J = 8.9 Hz, 2H), 7.30 (d, J = 3.7 Hz, 1H), 6.96 (d, J = 8.9 Hz, 2H), 6.71 (d, J = 3.7 Hz, 1H), 3.85 (s, 3H).

### (3) 5-(4-hydroxyphenyl)furan-2-carbaldehyde; (Intermediate 3)

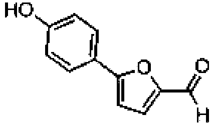

Intermediate 3 was prepared as a yellow solid in 90% yield using 5-bromofuran-2-carbaldehyde (250 mg, 1.43 mmol) and 4-hydroxyphenylboronic acid (246 mg, 1.78 mmol) in the same manner as general synthesis method A. The crude product was purified using silica gel column chromatography (hexane/EtOAc).

*R*_{f} = 0.30 (hexane/EtOAc = 2:1); ¹H NMR (600 MHz, DMSO) δ 10.02 (s, 1H), 9.53 (s, 1H), 7.71 (d, J = 8.7 Hz, 2H), 7.61 (d, J = 3.7 Hz, 1H), 7.06 (d, J = 3.7 Hz, 1H), 6.88 (d, J = 8.7 Hz, 2H).

### (4) 5-(4-(trifluoromethyl)phenyl)furan-2-carbaldehyde; (Intermediate 4)

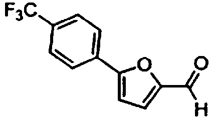

Intermediate 4 was prepared as a yellow solid in yield of 59% using 5-bromofuran-2-carbaldehyde (250 mg, 1.43 mmol) and 4-(trifluoromethyl)phenylboronic acid (339 mg, 1.78 mmol) in the same manner as general synthesis method A. The crude product was purified using silica gel column chromatography (hexane/EtOAc).

*R*_{f} = 0.50 (hexane/EtOAc = 3:1); ¹H NMR (600 MHz, CDCl₃) δ 9.70 (s, 1H), 7.93 (d, J = 8.2 Hz, 2H), 7.71 (d, J = 8.2 Hz, 2H), 7.35 (d, J = 3.7 Hz, 1H), 6.95 (d, J = 3.7 Hz, 1H).

### (5) 5-(4-fluorophenyl)furan-2-carbaldehyde; (Intermediate 5)

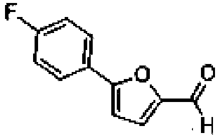

Intermediate 5 was prepared as a yellow solid in 95% yield using 5-bromofuran-2-carbaldehyde (250 mg, 1.43 mmol) and 4-fluorophenylboronic acid (250 mg, 1.78 mmol) in the same manner as general synthesis method A. The crude product was purified using silica gel column chromatography (hexane/EtOAc).

*R*_{f} = 0.46 (hexane/EtOAc = 7:3); ¹H NMR (600 MHz, CDCl₃) δ 9.64 (s, 1H), 7.87 - 7.75 (m, 2H), 7.32 (d, J = 3.5 Hz, 1H), 7.14 (t, J = 8.4 Hz, 2H), 6.79 (d, J = 3.5 Hz, 1H).

### (6) 5-(p-tolyl)furan-2-carbaldehyde; (Intermediate 6)

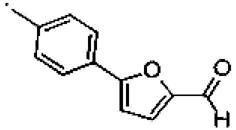

Intermediate 6 was prepared as a yellow solid in 97% yield using 5-bromofuran-2-carbaldehyde (250 mg, 1.43 mmol) and 4-methylphenylboronic acid (243 mg, 1.78 mmol) in the same manner as general synthesis method A. The crude product was purified using silica gel column chromatography (hexane/EtOAc).

*R*_{f} = 0.35 (hexane/EtOAc = 4:1). ¹H NMR (600 MHz, CDCl₃) δ 9.63 (s, 1H), 7.72 (d, J = 8.2 Hz, 2H), 7.31 (d, J = 3.7 Hz, 1H), 7.25 (d, J = 8.2 Hz, 2H), 6.79 (d, J = 3.7 Hz, 1H), 2.39 (s, 3H).

### (7) 5-(4-acetylphenyl)furan-2-carbaldehyde; (Intermediate 7)

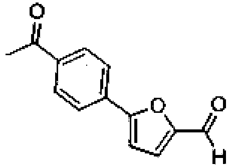

Intermediate 7 was prepared as a yellow solid in 39% yield using 5-bromofuran-2-carbaldehyde (250 mg, 1.43 mmol) and 4-acetylphenylboronic acid (293 mg, 1.78 mmol) in the same manner as general synthesis method A. The crude product was purified using silica gel column chromatography (hexane/EtOAc).

*R*_{f} = 0.30 (hexane/EtOAc = 7:3). ¹H NMR (600 MHz, CDCl₃) δ 9.71 (s, 1H), 8.03 (d, J = 8.6 Hz, 2H), 7.92 (d, J = 8.6 Hz, 2H), 7.35 (d, J = 3.7 Hz, 1H), 6.97 (d, J = 3.7 Hz, 1H), 2.64 (s, 3H).

### (8) 5-(3-chlorophenyl)furan-2-carbaldehyde; (Intermediate 8)

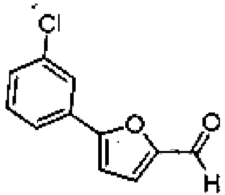

Intermediate 8 was prepared as a yellow solid in 77% yield using 5-bromofuran-2-carbaldehyde (200 mg, 1.14 mmol) and 3-chlorophenylboronic acid (223 mg, 1.43 mmol) in the same manner as general synthesis method A. The crude product was purified using silica gel column chromatography (hexane/EtOAc).

*R*_{f} = 0.36 (hexane/EtOAc = 4:1). ¹H NMR (600 MHz, CDCl₃) δ 9.67 (s, 1H), 7.81 (t, J = 1.6 Hz, 1H), 7.70 (dt, J = 7.1, 1.6 Hz, 1H), 7.41 - 7.34 (m, 2H), 7.32 (d, J = 3.7 Hz, 1H), 6.86 (d, J = 3.7 Hz, 1H).

### (9) 5-(3-hydroxyphenyl)furan-2-carbaldehyde; (Intermediate 9)

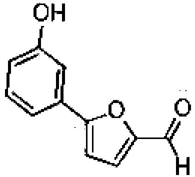

Intermediate 9 was prepared as a yellow solid in 69% yield using 5-bromofuran-2-carbaldehyde (250 mg, 1.43 mmol) and 3-hydroxyphenylboronic acid (246 mg, 1.78 mmol) in the same manner as general synthesis method A. The crude product was purified using silica gel column chromatography (hexane/EtOAc).

*R*_{f} = 0.30 (hexane/EtOAc = 2:1). ¹H NMR (600 MHz, DMSO) δ 9.75 (s, 1H), 9.59 (s, 1H), 7.63 (d, J = 3.7 Hz, 1H), 7.32 - 7.28 (m, 2H), 7.26 (s, 1H), 7.22 (d, J = 3.7 Hz, 1H), 6.87 - 6.79 (m, 1H).

### (10) 5-(3-(trifluoromethyl)phenyl)furan-2-carbaldehyde; (Intermediate 10)

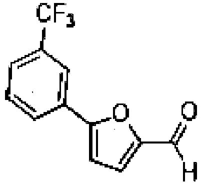

Intermediate 10 was prepared as a yellow liquid with 97% yield using 5-bromofuran-2-carbaldehyde (250 mg, 1.43 mmol) and 3-(trifluoromethyl)phenylboronic acid (339 mg, 1.78 mmol) in the same manner as general synthesis method A. The crude product was purified using silica gel column chromatography (hexane/EtOAc).

*R*_{f} = 0.41 (hexane/EtOAc = 7:3). ¹H NMR (600 MHz, CDCl₃) δ 9.69 (s, 1H), 8.04 (s, 1H), 7.99 (d, J = 7.8 Hz, 1H), 7.64 (d, J = 7.8 Hz, 1H), 7.57 (t, J = 7.8 Hz, 1H), 7.34 (d, J = 3.7 Hz, 1H), 6.93 (d, J = 3.7 Hz, 1H).

### (11) 5-(3-fluorophenyl)furan-2-carbaldehyde; (Intermediate 11)

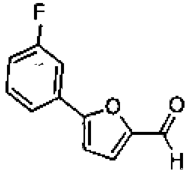

Intermediate 11 was prepared as a yellow solid in 96% yield using 5-bromofuran-2-carbaldehyde (250 mg, 1.43 mmol) and 3-fluorophenylboronic acid (250 mg, 1.78 mmol) in the same manner as general synthesis method A. The crude product was purified using silica gel column chromatography (hexane/EtOAc).

*R*_{f} = 0.50 (hexane/EtOAc = 7:3); ¹H NMR (600 MHz, CDCl₃) δ 9.67 (s, 1H), 7.60 (d, J = 7.8 Hz, 1H), 7.51 (d, J = 9.6 Hz, 1H), 7.44 - 7.38 (m, 1H), 7.32 (d, J = 3.7 Hz, 1H), 7.09 (t, J = 8.3 Hz, 1H), 6.86 (d, J = 3.7 Hz, 1H).

### (12) 5-(3-acetylphenyl)furan-2-carbaldehyde; (Intermediate 12)

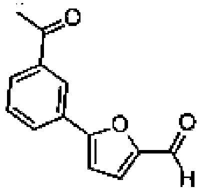

Intermediate 12 was prepared as a yellow solid in 29% yield using 5-bromofuran-2-carbaldehyde (250 mg, 1.43 mmol) and 3-acetylphenylboronic acid (293 mg, 1.78 mmol) in the same manner as general synthesis method A. The crude product was purified using silica gel column chromatography (hexane/EtOAc).

*R*_{f} = 0.26 (hexane/EtOAc = 7:3); ¹H NMR (600 MHz, CDCl₃) δ 9.69 (s, 1H), 8.37 (s, 1H), 8.02 (d, J = 7.7 Hz, 1H), 7.97 (d, J = 7.7 Hz, 1H), 7.56 (t, J = 7.7 Hz, 1H), 7.35 (d, J = 3.7 Hz, 1H), 6.94 (d, J = 3.7 Hz, 1H), 2.67 (s, 3H).

### (13) 5-(3,4-difluorophenyl)furan-2-carbaldehyde; (Intermediate 13)

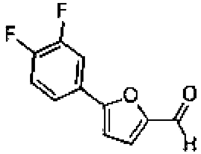

Intermediate 13 was prepared as a yellow solid in 92% yield using 5-bromofuran-2-carbaldehyde (250 mg, 1.43 mmol) and 3,4-difluorophenylboronic acid (282 mg, 1.78 mmol) in the same manner as general synthesis method A. The crude product was purified using silica gel column chromatography (hexane/EtOAc).

*R*_{f} = 0.65 (hexane/EtOAc = 7:3); ¹H NMR (600 MHz, CDCl₃) δ 9.66 (s, 1H), 7.63 (ddd, J = 10.8, 7.5, 2.1 Hz, 1H), 7.58 - 7.53 (m, 1H), 7.31 (d, J = 3.7 Hz, 1H), 7.28 - 7.21 (m, 1H), 6.80 (d, J = 3.7 Hz, 1H).

### (14) 5-(3,4-dichlorophenyl)furan-2-carbaldehyde; (Intermediate 14)

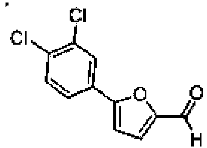

Intermediate 14 was prepared as a yellow solid in 74% yield using 5-bromofuran-2-carbaldehyde (250 mg, 1.43 mmol) and 3,4-dichlorophenylboronic acid (340 mg, 1.78 mmol) in the same manner as general synthesis method A. The crude product was purified using silica gel column chromatography (hexane/EtOAc).

*R*_{f} = 0.27 (hexane/EtOAc = 6:1); ¹H NMR (600 MHz, CDCl₃) δ 9.87 (s, 1H), 7.72 (d, J = 3.9 Hz, 1H), 7.69 (d, J = 2.2 Hz, 1H), 7.54 (dd, J = 8.6, 2.2 Hz, 1H), 7.30 (d, J = 3.9 Hz, 1H), 6.98 (d, J = 8.6 Hz, 1H), 3.95 (s, 3H).

### (15) 5-(3,4-dimethoxyphenyl)furan-2-carbaldehyde; (Intermediate 15)

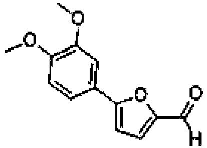

Intermediate 15 was prepared as a yellow solid in 78% yield using 5-bromofuran-2-carbaldehyde (250 mg, 1.43 mmol) and 3,4-dimethoxyphenylboronic acid (259 mg, 1.78 mmol) in the same manner as general synthesis method A. The crude product was purified using silica gel column chromatography (hexane/EtOAc).

*R*_{f} = 0.25 (hexane/EtOAc = 7:3); ¹H NMR (600 MHz, CDCl₃) δ 9.61 (s, 1H), 7.40 (dd, J = 8.4, 2.0 Hz, 1H), 7.32 (d, J = 2.0 Hz, 1H), 7.31 (d, J = 3.7 Hz, 1H), 6.93 (d, J = 8.4 Hz, 1H), 6.74 (d, J = 3.7 Hz, 1H), 3.97 (s, 3H), 3.93 (s, 3H).

### (16) 5-(3-fluoro-4-methoxyphenyl)furan-2-carbaldehyde; (Intermediate 16)

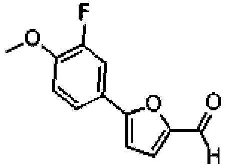

Intermediate 16 was prepared as a yellow solid in 58% yield using 5-bromofuran-2-carbaldehyde (250 mg, 1.43 mmol) and 3-fluoro-4-methoxyphenylboronic acid (303 mg, 1.78 mmol) in the same manner as general synthesis method A. The crude product was purified using silica gel column chromatography (hexane/EtOAc).

*R*_{f} = 0.10 (hexane/EtOAc = 9:1); ¹H NMR (600 MHz, CDCl₃) δ 9.62 (s, 1H), 7.60 - 7.56 (m, 1H), 7.53 (dd, J = 11.9, 2.1 Hz, 1H), 7.30 (d, J = 3.7 Hz, 1H), 7.02 (t, J = 8.5 Hz, 1H), 6.73 (d, J = 3.7 Hz, 1H), 3.94 (s, 3H).

### (17) 5-(4-methoxy-3-methylphenyl)furan-2-carbaldehyde; (Intermediate 17)

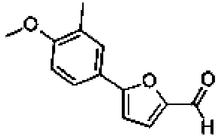

Intermediate 17 was prepared as a yellow solid in 95% yield using 5-bromofuran-2-carbaldehyde (250 mg, 1.43 mmol) and 4-methoxy-3-methylphenylboronic acid (296 mg, 1.78 mmol) in the same manner as general synthesis method A. The crude product was purified using silica gel column chromatography (hexane/EtOAc).

*R*_{f} = 0.60 (hexane/EtOAc = 7:3); ¹H NMR (600 MHz, CDCl₃) δ 9.59 (s, 1H), 7.65 (dd, J = 8.5, 2.2 Hz, 1H), 7.61 (d, J = 2.2 Hz, 1H), 7.30 (d, J = 3.7 Hz, 1H), 6.87 (d, J = 8.5 Hz, 1H), 6.70 (d, J = 3.7 Hz, 1H), 3.88 (s, 3H), 2.26 (s, 3H).

### 1-2. General synthesis method B: Reductive amination

An amine (1.0 equivalent) was added to a solution in which aldehydes (Intermediates 1 to 17) were dissolved in methanol (5 mL). The reaction mixture was stirred at room temperature for 1 hour and then cooled to 0 °C. NaBH₄ (3.0 equivalents) was added with stirring, and then the mixture was stirred at room temperature until the reaction was fully complete (typically 2 hours) by TLC analysis. The reaction was stopped with distilled water, and the methanol was removed under reduced pressure. The residue was diluted with ethyl acetate, and the organic layer was washed with a saturated aqueous sodium bicarbonate solution and brine, then dried with magnesium sulfate and concentrated under reduced pressure. The residue was recrystallized from ether and purified to obtain the desired pure product.

### (1) 2-(((5-(3-chloro-4-methoxyphenyl)furan-2-yl)methyl)amino)-1-phenylethanol; (Compound 1a)

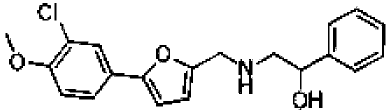

Compound 1a was prepared as a white solid in 60% yield using 5-(4-methoxyphenyl)furan-2-carbaldehyde (1) (126 mg, 0.53 mmol) and 2-amino-1-phenylethanol (73 mg, 0.53 mmol) according to the same procedure as described in general synthesis method B.

*R*_{f} = 0.1 (Hexane/EtOAc = 7:3); ¹H NMR (600 MHz, CDCl₃) δ 7.65 (d, J = 1.9 Hz, 1H), 7.49 (dd, J = 8.6, 1.9 Hz, 1H), 7.37 - 7.33 (m, 5H), 7.28 (d, J = 7.0 Hz, 1H), 6.93 (d, J = 8.6 Hz, 1H), 6.46 (d, J = 3.2 Hz, 1H), 6.23 (d, J = 3.2 Hz, 1H), 4.75 (dd, J = 8.8, 3.4 Hz, 1H), 3.92 (s, 3H), 3.87 (d, J = 1.1 Hz, 2H), 2.97 (dd, J = 12.3, 3.4 Hz, 1H), 2.78 (dd, J = 12.3, 8.8 Hz, 1H), 1.95 - 1.52 (br, 2H). HRMS (ESI) m/z calculated for C₂₀H₂₀ClNNaO₃⁺ [M + Na]⁺: 380.0992; found: 380.1350

### (2) 2-(((5-(4-methoxyphenyl)furan-2-yl)methyl)amino)-1-phenylethanol; (Compound 2a)

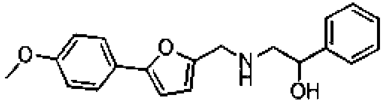

Compound 2a was prepared as a white solid in 81% yield using 5-(4-methoxyphenyl)furan-2-carbaldehyde (2) (300 mg, 1.48 mmol) and 2-amino-1-phenylethanol (203 mg, 1.48 mmol) according to the same procedure as described in general synthesis method B.

*R*_{f} = 0.37 (CH₂Cl₂/MeOH = 15:1); ¹H NMR (600 MHz, CDCl₃) δ 7.56 (d, J = 8.3 Hz, 2H), 7.39 - 7.31 (m, 4H), 7.29 - 7.27 (m, 1H), 6.91 (d, J = 8.3 Hz, 2H), 6.43 (d, J = 2.7 Hz, 1H), 6.22 (d, J = 2.7 Hz, 1H), 4.73 (dd, J = 8.9, 3.3 Hz, 1H), 3.88 (d, J = 15.6 Hz, 1H), 3.86 (d, J = 15.6 Hz, 1H), 3.83 (s, 3H), 3.49 (br, 1H), 2.98 (dd, J = 12.1, 3.3 Hz, 1H), 2.77 (dd, J = 12.1, 8.9 Hz, 1H). HRMS (ESI) m/z calculated for C₁₂H₁₁O₂ ⁺ [M - C₈H₁₀NO]⁺: 187.0754; found: 187.0766, C₂₀H₂₁NO₃Na ⁺ [M + Na]⁺: 346.1413; found: 346.1413.

### (3) 4-(5-(((2-hydroxy-2-phenylethyl)amino)methyl)furan-2-yl)phenol; (Compound 3a)

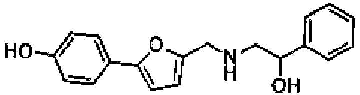

Compound 3a was prepared as a brown solid in 50% yield using 5-(4-hydroxyphenyl)furan-2-carbaldehyde (3) (130 mg, 0.69 mmol) and 2-amino-1-phenylethanol (94 mg, 0.69 mmol) according to the same procedure as described in general synthesis method B.

*R*_{f} = 0.06 (CH₂Cl₂/MeOH = 15:1); ¹H NMR (600 MHz, DMSO) δ 9.57 (s, 1H), 7.46 (d, J = 8.6 Hz, 2H), 7.36 - 7.27 (m, 4H), 7.22 (t, J = 7.1 Hz, 1H), 6.79 (d, J = 8.6 Hz, 2H), 6.57 (d, J = 3.1 Hz, 1H), 6.26 (d, J = 3.1 Hz, 1H), 5.28 (s, 1H), 4.64 (s, 1H), 3.75 (d, J = 14.7 Hz, 1H), 3.72 (d, J = 14.7 Hz, 1H), 2.68 - 2.64 (m, 2H), 2.06 (s, 1H). HRMS (ESI) m/z calculated for C₁₁H₉O₂ ⁺ [M - C₈H₁₀NO]⁺: 173.0597; found: 173.0585, C₁₉H₁₉NO₃Na ⁺ [M + Na]⁺: 332.1257; found: 332.1247.

### (4) 1-phenyl-2-(((5-(4-(trifluoromethyl)phenyl)furan-2-yl)methyl)amino)ethanol; (Compound 4a)

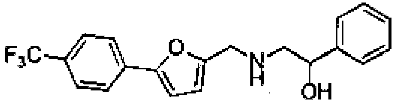

Compound 4a was prepared as a white solid in 54% yield using 5-(4-(trifluoromethyl)phenyl)furan-2-carbaldehyde (4) (150 mg, 0.62 mmol) and 2-amino-1-phenylethanol (85 mg, 0.62 mmol) according to the same procedure as described in general synthesis method B.

*R*_{f} = 0.31 (CH₂Cl₂/MeOH = 15:1); ¹H NMR (600 MHz, CDCl₃) δ 7.72 (d, J = 8.2 Hz, 2H), 7.61 (d, J = 8.2 Hz, 2H), 7.38 - 7.32 (m, 4H), 7.30 - 7.27 (m, 1H), 6.69 (d, J = 3.3 Hz, 1H), 6.30 (d, J = 3.3 Hz, 1H), 4.75 (dd, J = 8.8, 3.6 Hz, 1H), 3.92 (d, J = 15.3 Hz, 1H), 3.90 (d, J = 15.3 Hz, 1H), 3.38 (s, 1H), 2.98 (dd, J = 12.2, 3.6 Hz, 1H), 2.80 (dd, J = 12.2, 8.8 Hz, 1H). HRMS (ESI) m/z calculated for C₁₂H₈F₃O ⁺ [M - C₈H₁₀NO]⁺: 225.0522; found: 225.0519, C₂₀H₁₉F₃NO₂ ⁺ [M + H]⁺: 362.1363; found: 362.1360.

### (5) 2-(((5-(4-fluorophenyl)furan-2-yl)methyl)amino)-1-phenylethanol; (Compound 5a)

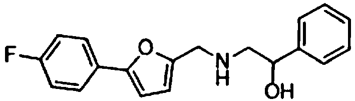

Compound 5a was prepared as a yellow solid in 35% yield using 5-(4-fluorophenyl)furan-2-carbaldehyde (5) (136 mg, 0.71 mmol) and 2-amino-1-phenylethanol (98 mg, 0.71 mmol) according to the same procedure as described in general synthesis method B.

*R*_{f} = 0.28 (CH₂Cl₂/MeOH = 15:1). ¹H NMR (600 MHz, CDCl₃) δ 7.64 - 7.56 (m, 2H), 7.40 - 7.31 (m, 4H), 7.30 - 7.27 (m, 1H), 7.06 (t, J = 8.7 Hz, 2H), 6.50 (d, J = 3.2 Hz, 1H), 6.24 (d, J = 3.2 Hz, 1H), 4.74 (dd, J = 8.9, 3.6 Hz, 1H), 3.89 (d, J = 15.0 Hz, 1H), 3.86 (d, J = 15.0 Hz, 1H), 3.46 (br, 1H), 2.97 (dd, J = 12.3, 3.6 Hz, 1H), 2.78 (dd, J = 12.2, 8.9 Hz, 1H). HRMS (ESI) m/z calculated for C₁₁H₈FO ⁺ [M - C₈H₁₀NO]⁺: 175.0554; found: 175.0549, C₁₉H₁₉FNO₂ ⁺ [M + H]⁺:312.1395; found: 312.1393.

### (6) 1-phenyl-2-(((5-(p-tolyl)furan-2-yl)methyl)amino)ethanol; (Compound 6a)

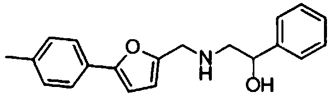

Compound 6a was prepared as a white solid in 62% yield using 5-(p-tolyl)furan-2-carbaldehyde (6) (136 mg, 0.73 mmol) and 2-amino-1-phenylethanol (100 mg, 0.73 mmol) according to the same procedure as described in general synthesis method B.

*R*_{f} = 0.30 (CH₂Cl₂/MeOH = 15:1); ¹H NMR (600 MHz, CDCl₃) δ 7.53 (d, J = 8.1 Hz, 2H), 7.39 - 7.31 (m, 4H), 7.27 (d, J = 7.2 Hz, 1H), 7.18 (d, J = 8.1 Hz, 2H), 6.51 (d, J = 3.2 Hz, 1H), 6.23 (d, J = 3.2 Hz, 1H), 4.73 (dd, J = 8.9, 3.5 Hz, 1H), 3.89 (d, J = 14.8 Hz, 1H), 3.86 (d, J = 14.8 Hz, 1H), 2.98 (dd, J = 12.3, 3.5 Hz, 1H), 2.77 (dd, J = 12.3, 8.9 Hz, 1H), 2.36 (s, 3H). HRMS (ESI) m/z calculated for C₁₂H₁₁O ⁺ [M - C₈H₁₀NO]⁺: 171.0804; found: 175.0804, C₂₀H₂₁NO₂Na ⁺ [M + Na]⁺: 330.1464; found: 330.1456.

### (7) 1-(4-(5-(((2-hydroxy-2-phenylethyl)amino)methyl)furan-2-yl)phenyl)ethenone; (Compound 7a)

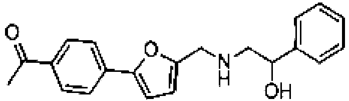

2-amino-1-phenylethanol (64 mg, 0.47 mmol) was added to a methanol (5 mL) solution of 5-(4-acetylphenyl)furan-2-carbaldehyde (7) (101 mg, 0.47 mmol). The reaction mixture was stirred at room temperature for 1 hour and then cooled to 0 °C. NaBH4 (17 mg, 0.47 mmol) was added with stirring, and the mixture was stirred at 0 °C until the transition to TLC analysis was complete (typically 30 minutes). The reaction was stopped with distilled water and methanol was removed under reduced pressure. The residue was diluted with ethyl acetate, and the organic layer was washed with a saturated sodium bicarbonate aqueous solution and brine, then dried with magnesium sulfate and concentrated under reduced pressure. The residue was then purified by silica gel column chromatography (CH₂Cl₂/MeOH=97:3) to obtain Compound 7a as a white solid (35 mg, 15%).

*R*_{f} = 0.25 (CH₂Cl₂/MeOH = 40:1); ¹H NMR (600 MHz, CDCl₃) δ 7.96 (d, J = 8.5 Hz, 2H), 7.71 (d, J = 8.5 Hz, 2H), 7.39 - 7.32 (m, 4H), 7.30 - 7.26 (m, 1H), 6.73 (d, J = 3.3 Hz, 1H), 6.31 (d, J = 3.3 Hz, 1H), 4.76 (dd, J = 8.8, 3.6 Hz, 1H), 3.92 (d, J = 15.6 Hz, 1H), 3.90 (d, J = 15.6 Hz, 1H), 2.98 (dd, J = 12.3, 3.6 Hz, 1H), 2.80 (dd, J = 12.3, 8.8 Hz, 1H), 2.61 (s, 3H). HRMS (ESI) m/z calculated for C₁₃H₁₁O₂ ⁺ [M - C₈H₁₀NO]⁺: 199.0754; found: 199.0752, C₂₁H₂₂NO₃ ⁺ [M + H]⁺: 336.1594; found: 336.1587.

### (8) 2-(((5-(4-(1-hydroxyethyl)phenyl)furan-2-yl)methyl)amino)-1-phenylethanol; (Compound 7b)

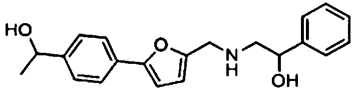

Compound 7b was prepared as a white solid in 19% yield using 5-(4-acetylphenyl)furan-2-carbaldehyde (7) (77 mg, 0.36 mmol) and 2-amino-1-phenylethanol (49 mg, 0.36 mmol) according to the same procedure as described in general synthesis method B.

*R*_{f} = 0.18 (CH₂Cl₂/MeOH = 40:1); ¹H NMR (600 MHz, CDCl₃) δ 7.62 (d, J = 8.3 Hz, 2H), 7.40 - 7.31 (m, 6H), 7.28 (d, J = 7.1 Hz, 1H), 6.56 (d, J = 3.2 Hz, 1H), 6.25 (d, J = 3.2 Hz, 1H), 4.91 (q, J = 6.5 Hz, 1H), 4.74 (dd, J = 8.9, 3.6 Hz, 1H), 3.90 (d, J = 15.0 Hz, 1H), 3.88 (d, J = 15.0 Hz, 1H), 2.98 (dd, J = 12.3, 3.6 Hz, 1H), 2.78 (dd, J = 12.3, 8.9 Hz, 1H), 1.51 (d, J = 6.5 Hz, 3H). HRMS (ESI) m/z calculated for C₁₃H₁₃O₂ ⁺ [M - C₈H₁₀NO]⁺: 201.0910; found: 201.0909, C₂₁H₂₃NO₃Na ⁺ [M + Na]⁺: 360.1570; found: 360.1561.

### (9) 2-(((5-(3-chlorophenyl)furan-2-yl)methyl)amino)-1-phenylethanol; (Compound 8a)

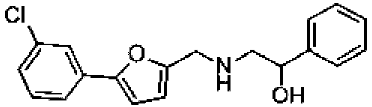

Compound 8a was prepared as a white solid in 52% yield using 5-(3-chlorophenyl)furan-2-carbaldehyde (8) (183 mg, 0.88 mmol) and 2-amino-1-phenylethanol (121 mg, 0.88 mmol) according to the same procedure as described in general synthesis method B.

*R*_{f} = 0.41 (CH₂Cl₂/MeOH = 15:1); ¹H NMR (600 MHz, CDCl₃) δ 7.62 (s, 1H), 7.50 (d, J = 7.7 Hz, 1H), 7.39 - 7.32 (m, 4H), 7.31 - 7.27 (m, 2H), 7.21 (d, J = 7.9 Hz, 1H), 6.60 (d, J = 3.1 Hz, 1H), 6.26 (d, J = 3.1 Hz, 1H), 4.75 (dd, J = 8.8, 3.5 Hz, 1H), 3.90 (d, J = 15.6 Hz, 1H), 3.87 (d, J = 15.6 Hz, 1H), 3.42 (br, 1H), 2.97 (dd, J = 12.2, 3.5 Hz, 1H), 2.78 (dd, J = 12.2, 8.8 Hz, 1H). HRMS (ESI) m/z calculated for C₁₁H₈ClO ⁺ [M - C₈H₁₀NO]⁺: 191.0258; found: 191.0241, C₁₉H₁₉ClNO₂ ⁺ [M + H]⁺: 328.1099; found: 328.1074.

### (10) 3-(5-(((2-hydroxy-2-phenylethyl)amino)methyl)furan-2-yl)phenol; (Compound 9a)

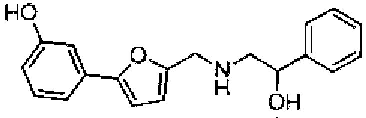

Compound 9a was prepared as a yellow solid in 67% yield using 5-(3-hydroxyphenyl)furan-2-carbaldehyde (9) (135 mg, 0.71 mmol) and 2-amino-1-phenylethanol (98 mg, 0.71 mmol) according to the same procedure as described in general synthesis method B.

*R*_{f} = 0.25 (CH₂Cl₂/MeOH = 15:1); ¹H NMR (600 MHz, CDCl₃) δ 7.37 - 7.30 (m, 4H), 7.29 - 7.25 (m, 2H), 7.23 (t, J = 7.9 Hz, 1H), 7.19 - 7.15 (m, 2H), 6.73 (ddd, J = 7.9, 2.4, 0.8 Hz, 1H), 6.54 (d, J = 3.2 Hz, 1H), 6.24 (d, J = 3.2 Hz, 1H), 4.79 (dd, J = 9.1, 3.5 Hz, 1H), 3.89 (d, J = 14.8 Hz, 1H), 3.86 (d, J = 14.8 Hz, 1H), 2.92 (dd, J = 12.2, 3.5 Hz, 2H), 2.81 (dd, J = 12.2, 9.1 Hz, 2H). HRMS (ESI) m/z calculated for C₁₁H₉O₂ ⁺ [M - C₈H₁₀NO]⁺: 173.0597; found: 173.0598, C₁₉H₂₀NO₃ ⁺ [M + H]⁺: 310.1438; found: 310.1457.

### (11) 1-phenyl-2-(((5-(3-(trifluoromethyl)phenyl)furan-2-yl)methyl)amino)ethanol; (Compound 10a)

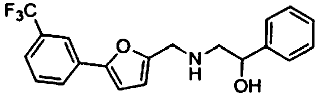

Compound 10a was prepared as a white solid in 63% yield using 5-(3-(trifluoromethyl)phenyl)furan-2-carbaldehyde (10) (130 mg, 0.54 mmol) and 2-amino-1-phenylethanol (74 mg, 0.54 mmol) according to the same procedure as described in general synthesis method B.

*R*_{f} = 0.31 (CH₂Cl₂/MeOH = 15:1); ¹H NMR (600 MHz, CDCl₃) δ 7.87 (s, 1H), 7.81 - 7.76 (m, 1H), 7.50-7.47(m, 2H), 7.40 - 7.31 (m, 4H), 7.30 - 7.26 (m, 1H), 6.66 (d, J = 3.3 Hz, 1H), 6.29 (d, J = 3.3 Hz, 1H), 4.75 (dd, J = 8.8, 3.6 Hz, 1H), 3.92 (d, J = 15.1 Hz, 1H), 3.89 (d, J = 15.1 Hz, 1H), 2.98 (dd, J = 12.2, 3.6 Hz, 1H), 2.79 (dd, J = 12.2, 8.8 Hz, 1H). HRMS (ESI) m/z calculated for C₁₂H₈F₃O ⁺ [M - C₈H₁₀NO]⁺: 225.0522; found: 225.0518, C₂₀H₁₉F₃NO₂ ⁺ [M + H]⁺: 362.1363; found: 362.1359.

### (12) 2-(((5-(3-fluorophenyl)furan-2-yl)methyl)amino)-1-phenylethanol; (Compound 11a)

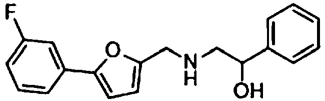

Compound 11a was prepared in 56% yield as a yellow solid using 5-(3-fluorophenyl)furan-2-carbaldehyde (11) (136 mg, 0.71 mmol) and 2-amino-1-phenylethanol (98 mg, 0.71 mmol) according to the same procedure as described in general synthesis method B.

*R*_{f} = 0.31 (CH₂Cl₂/MeOH = 15:1); ¹H NMR (600 MHz, CDCl₃) δ 7.40 (d, J = 8.1 Hz, 1H), 7.38 - 7.29 (m, 6H), 7.29 - 7.27 (m, 1H), 6.93 (td, J = 8.1, 2.2 Hz, 1H), 6.59 (d, J = 3.2 Hz, 1H), 6.26 (d, J = 3.2 Hz, 1H), 4.74 (dd, J = 8.8, 3.6 Hz, 1H), 3.90 (d, J = 15.1 Hz, 1H), 3.87 (d, J = 15.1 Hz, 1H), 2.97 (dd, J = 12.3, 3.6 Hz, 1H), 2.78 (dd, J = 12.3, 8.8 Hz, 1H), 1.70 (br, 1H). HRMS (ESI) m/z calculated for C₁₁H₈FO ⁺ [M - C₈H₁₀NO]⁺: 175.0554; found: 175.0558, C₁₉H₁₉FNO₂ ⁺ [M + H]⁺: 312.1395; found: 312.1395.

### (13) 1-(3-(5-(((2-hydroxy-2-phenylethyl)amino)methyl)furan-2-yl)phenyl)ethenone; (Compound 12a)

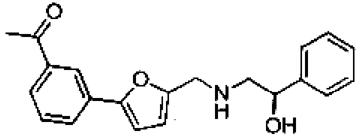

2-amino-1-phenylethanol (38 mg, 0.28 mmol) was added to a solution in which 5-(3-acetylphenyl)furan-2-carbaldehyde (12) (60 mg, 0.28 mmol) was dissolved in methanol (5 mL). The reaction mixture was stirred at room temperature for 1 hour and then cooled to 0 °C. NaBH₄ (8 mg, 0.22 mmol) was added with stirring, and the mixture was stirred at 0 °C for 30 minutes. The reaction was stopped with distilled water, and the methanol was removed under reduced pressure. The residue was diluted with ethyl acetate, the organic layer was washed with a saturated sodium bicarbonate aqueous solution and brine, dried with magnesium sulfate, and concentrated under reduced pressure. The residue was purified by silica gel column chromatography (CH₂Cl₂/MeOH=97:3) to obtain Compound 12a as a yellow solid (21 mg, 22%).

*R*_{f} = 0.21 (CH₂Cl₂/MeOH = 40:1); ¹H NMR (600 MHz, CDCl₃) δ 8.21 (t, J = 1.6 Hz, 1H), 7.82 (dd, J = 7.8, 1.6 Hz, 2H), 7.47 (t, J = 7.8 Hz, 1H), 7.39 - 7.30 (m, 4H), 7.30 - 7.26 (m, 1H), 6.67 (d, J = 3.3 Hz, 1H), 6.29 (d, J = 3.3 Hz, 1H), 4.75 (dd, J = 8.9, 3.6 Hz, 1H), 3.92 (d, J = 15.1 Hz, 1H), 3.90 (d, J = 15.1 Hz, 1H), 2.98 (dd, J = 12.3, 3.6 Hz, 1H), 2.79 (dd, J = 12.3, 8.9 Hz, 1H), 2.64 (s, 3H). HRMS (ESI) m/z calculated for C₁₃H₁₁O₂ ⁺ [M - C₈H₁₀NO]⁺: 199.0754; found: 199.0760, C₂₁H₂₂NO₃ ⁺ [M + H]⁺: 336.1594; found: 336.1609.

### (14) 2-(((5-(3-(1-hydroxyethyl)phenyl)furan-2-yl)methyl)amino)-1-phenylethanol; (Compound 12b)

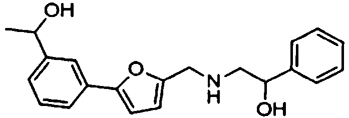

2-amino-1-phenylethanol (38 mg, 0.28 mmol) was added to a methanol (5 mL) solution of 5-(3-acetylphenyl)furan-2-carbaldehyde (12) (60 mg, 0.28 mmol). The reaction mixture was stirred at room temperature for 1 hour and then cooled to 0 °C. NaBH₄ (8 mg, 0.22 mmol) was added with stirring, and the reaction mixture was stirred at 0 °C for 30 minutes. The reaction was stopped with distilled water, and the methanol was removed under reduced pressure. The residue was diluted with ethyl acetate, the organic layer was washed with a saturated sodium bicarbonate aqueous solution and brine, dried with magnesium sulfate, and concentrated under reduced pressure. The residue was purified by silica gel column chromatography (CH₂Cl₂/MeOH=97:3) to obtain Compound 12b as a yellow solid (38 mg, 40%).

*R*_{f} = 0.18 (CH₂Cl₂/MeOH = 40:1); ¹H NMR (600 MHz, CDCl₃) δ 7.67 (s, 1H), 7.54 (d, J = 7.8 Hz, 1H), 7.38 - 7.29 (m, 5H), 7.28 - 7.22 (m, 2H), 6.59 (d, J = 3.2 Hz, 1H), 6.25 (d, J = 3.2 Hz, 1H), 4.93 (q, J = 6.5 Hz, 1H), 4.74 (dd, J = 8.9, 3.5 Hz, 1H), 3.89 (d, J = 15.3 Hz, 1H), 3.87 (d, J = 15.3 Hz, 1H), 2.97 (dd, J = 12.3, 3.5 Hz, 1H), 2.78 (dd, J = 12.3, 8.9 Hz, 1H), 2.04 (br, 2H), 1.52 (d, J = 6.5 Hz, 3H). HRMS (ESI) m/z calculated for C₁₃H₁₃O₂ ⁺ [M - C₈H₁₀NO]⁺: 201.0910; found: 201.0908, C₂₁H₂₃NO₃Na ⁺ [M + Na]⁺: 360.1570; found: 360.1551.

### (15) 2-(((5-(3,4-difluorophenyl)furan-2-yl)methyl)amino)-1-phenylethanol; (Compound 13a)

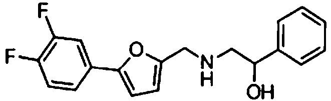

Compound 13a was prepared as a white solid in 45% yield using 5-(3,4-difluorophenyl)furan-2-carbaldehyde (13) (136 mg, 0.65 mmol) and 2-amino-1-phenylethanol (89 mg, 0.65 mmol) according to the same procedure as described in general synthesis method B.

*R*_{f} = 0.31 (CH₂Cl₂/MeOH = 15 : 1); ¹H NMR (600 MHz, CDCl₃) δ 7.42 (ddd, J = 11.4, 7.6, 2.1 Hz, 1H), 7.38 - 7.31 (m, 5H), 7.28 (dt, J = 4.5, 2.0 Hz, 1H), 7.15 (dt, J = 9.9, 8.4 Hz, 1H), 6.52 (d, J = 3.3 Hz, 1H), 6.25 (d, J = 3.3 Hz, 1H), 4.74 (dd, J = 8.8, 3.6 Hz, 1H), 3.88(d, J = 15.4 Hz, 1H), 3.86 (d, J = 15.4 Hz, 1H), 3.39 (br, 1H), 2.97 (dd, J = 12.3, 3.6 Hz, 1H), 2.78 (dd, J = 12.3, 8.8 Hz, 1H). HRMS (ESI) m/z calculated for C₁₁H₇F₂O ⁺ [M - C₈H₁₀NO]⁺: 193.0459; found: 193.0460, C₁₉H₁₈F₂NO₂ ⁺ [M + H]⁺: 330.1300; found: 330.1293.

### (16) 2-(((5-(3,4-dichlorophenyl)furan-2-yl)methyl)amino)-1-phenylethanol; (Compound 14a)

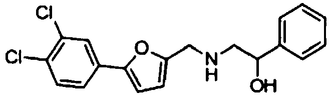

Compound 14a was prepared as a white solid in 47% yield using 5-(3,4-dichlorophenyl)furan-2-carbaldehyde (14) (145 mg, 0.60 mmol) and 2-amino-1-phenylethanol (82 mg, 0.60 mmol) according to the same procedure as described in general synthesis method B.

*R*_{f} = 0.31 (CH₂Cl₂/MeOH = 15:1); ¹H NMR (600 MHz, CDCl₃) δ 7.71 (d, J = 1.4 Hz, 1H), 7.47 - 7.39 (m, 2H), 7.38 - 7.32 (m, 4H), 7.31 - 7.27 (m, 1H), 6.59 (d, J = 3.3 Hz, 1H), 6.27 (d, J = 3.3 Hz, 1H), 4.75 (dd, J = 8.8, 3.5 Hz, 1H), 3.89 (d, J = 15.4 Hz, 1H), 3.87 (d, J = 15.4 Hz, 1H), 3.38 (br, 1H), 2.96 (dd, J = 12.2, 3.5 Hz, 1H), 2.79 (dd, J = 12.2, 8.8 Hz, 1H). HRMS (ESI) m/z calculated for C₁₁H₇Cₗ₂O ⁺ [M - C₈H₁₀NO]⁺: 224.9868; found: 224.9855, C₁₉H₁₈Cl₂NO₂ ⁺ [M + H]⁺: 362.0709; found: 362.0688.

### (17) 2-(((5-(3,4-dimethoxyphenyl)furan-2-yl)methyl)amino)-1-phenylethanol; (Compound 15a)

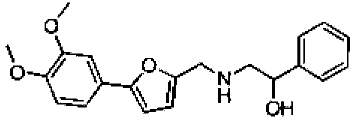

Compound 15a was prepared as a white solid in 56% yield using 5-(3,4-dimethoxyphenyl)furan-2-carbaldehyde (15) (147 mg, 0.63 mmol) and 2-amino-1-phenylethanol (86 mg, 0.63 mmol) according to the same procedure as described in general synthesis method B.

*R*_{f} = 0.26 (CH₂Cl₂/MeOH = 15:1); ¹H NMR (600 MHz, CDCl₃) δ 7.39 - 7.31 (m, 4H), 7.30 - 7.27 (m, 1H), 7.20 (dd, J = 8.4, 1.9 Hz, 1H), 7.15 (d, J = 1.9 Hz, 1H), 6.88 (d, J = 8.4 Hz, 1H), 6.45 (d, J = 3.2 Hz, 1H), 6.23 (d, J = 3.2 Hz, 1H), 4.74 (dd, J = 8.9, 3.5 Hz, 1H), 3.93 (s, 3H), 3.90 (s, 3H), 3.89 (d, J = 14.8 Hz, 1H), 3.87 (d, J = 14.8 Hz, 1H), 3.48 (br, 1H), 2.99 (dd, J = 12.3, 3. Hz, 1H), 2.78 (dd, J = 12.3, 8.9 Hz, 1H). HRMS (ESI) m/z calculated for C₁₃H₁₃O₃ ⁺ [M - C₈H₁₀NO]⁺: 217.0859; found: 217.0851, C₂₁H₂₃NO₄Na ⁺ [M + Na]⁺: 376.1519; found: 376.1505.

### (18) 2-(((5-(3-fluoro-4-methoxyphenyl)furan-2-yl)methyl)amino)-1-phenylethanol; (Compound 16a)

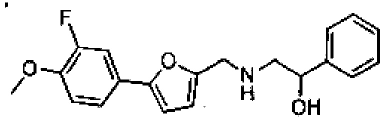

Compound 16a was prepared as a white solid in 67% yield using 5-(3-fluoro-4-methoxyphenyl)furan-2-carbaldehyde (16) (100 mg, 0.45 mmol) and 2-amino-1-phenylethanol (62 mg, 0.45 mmol) according to the same procedure as described in general synthesis method B.

*R*_{f} = 0.26 (CH₂Cl₂/MeOH = 15:1); ¹H NMR (600 MHz, CDCl₃) δ 7.40 - 7.32 (m, 6H), 7.29 - 7.27 (m, 1H), 6.96 (t, J = 8.7 Hz, 1H), 6.45 (d, J = 3.2 Hz, 1H), 6.23 (d, J = 3.2 Hz, 1H), 4.74 (dd, J = 8.8, 3.6 Hz, 1H), 3.91 (s, 3H), 3.88 (d, J = 15.0 Hz, 1H), 3.86 (d, J = 15.0 Hz, 1H), 3.48 (br, 1H), 2.97 (dd, J = 12.2, 3.6 Hz, 1H), 2.78 (dd, J = 12.2, 8.8 Hz, 1H). HRMS (ESI) m/z calculated for C₁₂H₁₀FO₂ ⁺ [M - C₈H₁₀NO]⁺: 205.0659; found: 205.0673, C₂₀H₂₀FNO₃Na ⁺ [M + Na]⁺: 364.1319; found: 364.1331.

### (19) 2-(((5-(4-methoxy-3-methylphenyl)furan-2-yl)methyl)amino)-1-phenylethanol; (Compound 17a)

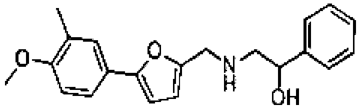

Compound 17a was prepared as a white solid in 56% yield using 5-(4-methoxy-3-methylphenyl)furan-2-carbaldehyde (17) (150 mg, 0.69 mmol) and 2-amino-1-phenylethanol (95 mg, 0.69 mmol) according to the same procedure as described in general synthesis method B.

*R*_{f} = 0.26 (CH₂Cl₂/MeOH = 15:1); ¹H NMR (600 MHz, CDCl₃) δ 7.46 - 7.40 (m, 2H), 7.38 - 7.31 (m, 4H), 7.29 - 7.27 (m, 1H), 6.82 (d, J = 8.4 Hz, 1H), 6.41 (d, J = 3.2 Hz, 1H), 6.21 (d, J = 3.2 Hz, 1H), 4.73 (dd, J = 8.9, 3.5 Hz, 1H), 3.88 (d, J = 14.8 Hz, 1H), 3.86 (d, J = 14.8 Hz, 1H), 3.85 (s, 3H), 3.50(s, 1H) 2.98 (dd, J = 12.3, 3.5 Hz, 1H), 2.77 (dd, J = 12.3, 8.9 Hz, 1H), 2.25 (s, 3H). HRMS (ESI) m/z calculated for C₁₃H₁₃O₂⁺ [M - C₈H₁₀NO]⁺: 201.0910; found: 201.0898, C₂₁H₂₃NO₃Na⁺ [M + Na]⁺: 360.1570; found: 360.1554.

### <Synthesis Example 2> Synthesis of β-amino alcohol derivative (2)

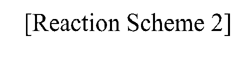

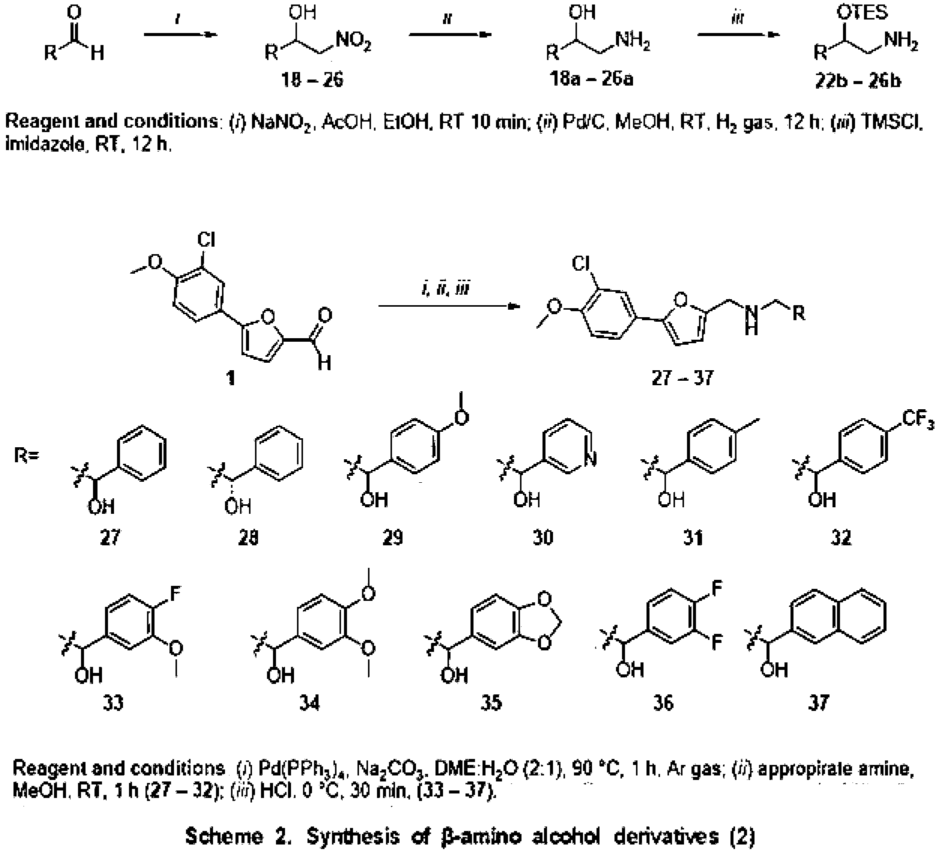

### 2-1. General synthesis method C: Synthesis of 2-amino-1-phenylethanol derivative

Step 1: Nitromethane (1.0 equivalent), aqueous sodium hydroxide solution (10 M, 1.0 equivalent), and acetic acid (1.0 equivalent) were added to a solution in which aldehyde (1.0 mmol) was dissolved in ethanol (4 mL). The reaction mixture was stirred at room temperature for 10 minutes. Subsequently, 1 M HCl was added to the reaction mixture until the pH became acidic, and extracted with dichloromethane (30 mL twice). The organic layers were combined, dried with magnesium sulfate, filtered, and the solvent was evaporated. The residue was purified by silica gel column chromatography (CH₂Cl₂/MeOH) to obtain the desired pure product.

Step 2: 10% Pd/C (10 wt%) was added to a solution in which the nitro intermediate (1.0 mmol) produced in step 1 was dissolved in methanol (10 mL). The reaction mixture was stirred at room temperature under a hydrogen stream for 12 hours. The reaction mixture was filtered through a Celite pad, and the filtrate was concentrated under reduced pressure to obtain the product. The product was used in the next step without further purification.

Step 3: Imidazole (5.0 equivalents) and chlorotrimethylsilane (2.5 equivalents) were added to a solution in which the alcohol intermediate (1.0 mmol) produced in step 2 was dissolved in dichloromethane (DCM, 10 mL). The reaction mixture was stirred at room temperature for 12 hours. Afterward, the reaction mixture was diluted with water and extracted with dichloromethane (30 mL twice). The organic layers were combined, dried with magnesium sulfate, filtered, and the solvent evaporated. The residue was purified by silica gel column chromatography (CH₂Cl₂/MeOH) to obtain the desired pure product.

### (1) 1-(4-Methoxyphenyl)-2-nitroethanol; (Intermediate 18)

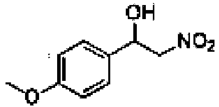

Intermediate 18 was prepared as a pale yellow liquid in 74% yield using 4-methoxybenzaldehyde (268 µL, 2.20 mmol) according to the same procedure as described in general synthesis method C (step 1).

*R*_{f} = 0.4 (Hexane/EtOAc = 7:3); ¹H NMR (600 MHz, CDCl₃) δ 7.32 (d, J = 8.7 Hz, 2H), 6.92 (d, J = 8.7 Hz, 2H), 5.40 (dd, J = 9.7, 3.0 Hz, 1H), 4.60 (dd, J = 13.2, 9.7 Hz, 1H), 4.48 (dd, J = 13.2, 3.0 Hz, 1H), 3.81 (s, 3H), 2.80 (s, 1H).

### (2) 2-Amino-1-(4-methoxyphenyl)ethanol; (Intermediate 18a)

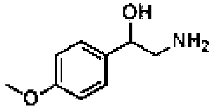

Intermediate 18a was prepared in the form of a yellow gel in 92% yield using 1-(4-methoxyphenyl)-2-nitroethanol (180 mg, 0.91 mmol) according to the same procedure as described in general synthesis method C (step 2).

*R*_{f} = 0.01 (Hexane/EtOAc = 7:3); ¹H NMR (600 MHz, CDCl₃) δ 7.27 (d, J = 7.8 Hz, 2H), 6.88 (d, J = 7.8 Hz, 2H), 4.60-4.57 (m, 1H), 3.80 (s, 3H), 2.95 (d, J = 11.8 Hz, 1H), 2.80 (d, J = 11.8, 8.2 Hz, 1H), 2.19 (brs, 3H).

### (3) 2-Nitro-1-(pyridin-3-yl)ethanol; (Intermediate 19)

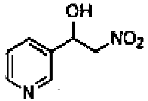

Intermediate 19 was prepared as an orange liquid in 94% yield using nicotinaldehyde (413 µL, 4.40 mmol) according to the same procedure as described in general synthesis method C (step 1).

*R*_{f} = 0.05 (Hexane/EtOAc = 7:3); ¹H NMR (600 MHz, CDCl₃) δ 8.49 (d, J = 1.9 Hz, 1H), 8.42 (dd, J = 4.9, 1.9 Hz, 1H), 7.81 (dt, J = 7.9, 1.9 Hz, 1H), 7.33 (dd, J = 7.9, 4.9 Hz, 1H), 5.50 (dd, J = 9.6, 3.3 Hz, 1H), 4.61 (dd, J = 12.9, 9.6 Hz, 1H), 4.52 (dd, J = 12.9, 3.3 Hz, 1H).

### (4) 2-Amino-1-(pyridin-3-yl)ethanol; (Intermediate 19a)

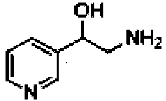

Intermediate 19a was prepared in the form of a brown gel in 92% yield using 2-nitro-1-(pyridin-3-yl)ethanol (180 mg, 0.91 mmol) according to the same procedure as described in general synthesis method C (step 2).

*R*_{f} = 0.01 (Hexane/EtOAc = 7:3); ¹H NMR (600 MHz, DMSO) δ 8.56 (d, J = 1.7 Hz, 1H), 8.48 (dd, J = 4.8, 1.7 Hz, 1H), 7.79 - 7.75 (dt, J = 7.8, 1.7 Hz, 1H), 7.37 (dt, J = 7.8, 4.8 Hz, 1H), 4.74 (dd, J = 8.7, 3.8 Hz, 1H), 2.92 (dd, J = 12.8, 3.8 Hz, 1H), 2.80 (dd, J = 12.8, 8.7 Hz, 1H).

### (5) 2-nitro-1-(p-tolyl)ethanol; (Intermediate 20)

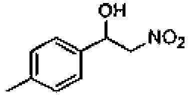

Intermediate 20 was prepared as a white solid in 78% yield using 4-methylbenzaldehyde (519 µL, 4.40 mmol) according to the same procedure as described in general synthesis method C (step 1).

*R*_{f} = 0.6 (Hexane/EtOAc = 7:3); ¹H NMR (600 MHz, CDCl₃) δ 7.27 (d, J = 8.0 Hz, 2H), 7.19 (d, J = 8.0 Hz, 2H), 5.40 (dd, J = 9.7, 2.9 Hz, 1H), 4.58 (dd, J = 13.3, 9.7 Hz, 1H), 4.47 (dd, J = 13.3, 2.9 Hz, 1H), 2.35 (s, 3H).

### (6) 2-Amino-1-(p-tolyl)ethanol; (Compound 20a)

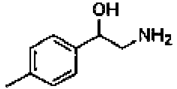

Intermediate 20a was prepared in the form of a yellow gel in 99% yield using 2-nitro-1-(p-tolyl)ethanol (672 mg, 3.44 mmol) according to the same procedure as described in general synthesis method C (step 2).

*R*_{f} = 0.05 (Hexane/EtOAc = 7:3).; ¹H NMR (600 MHz, CDCl₃) δ 7.22 (d, J = 7.5 Hz, 2H), 7.14 (d, J = 7.5 Hz, 2H), 4.62 (dd, J = 7.7, 3.5 Hz, 1H), 2.96 (dd, J = 12.6, 3.5 Hz, 1H), 2.87 (brs, 2H), 2.85 - 2.78 (m, 1H), 2.33 (s, 3H).

### (7) 2-Nitro-1-(4-(trifluoromethyl)phenyl)ethanol; (Intermediate 21)

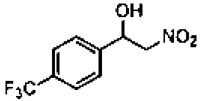

Intermediate 21 was prepared as a white solid in 68% yield using 4-(trifluoromethyl)benzaldehyde (600 µL, 4.40 mmol) according to the same procedure as described in general synthesis method C (step 1).

*R*_{f} = 0.7 (Hexane/EtOAc = 7:3); ¹H NMR (600 MHz, CDCl₃) δ 7.67 (d, J = 8.2 Hz, 2H), 7.54 (d, J = 8.2 Hz, 2H), 5.53 (dd, J = 9.4, 3.0 Hz, 1H), 4.59 (dd, J = 13.5, 9.4 Hz, 1H), 4.53 (dd, J = 13.5, 3.0 Hz, 1H), 3.23 (brs, 1H).

### (8) 2-Amino-1-(4-(trifluoromethyl)phenyl)ethanol; (Intermediate 21a)

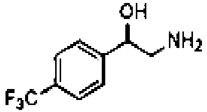

Intermediate 21a was prepared in the form of a brown gel in 99% yield using 2-nitro-1-(4-(trifluoromethyl)phenyl)ethanol (706 mg, 3.00 mmol) according to the same procedure as described in general synthesis method C (step 2).

*R*_{f} = 0.05 (Hexane/EtOAc = 7:3); ¹H NMR (600 MHz, CDCl₃) δ 7.59 (d, J = 8.0 Hz, 2H), 7.46 (d, J = 8.0 Hz, 2H), 4.73 (dd, J = 7.9, 3.4 Hz, 1H), 3.09 - 3.03 (m, 1H), 2.79 (dd, J = 12.8, 7.9 Hz, 2H), 2.73 (brs, 2H).

### (9) 1-(4-Fluoro-3-methoxyphenyl)-2-nitroethanol; (Intermediate 22)

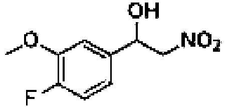

Intermediate 22 was prepared as a white solid in 70% yield using 4-fluoro-3-methoxybenzaldehyde (678 mg, 4.40 mmol) according to the same procedure described in general synthesis method C (step 1).

*R*_{f} = 0.6 (Hexane/EtOAc = 7:3); ¹H NMR (600 MHz, CDCl₃) δ 7.09 (dd, J = 10.9, 8.0 Hz, 1H), 7.04 (dd, J = 8.0, 1.9 Hz, 1H), 6.91-6.89 (m, 1H), 5.43 (d, J = 9.5 Hz, 1H), 4.58 (dd, J = 13.4, 9.5 Hz, 1H), 4.50 (dd, J = 13.4, 3.0 Hz, 1H), 3.91 (s, 3H), 2.91 (brs, 1H).

### (10) 2-Amino-1-(4-fluoro-3-methoxyphenyl)ethanol; (Intermediate 22a)

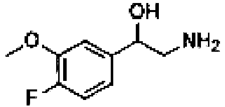

Intermediate 22a was prepared in the form of a yellow gel in 99% yield using 1-(4-fluoro-3-methoxyphenyl)-2-nitroethanol (662 mg, 3.07 mmol) according to the same procedure described in general synthesis method C (step 2).

*R*_{f} = 0.1 (Hexane/EtOAc = 7:3); ¹H NMR (600 MHz, CDCl₃) δ 7.03-6.99 (m, 2H), 6.83-6.80 (m, 1H), 4.65 (dd, J = 8.1, 3.3 Hz, 1H), 3.86 (s, 3H), 3.11 (brs, 2H), 3.05 - 2.96 (m, 1H), 2.80 (dd, J = 11.9, 8.1 Hz, 1H).

### (11) 2-(4-Fluoro-3-methoxyphenyl)-2-((triethylsilyl)oxy)ethanamine; (Intermediate 22b)

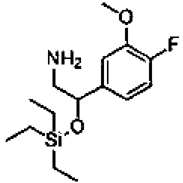

Intermediate 22b was prepared as a brown liquid in 57% yield using 2-amino-1-(4-fluoro-3-methoxyphenyl)ethanol (150 mg, 0.81 mmol) according to the same procedure described in general synthesis method C (step 3).

*R*_{f} = 0.1 (Hexane/EtOAc = 7:3); ¹H NMR (600 MHz, CDCl₃) δ 7.00 (dd, J = 11.1, 8.3 Hz, 1H), 6.97 (dd, J = 8.3, 1.9 Hz, 1H), 6.79 (ddd, J = 8.3, 4.2, 1.9 Hz, 1H), 4.60 (t, J = 5.4 Hz, 1H), 3.88 (s, 3H), 2.80 (d, J = 5.4 Hz, 3H), 1.36 (brs, 2H), 0.90 (t, J = 7.9 Hz, 9H), 0.56 (q, J = 7.9 Hz, 6H).

### (12) 1-(3,4-Dimethoxyphenyl)-2-nitroethanol; (Intermediate 23)

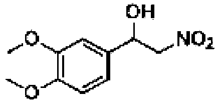

Intermediate 23 was prepared as a yellow solid in 66% yield using 3,4-dimethoxybenzaldehyde (731 mg, 4.40 mmol) according to the same procedure described in general synthesis method C (step 1).

*R*_{f} = 0.3 (Hexane/EtOAc = 7:3) ¹H NMR (600 MHz, CDCl₃) δ 6.92 (s, 1H), 6.91 (d, J = 8.3 Hz, 1H), 6.86 (d, J = 8.3 Hz, 1H), 5.39 (dt, J = 9.7, 3.0 Hz, 1H), 4.60 (dd, J = 13.2, 9.7 Hz, 1H), 4.48 (dd, J = 13.2, 3.0 Hz, 1H), 3.88 (s, 3H), 3.86 (s, 3H), 2.92 (brs, 1H).

### (13) 2-Amino-1-(3,4-dimethoxyphenyl)ethanol; (Intermediate 23a)

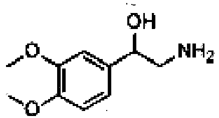

Intermediate 23a was prepared as a yellow gel in 99% yield using 1-(3,4-dimethoxyphenyl)-2-nitroethanol (665 mg, 2.92 mmol) according to the same procedure described in general synthesis method C (step 2).

*R*_{f} = 0.1 (Hexane/EtOAc = 7:3); ¹H NMR (600 MHz, DMSO) δ 6.94 (d, J = 1.6 Hz, 1H), 6.90 (d, J = 8.2 Hz, 1H), 6.85 (dd, J = 8.2, 1.6 Hz, 1H), 4.57 (dd, J = 8.7, 3.7 Hz, 1H), 3.75 (s, 3H), 3.72 (s, 3H), 2.81 (dd, J = 12.8, 3.7 Hz, 1H), 2.70 (dd, J = 12.8, 8.7 Hz, 1H).

### (14) 2-(3,4-Dimethoxyphenyl)-2-((triethylsilyl)oxy)ethanamine; (Intermediate 23b)

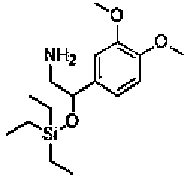

Intermediate 23b was prepared as a pale yellow liquid in 33% yield using 2-amino-1-(3,4-dimethoxyphenyl)ethanol (319 mg, 1.62 mmol) according to the same procedure described in general synthesis method C (Step 3).

*R*_{f} = 0.1 (Hexane/EtOAc = 7:3); ¹H NMR (600 MHz, CDCl₃) δ 6.89 (s, 1H), 6.82-6.80 (m, 2H), 4.59 (dd, J = 6.2, 4.8 Hz, 1H), 3.88 (s, 3H), 3.87 (s, 3H), 2.84 - 2.77 (m, 2H), 1.44 (s, 3H), 0.90 (t, J = 7.9 Hz, 9H), 0.55 (q, J = 7.9 Hz, 6H).

### (15) 1-(Benzo[d][1,3]dioxol-5-yl)-2-nitroethanol; (Intermediate 24)

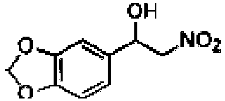

Intermediate 24 was prepared as a white solid in 78% yield using benzo[d][1,3]dioxol-5-carbaldehyde (660 mg, 4.40 mmol) according to the same procedure described in general synthesis method C (step 1).

*R*_{f} = 0.5 (Hexane/EtOAc = 7:3); ¹H NMR (600 MHz, CDCl₃) δ 6.89 (d, J = 1.6 Hz, 1H), 6.85 (dd, J = 8.0, 1.6 Hz, 1H), 6.81 (d, J = 8.0 Hz, 1H), 5.98 (s, 2H), 5.37 (dt, J = 9.6, 3.0 Hz, 1H), 4.57 (dd, J = 13.3, 9.6 Hz, 1H), 4.47 (dd, J = 13.3, 3.0 Hz, 1H), 2.76 (brs, 1H).

### (16) 2-Amino-1-(benzo[d][1,3]dioxol-5-yl)ethanol; (Intermediate 24a)

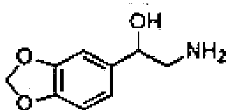

Intermediate 24a was prepared as a colorless gel in 99% yield using 1-(benzo[d][1,3]dioxol-5-yl)-2-nitroethanol (680 mg, 3.22 mmol) according to the same procedure described in general synthesis method C (step 2).

*R*_{f} = 0.1 (Hexane/EtOAc = 7:3); ¹H NMR (600 MHz, CDCl₃) δ 6.86 (s, 1H), 6.80 (d, J = 7.8 Hz, 1H), 6.77 (d, J = 7.8 Hz, 1H), 5.94 (s, 2H), 4.54 (dd, J = 7.8, 4.0 Hz, 1H), 2.94 (dd, J = 12.7, 4.0 Hz, 1H), 2.77 (dd, J = 12.7, 7.8 Hz, 2H), 2.12 (brs, 2H).

### (17) 2-(Benzo[d][1,3]dioxol-5-yl)-2-((triethylsilyl)oxy)ethanamine; (Intermediate 24b)

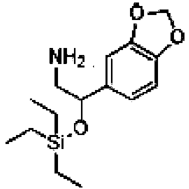

Intermediate 24b was prepared as a pale yellow liquid in 76% yield using 2-amino-1-(benzo[d][1,3]dioxol-5-yl)ethanol (293 mg, 1.62 mmol) according to the same procedure described in general synthesis method C (step 3).

*R*_{f} = 0.3 (Hexane/EtOAc = 5:5); ¹H NMR (600 MHz, CDCl₃) δ 6.83 (s, 1H), 6.75-6.70 (m, 2H), 5.94 (dd, J = 5.9, 1.4 Hz, 2H), 4.55 (t, J = 5.4 Hz, 1H), 2.77 (d, J = 5.4 Hz, 3H), 1.35 (brs, 2H), 0.90 (t, J = 7.9 Hz, 9H), 0.55 (m, 6H).

### (18) 2-(3,4-Difluorophenyl)-2-((triethylsilyl)oxy)ethanamine; (Intermediate 25b)

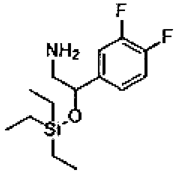

Intermediate 25b was prepared as a pale yellow liquid in 62% yield using 2-amino-1-(3,4-difluorophenyl)ethanol (280 mg, 1.62 mmol) according to the same procedure described in general synthesis method C (step 2).

*R*_{f} = 0.5 (Hexane/EtOAc = 5:5); ¹H NMR (600 MHz, CDCl₃) δ 7.17-7.13 (m, 1H), 7.12-7.08 (m, 1H), 7.02 - 6.99 (m, 1H), 4.61 (t, J = 5.28 Hz, 1H), 2.82-2.75 (m, 2H), 1.37 (brs, 2H), 0.90 (t, J = 8.0 Hz, 9H), 0.57-0.52 (m, 6H).

### (19) 1-(Naphthalen-2-yl)-2-nitroethanol; (Intermediate 26)

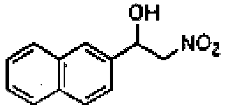

Intermediate 26 was prepared as a white solid in 78% yield using 2-naphthaldehyde (687 mg, 4.40 mmol) according to the same procedure described in general synthesis method C (step 1).

*R*_{f} = 0.6 (Hexane/EtOAc = 7:3); ¹H NMR (600 MHz, CDCl₃) δ 7.89 - 7.87 (m, 2H), 7.87 - 7.83 (m, 2H), 7.55 - 7.51 (m, 2H), 7.46 (dd, J = 8.5, 1.6 Hz, 1H), 5.61 (d, J = 9.5 Hz, 1H), 4.68 (dd, J = 13.4, 9.5 Hz, 1H), 4.59 (dd, J = 13.4, 3.0 Hz, 1H), 3.01 (brs, 1H).

### (20) 2-Amino-1-(naphthalen-2-yl)ethanol; (Intermediate 26a)

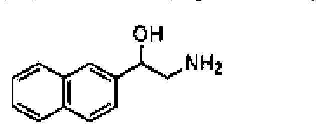

Intermediate 26a was prepared as a white solid in 67% yield using 1-(naphthalene-2-yl)-2-nitroethanol (687 mg, 3.43 mmol) according to the same procedure described in general synthesis method C (step 2).

*R*_{f} = 0.1 (Hexane/EtOAc = 7:3); ¹H NMR (600 MHz, CDCl₃) δ 7.85 - 7.82 (m, 4H), 7.50 - 7.45 (m, 3H), 4.81 (dd, J = 7.5, 4.1 Hz, 1H), 3.12 (dd, J = 12.8, 4.1 Hz, 1H), 2.91 (dd, J = 12.8, 7.5 Hz, 1H), 1.76 (brs, 2H).

### (21) 2-(Naphthalen-2-yl)-2-((triethylsilyl)oxy)ethanamine; (Intermediate 26b)

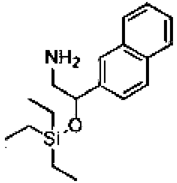

Intermediate 26b was prepared as a pale yellow liquid in 72% yield using 2-amino-1-(naphthalene-2-yl)ethanol (303 mg, 1.62 mmol) according to the same procedure as described in general synthesis method C (step 3).

*R*_{f} = 0.5 (Hexane/EtOAc = 5:5); ¹H NMR (600 MHz, CDCl₃) δ 7.84-7.80 (m, 3H), 7.77 (s, 1H), 7.48 - 7.44 (m, 3H), 4.82 (t, J = 5.3 Hz, 1H), 2.91 (d, J = 5.3 Hz, 2H), 1.49 (brs, 2H), 0.91 (t, J = 7.98 Hz 9H), 0.61 - 0.55 (m, 6H).

### (22) (R)-2-(((5-(3-chloro-4-methoxyphenyl)furan-2-yl)methyl)amino)-1-phenylethanol; (Compound 27)

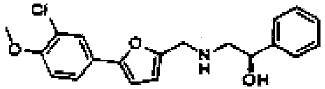

Compound 27 was prepared as a white solid in 34% yield using 5-(4-methoxyphenyl)furan-2-carbaldehyde (31b) (191 mg, 0.80 mmol) and (R)-2-amino-1-phenylethanol (110 mg, 0.80 mmol) according to the same procedure as described in general synthesis method B.

*R_{f}* = 0.1 (Hexane/EtOAc = 7:3); ¹H NMR (600 MHz, CDCl₃) δ 7.65 (d, J = 2.1 Hz, 1H), 7.48 (dd, J = 8.6, 2.1 Hz, 1H), 7.38 - 7.32 (m, 4H), 7.28 (dt, J = 6.6, 1.8 Hz, 1H), 6.93 (d, J = 8.6 Hz, 1H), 6.46 (d, J = 3.2 Hz, 1H), 6.23 (d, J = 3.2 Hz, 1H), 4.74 (dd, J = 8.8, 3.5 Hz, 1H), 3.92 (s, 2H), 3.88 (d, J = 15.6 Hz, 1H), 3.85 (d, J = 15.6 Hz, 1H), 2.97 (dd, J = 12.3, 3.5 Hz, 1H), 2.78 (dd, J = 12.3, 8.8 Hz, 1H), 2.20 (br, 2H); HRMS (ESI) m/z calculated for C₁₂H₁₀O₂⁺ [M - C₈H₁₀NO]⁺: 221.0364; found: 221.0358.

### (23) (S)-2-(((5-(3-chloro-4-methoxyphenyl)furan-2-yl)methyl)amino)-1-phenylethanol; (Compound 28)

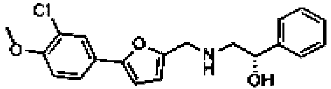

Compound 28 was prepared as a white solid in 14% yield using 5-(4-methoxyphenyl)furan-2-carbaldehyde (31b) (126 mg, 0.53 mmol) and (S)-2-amino-1-phenylethanol (73 mg, 0.53 mmol) according to the same procedure as described in general synthesis method B.

*R*_{f} = 0.1 (Hexane/EtOAc = 7:3); ¹H NMR (600 MHz, CDCl₃) δ 7.65 (d, J = 2.1 Hz, 1H), 7.48 (dd, J = 8.6, 2.1 Hz, 1H), 7.37 - 7.33 (m, 4H), 7.28 (dt, J = 7.2, 1.8 Hz, 1H), 6.92 (d, J = 8.6 Hz, 1H), 6.46 (d, J = 3.2 Hz, 1H), 6.23 (d, J = 3.2 Hz, 1H), 4.75 (dd, J = 8.9, 3.5 Hz, 1H), 3.92 (s, 3H), 3.88 (d, J = 15.6 Hz, 1H), 3.86 (d, J = 15.6 Hz, 1H), 2.96 (dd, J = 12.3, 3.5 Hz, 1H), 2.78 (dd, J = 12.3, 8.9 Hz, 1H), 2.47 (br, 2H); HRMS (ESI) m/z calculated for C₁₂H₁₀O₂⁺ [M - C₈H₁₀NO]⁺: 221.0364; found: 221.0358.

### (24) 2-(((5-(3-chloro-4-methoxyphenyl)furan-2-yl)methyl)amino)-1-(4-methoxyphenyl)ethanol; (Compound 29)

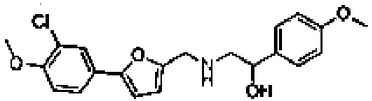

Compound 29 was prepared as a white solid in 32% yield using 5-(4-methoxyphenyl)furan-2-carbaldehyde (31b) (126 mg, 0.53 mmol) and 2-amino-1-(4-methoxyphenyl)ethanol (88 mg, 0.53 mmol) according to the same procedure as described in general synthesis method B.

*R*_{f} = 0.1 (Hexane/EtOAc = 7:3); ¹H NMR (600 MHz, CDCl₃) δ 7.65 (d, J = 2.1 Hz, 1H), 7.48 (dd, J = 8.6, 2.1 Hz, 1H), 7.28 (d, J = 8.6 Hz, 1H), 6.93 (d, J = 8.6 Hz, 1H), 6.88 (d, J = 8.6 Hz, 1H), 6.46 (d, J = 3.2 Hz, 1H), 6.23 (d, J = 3.2 Hz, 1H), 4.69 (dd, J = 8.9, 3.6 Hz, 1H), 3.92 (s, 3H), 3.88 (d, J = 14.4 Hz, 1H), 3.85 (d, J = 14.4 Hz, 1H), 3.79 (s, 2H), 2.92 (dd, J = 12.2, 3.6 Hz, 1H), 2.76 (dd, J = 12.2, 8.9 Hz, 1H), 1.71 (s, 1H). HRMS (ESI) m/z calculated for C₁₂H₁₀O₂⁺ [M - C₉H₁₂NO₂]⁺: 221.0364; found: 221.0361, C₂₁H₂₂ClNNaO₄⁺ [M + Na]⁺: 410.1092; found: 410.1108

### (25) 2-(((5-(3-chloro-4-methoxyphenyl)furan-2-yl)methyl)amino)-1-(pyridin-3-yl)ethanol; (Compound 30)

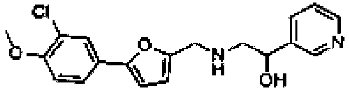

Compound 30 was prepared as a white solid in 35% yield using 5-(4-methoxyphenyl)furan-2-carbaldehyde (31b) (126 mg, 0.53 mmol) and 2-amino-1-(pyridine-3-yl)ethanol (73 mg, 0.53 mmol) according to the same procedure as described in general synthesis method B.

*R*_{f} = 0.01 (Hexane/EtOAc = 7:3); ¹H NMR (600 MHz, CDCl₃) δ 8.58 (d, J = 2.1 Hz, 1H), 8.51 (dd, J = 4.8, 1.8 Hz, 1H), 7.72 (dt, J = 7.9, 1.8 Hz, 1H), 7.64 (d, J = 2.4 Hz, 1H), 7.48 (dd, J = 8.6, 2.4 Hz, 1H), 7.27 (dd, J = 7.9, 4.8 Hz, 1H), 6.93 (d, J = 8.6 Hz, 1H), 6.46 (d, J = 3.2 Hz, 1H), 6.24 (d, J = 3.2 Hz, 1H), 4.75 (dd, J = 9.2, 3.5 Hz, 1H), 3.92 (s, 2H), 3.89 (d, J = 15.6 Hz, 1H), 3.87 (d, J = 15.6 Hz, 1 H), 2.99 (dd, J = 12.4, 3.6 Hz, 1H), 2.74 (dd, J = 12.4, 9.2 Hz, 1H), 2.34 (br, 2H). HRMS (ESI) m/z calculated for C₁₂H₁₀O₂⁺ [M - C₇H₉N₂O]⁺: 221.0364; found: 221.0384., C₁₉H₂₀ClN₂O₃⁺ [M + H]⁺: 359.1157; found: 359.1198

### (26) 2-(((5-(3-chloro-4-methoxyphenyl)furan-2-yl)methyl)amino)-1-(p-tolyl)ethanol; (Compound 31)

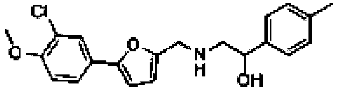

Compound 31 was prepared as a white solid in 53% yield using 5-(4-methoxyphenyl)furan-2-carbaldehyde (31b) (126 mg, 0.53 mmol) and 2-amino-1-(p-tolyl)ethanol (80 mg, 0.53 mmol) according to the same procedure as described in general synthesis method B.

*R*_{f} = 0.1 (Hexane/EtOAc = 7:3); ¹H NMR (600 MHz, CDCl₃) δ 7.60 (d, J = 2.1 Hz, 1H), 7.54 (dd, J = 8.5, 2.1 Hz, 1H), 7.19 (d, J = 8.0 Hz, 2H), 7.04 (d, J = 8.0 Hz, 2H), 6.86 (d, J = 8.5 Hz, 1H), 6.70 (d, J = 3.4 Hz, 1H), 6.42 (d, J = 3.4 Hz, 1H), 5.32 (dd, J = 10.1, 2.6 Hz, 1H), 4.39 (d, J = 15.0 Hz, 1H), 4.35 (d, J = 15.0 Hz, 1H), 3.87 (s, 3H), 3.17 - 3.08 (m, 2H), 2.27 (br, 2H). HRMS (ESI) m/z calculated for C₁₂H₁₀O₂⁺ [M - C₉H₁₂NO]⁺: 221.0364; found: 221.0381., C₂₁H₂₂ClNO₃Na⁺ [M + Na]⁺: 394.1192; found: 394.1209.

### (27) 2-(((5-(3-chloro-4-methoxyphenyl)furan-2-yl)methyl)amino)-1-(4-(trifluoromethyl)phenyl)ethanol; (Compound 32)

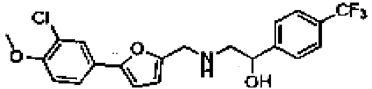

Compound 32 was prepared as a white solid in 26% yield using 5-(4-methoxyphenyl)furan-2-carbaldehyde (31b) (126 mg, 0.53 mmol) and 2-amino-1-(4-(trifluoromethyl)phenyl)ethanol (108 mg, 0.53 mmol) according to the same procedure as described in general synthesis method B.

*R*_{f} = 0.1 (Hexane/EtOAc = 7:3); ¹H NMR (600 MHz, CDCl₃) δ 7.64 (d, J = 1.6 Hz, 1H), 7.57 (d, J = 7.4 Hz, 2H), 7.47 (d, J = 7.4 Hz, 3H), 6.91 (d, J = 8.6 Hz, 1H), 6.45 (d, J = 2.7 Hz, 1H), 6.30 (s, 1H), 4.87 (s, 1H), 3.94 (s, 2H), 3.91 (s, 3H), 3.02 (d, J = 10.8 Hz, 1H), 2.81 - 2.72 (m, 1H), 2.54 (br, 2H). HRMS (ESI) m/z calculated for C₁₂H₁₀O₂⁺ [M - C₉H₉F₃NO]⁺: 221.0364; found: 221.0334.

### 2-2. General synthesis method D: Reductive amination reaction

5-(4-methoxyphenyl)furan-2-carbaldehyde (1.0 mmol) and a suitable amine (1.0 equivalent) were added to methanol (5 ml) and an anhydrous molecular sieve were added and stirred at room temperature. After 3 hours, NaBH₄ (3.0 equivalents) was added little by little with stirring in an ice bath and the mixture was stirred for 10 minutes. Then, concentrated HCl (13.0 equivalents) was added to the reaction mixture and stirred for 10 minutes. The reaction was stopped with an aqueous solution of saturated sodium bicarbonate, and methanol was removed under reduced pressure. The residue was diluted with EtOAc (30 mL), the organic layer was washed with water and brine, dried with anhydrous magnesium sulfate, and concentrated under reduced pressure. The residue was purified by silica gel column chromatography (DCM:MeOH) to obtain the desired pure product.

### (1) 2-(((5-(3-chloro-4-methoxyphenyl)furan-2-yl)methyl)amino)-1-(4-fluoro-3-methoxyphenyl)ethanol; (Compound 33)

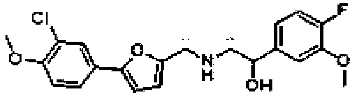

Compound 33 was prepared as a white solid in 22% yield using 5-(4-methoxyphenyl)furan-2-carbaldehyde (31b) (106 mg, 0.45 mmol) and 2-(4-fluoro-3-methoxyphenyl)-2-((triethylsilyl)oxy)ethanolamine (135 mg, 0.45 mmol) according to the same procedure as described in general synthesis method D.

*R*_{f} = 0.3 (DCM/MeOH = 95:5); ¹H NMR (600 MHz, CDCl₃) δ 7.65 (d, J = 1.7 Hz, 1H), 7.48 (dd, J = 8.6, 1.7 Hz, 1H), 7.04 - 7.00 (m, 2H), 6.93 (d, J = 8.6 Hz, 1H), 6.85 - 6.82 (m, 1H), 6.46 (d, J = 3.0 Hz, 1H), 6.24 (d, J = 3.0 Hz, 1H), 4.68 (dd, J = 9.0, 3.2 Hz, 1H), 3.92 (s, 3H), 3.88 (s, 3H), 3.88 - 3.87 (m, 2H), 2.95 (dd, J = 12.0, 3.2 Hz, 1H), 2.73 (dd, J = 12.0, 9.0 Hz, 1H), 1.68 (br, 2H). HRMS (ESI) m/z calculated for C₁₂H₁₀O₂⁺ [M - C₉H₁₁FNO₂]⁺: 221.0364; found: 221.0330.

### (2) 2-(((5-(3-chloro-4-methoxyphenyl)furan-2-yl)methyl)amino)-1-(3,4-dimethoxyphenyl)ethanol; (Compound 34)

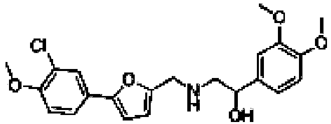

Compound 34 was prepared as a white solid in 12% yield using 5-(4-methoxyphenyl)furan-2-carbaldehyde (31b) (106 mg, 0.45 mmol) and 2-(3,4-dimethoxyphenyl)-2-((triethylsilyl)oxy)ethanolamine (140 mg, 0.45 mmol) according to the same procedure as described in general synthesis method D.

*R*_{f} = 0.1 (DCM/MeOH = 98:2); ¹H NMR (600 MHz, CDCl₃) δ 7.65 (d, J = 2.0 Hz, 1H), 7.48 (dd, J = 8.5, 2.0 Hz, 1H), 6.93 (d, J = 8.5 Hz, 1H), 6.93 (s, 1H), 6.87 (d, J = 8.2 Hz, 1H), 6.83 (d, J = 8.2 Hz, 1H), 6.46 (d, J = 3.1 Hz, 1H), 6.23 (d, J = 3.1 Hz, 1H), 4.69 (dd, J = 8.7, 3.5 Hz, 1H), 3.92 (s, 3H), 3.89 - 3.84 (m, 2H), 3.87 (s, 3H), 3.86 (s, 3H), 2.94 (dd, J = 12.1, 3.5 Hz, 1H), 2.77 (dd, J = 12.1, 8.7 Hz, 1H), 1.64 (br, 2H). HRMS (ESI) m/z calculated for C₁₂H₁₀O₂⁺ [M - C₁₀H₁₄NO₃]⁺: 221.0364; found: 221.0325.

### (3) 1-(benzo[d][1,3]dioxol-5-yl)-2-(((5-(3-chloro-4-methoxyphenyl)furan-2-yl)methyl)amino)ethanol; (Compound 35)

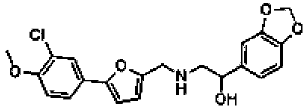

Compound 35 was prepared as a white solid in 50% yield using 5-(4-methoxyphenyl)furan-2-carbaldehyde (31b) (106 mg, 0.45 mmol) and 2-(benzo[d][1,3]dioxol-5-yl)-2-((triethylsilyl)oxy)ethanolamine (133 mg, 0.45 mmol) according to the same procedure as described in general synthesis method D.

*R*_{f} = 0.1 (DCM/MeOH = 98:2); ¹H NMR (600 MHz, CDCl₃) δ 7.65 (d, J = 1.8 Hz, 1H), 7.49 (dd, J = 8.5, 1.8 Hz, 1H), 6.93 (d, J = 8.5 Hz, 1H), 6.88 (s, 1H), 6.81 (d, J = 8.0 Hz, 1H), 6.77 (d, J = 8.0 Hz, 1H), 6.46 (d, J = 3.0 Hz, 1H), 6.24 (d, J = 3.0 Hz, 1H), 5.94 (s, 2H), 4.65 (dd, J = 9.0, 3.5 Hz, 1H), 3.92 (s, 3H), 3.88 (d, J = 15.3 Hz, 1H), 3.85 (d, J = 15.3 Hz, 1H), 2.92 (dd, J = 12.2, 3.5 Hz, 1H), 2.73 (dd, J = 12.2, 9.0 Hz, 1H). HRMS (ESI) m/z calculated for C₁₂H₁₀O₂⁺ [M - C₉H₁₀NO₃]⁺: 221.0364; found: 221.0329.

### (4) 2-(((5-(3-chloro-4-methoxyphenyl)furan-2-yl)methyl)amino)-1-(3,4-difluorophenyl)ethanol; (Compound 36)

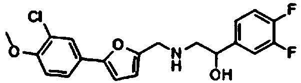

Compound 36 was prepared as a white solid in 37% yield using 5-(4-methoxyphenyl)furan-2-carbaldehyde (31b) (106 mg, 0.45 mmol) and 2-(3,4-difluorophenyl)-2-((triethylsilyl)oxy)ethanolamine (129 mg, 0.45 mmol) according to the same procedure as described in general synthesis method D.

*R*_{f} = 0.2 (DCM/MeOH = 98:2); ¹H NMR (600 MHz, CDCl₃) δ 7.65 (d, J = 2.1 Hz, 1H), 7.48 (dd, J = 8.5, 2.1 Hz, 1H), 7.23 - 7.19 (m, 1H), 7.14 - 7.08 (m, 1H), 7.06 - 7.10 (m, 1H), 6.93 (d, J = 8.5 Hz, 1H), 6.46 (d, J = 3.2 Hz, 1H), 6.24 (d, J = 3.2 Hz, 1H), 4.66 (dd, J = 9.0, 3.5 Hz, 1H), 3.93 (s, 3H), 3.88 (d, J = 15.0 Hz, 1H), 3.85 (d, J = 15.0 Hz, 1H), 2.95 (dd, J = 12.3, 3.5 Hz, 1H), 2.67 (dd, J = 12.3, 9.0 Hz, 1H), 1.59 (br, 2H). HRMS (ESI) m/z calculated for C₁₂H₁₀O₂⁺ [M - C₈H₈F₂NO]⁺: 221.0364; found: 221.0330.

### (5) 2-(((5-(3-chloro-4-methoxyphenyl)furan-2-yl)methyl)amino)-1-(naphthalen-2-yl)ethanol; (Compound 37)

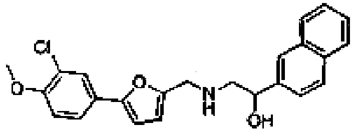

Compound 37 was prepared as a beige solid in 29% yield using 5-(4-methoxyphenyl)furan-2-carbaldehyde (31 b) (106 mg, 0.45 mmol) and 2-(naphthalene-2-yl)-2-((triethylsilyl)oxy)ethanolamine (135 mg, 0.45 mmol) according to the same procedure as described in general synthesis method D.

*R*_{f} *=* 0.3 (DCM/MeOH = 98:2); ¹H NMR (600 MHz, CDCl₃) δ 7.85 (s, 1H), 7.82 (d, J = 8.5 Hz, 3H), 7.65 (d, J = 2.1 Hz, 1H), 7.49 - 7.44 (m, 4H), 6.92 (d, J = 8.5 Hz, 1H), 6.46 (d, J = 3.2 Hz, 1H), 6.24 (d, J = 3.2 Hz, 1H), 4.91 (dd, J = 8.6, 3.7 Hz, 1H), 3.92 (s, 3H), 3.90 (d, J = 15.6 Hz, 1H), 3.87 (d, J = 15.6 Hz, 1H), 3.06 (dd, J = 12.3, 3.7 Hz, 1H), 2.87 (dd, J = 12.3, 8.6 Hz, 1H), 1.60 (br, 2H). HRMS (ESI) m/z calculated for C₁₂H₁₀O₂⁺ [M - C₁₂H₁₂NO]⁺: 221.0364; found: 221.0330.

### <Synthesis Example 3> Synthesis of β-amino alcohol derivative (2)

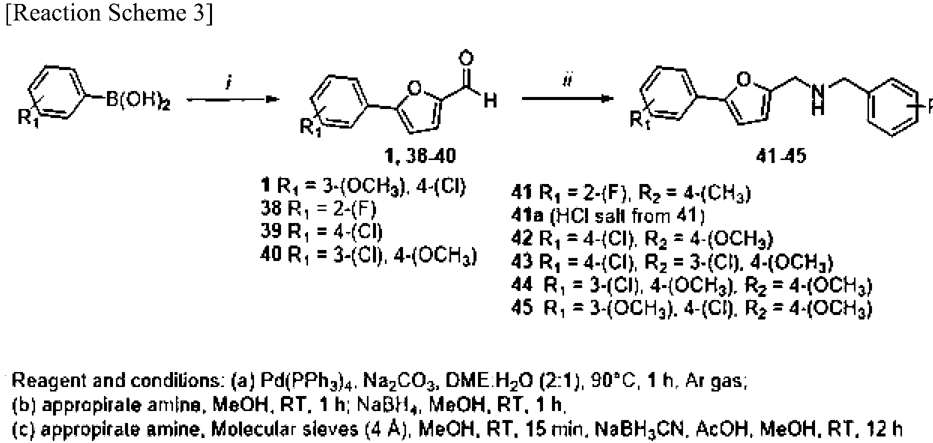

### (1) 5-(2-fluorophenyl)furan-2-carbaldehyde; (Intermediate 38)

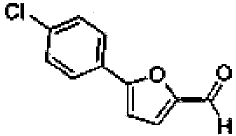

Intermediate 38 was prepared as a yellow solid in 90% yield using 5-bromofuran-2-carbaldehyde (400 mg, 2.29 mmol) and (2-fluorophenyl)boronic acid (400 mg, 2.89 mmol) according to the same procedure as described in general synthesis method A.

*R*_{f} = 0.4 (Hexane/EtOAc = 7:3); ¹H NMR (600 MHz, CDCl₃) δ 9.69 (s, 1H), 8.02 (td, J = 7.7, 1.5 Hz, 1H), 7.40 - 7.34 (m, 1H), 7.28 - 7.24 (m, 1H), 7.17 (dd, J = 11.2, 8.3 Hz, 1H), 7.05 - 7.02 (m, 1H).

### (2) 5-(4-chlorophenyl)furan-2-carbaldehyde; (Intermediate 39)

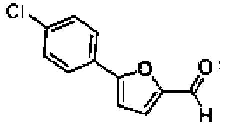

Intermediate 39 was prepared as a yellow solid in 80% yield using 5-bromofuran-2-carbaldehyde (300 mg, 1.71 mmol) and (4-chlorophenyl)boronic acid (340 mg, 2.14 mmol) according to the same procedure as described in general synthesis method A.

*R*_{f} = 0.33 (Hexane/EtOAc = 4:1); ¹H NMR (600 MHz, CDCl₃) δ 9.66 (s, 1H), 7.76 (d, J = 8.6 Hz, 2H), 7.43 (d, J = 8.6 Hz, 2H), 7.32 (d, J = 3.7 Hz, 1H), 6.83 (d, J = 3.7 Hz, 1H).

### (3) 5-(4-chloro-3-methoxyphenyl)furan-2-carbaldehyde; (Intermediate 40)

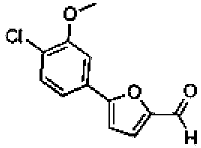

Intermediate 40 was prepared as an orange solid in 69% yield using 5-bromofuran-2-carbaldehyde (250 mg, 1.43 mmol) and (4-chloro-3-methoxyphenyl)boronic acid (330 mg, 1.79 mmol) according to the same procedure as described in general synthesis method A.

*R*_{f} = 0.4 (hexane/EtOAc = 7:3); ¹H NMR (600 MHz, CDCl₃) δ 9.66 (s, 1H), 7.43 (d, J = 8.2 Hz, 1H), 7.38 (d, J = 1.6 Hz, 1H), 7.34 - 7.32 (m, 2H), 6.85 (d, J = 3.7 Hz, 1H), 4.00 (s, 3H).

### (4) 1-(5-(2-fluorophenyl)furan-2-yl)-N-(4-methylbenzyl)methanamine; (Compound 41)

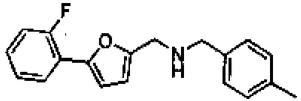

Compound 41 was prepared as a white solid in 73% yield using 5-(2-fluorophenyl)furan-2-carbaldehyde (38) (69 mg, 0.36 mmol) and p-tolylmethaneamine (46 µL, 0.36 mmol) according to the same procedure as described in general synthesis method B.

*R*_{f} = 0.42 (Hexane/EtOAc = 7:3); ¹H NMR (600 MHz, CDCl₃) δ 7.81 (td, J = 7.7, 1.9 Hz, 1H), 7.25 - 7.08 (m, 7H), 6.79 - 6.77 (m, 1H), 6.31 (d, J = 3.2 Hz, 1H), 3.85 (s, 2H), 3.81 (s, 2H), 2.34 (s, 3H). HRMS (ESI) m/z calculated for C₁₉H₁₉FNO ⁺ [M +H]⁺: 296.1445; found: 296.1456. C₁₁H₈FO⁺ [M - C₈H₁₀N]⁺: 175.0554; found: 175.0558.

### (5) 1-(5-(2-fluorophenyl)furan-2-yl)-N-(4-methylbenzyl)methanamine hydrochloride; (Compound 41a)

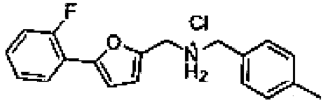

Compound 41a was prepared as a white solid in 10% yield. 1-(5-(2-fluorophenyl)furan-2-yl)-N-(4-methylbenzyl)methaneamine (41) (18 mg, 0.063 mmol) was added to ether (3 mL) and 1.25 M hydrochloric acid solution (50 µL, 0.063 mmol) was added to methanol. The precipitated solid was filtered and washed with ether.

¹H NMR (600 MHz, CDCl₃) δ 7.94 (td, J = 7.6, 1.7 Hz, 1H), 7.40 (d, J = 7.9 Hz, 2H), 7.25 - 7.19 (m, 2H), 7.15 (d, J = 7.9 Hz, 2H), 7.12 - 7.07 (m, 1H), 6.81 - 6.78 (m, 1H), 6.67 (d,

J = 3.2 Hz, 1H), 4.00 (s, 2H), 3.94 (s, 2H), 2.27 (s, 3H). HRMS (ESI) m/z calculated for C₁₉H₁₉FNO ⁺ [M +H]⁺: 296.1445; found: 296.1456. C₁₁H₈FO⁺ [M - C₈H₁₁ClN]⁺: 175.0554; found: 175.0558.

### (6) 1-(5-(4-chlorophenyl)furan-2-yl)-N-(4-methoxybenzyl)methanamine; (Compound 42)

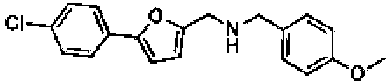

Compound 42 was prepared as a beige solid in 46% yield using 5-(4-chlorophenyl)furan-2-carbaldehyde (39) (100 mg, 0.48 mmol) and (4-methoxyphenyl)methaneamine (64 µL, 0.48 mmol) according to the same procedure as described in general synthesis method B.

*R*_{f} = 0.10 (Hexane/EtOAc = 4:1); ¹H NMR (600 MHz, CDCl₃) δ 7.58 (d, J = 8.6 Hz, 2H), 7.34 (d, J = 8.6 Hz, 2H), 7.27 (d, J = 8.7 Hz, 2H), 6.88 (d, J = 8.7 Hz, 2H), 6.58 (d, J = 3.2 Hz, 1H), 6.27 (d, J = 3.2 Hz, 1H), 3.83 (s, 2H), 3.81 (s, 3H), 3.78 (s, 2H). HRMS (ESI) m/z calculated for C₁₉H₁₉ClNO₂⁺ [M + H]⁺: 328.1099; found: 328.1044; C₁₁H₈ClO⁺ [M - C₈H₁₀NO]⁺: 191.0258; found: 191.0232.

### (7) N-(3-chloro-4-methoxybenzyl)-1-(5-(4-chlorophenyl)furan-2-yl)methanamine; (Compound 43)

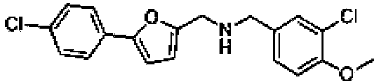

Compound 43 was prepared as a white solid in 46% yield using 5-(4-chlorophenyl)furan-2-carbaldehyde (39) (100 mg, 0.48 mmol) and (3-chloro-4-methoxyphenyl)methaneamine (69 µL, 0.48 mmol) according to the same procedure as described in general synthesis method B.

*R*_{f} = 0.30 (Hexane/EtOAc = 1:1); ¹H NMR (600 MHz, CDCl₃) δ 7.57 (d, J = 8.6 Hz, 2H), 7.37 (d, J = 2.1 Hz, 1H), 7.34 (d, J = 8.6 Hz, 2H), 7.19 (dd, J = 8.4, 2.1 Hz, 1H), 6.88 (d, J = 8.4 Hz, 1H), 6.57 (d, J = 3.2 Hz, 1H), 6.26 (d, J = 3.2 Hz, 1H), 3.89 (s, 3H), 3.82 (s, 2H), 3.75 (s, 2H). HRMS (ESI) m/z calculated for C₁₁H₈ClO⁺ [M - C₈H₉ClNO]⁺: 191.0258; found: 191.0230.

### (8) 1-(5-(3-chloro-4-methoxyphenyl)furan-2-yl)-N-(4-methoxybenzyl)methanamine; (Compound 44)

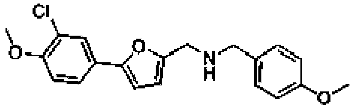

Compound 44 was prepared as a colorless liquid in 21% yield using 5-(3-chloro-4-methoxyphenyl)furan-2-carbaldehyde (1) (120 mg, 0.50 mmol) and (4-methoxyphenyl)methaneamine (65 µL, 0.50 mmol) according to the same procedure as described in general synthesis method B.

*R*_{f} = 0.13 (Hexane/EtOAc = 7:3); ¹H NMR (600 MHz, CDCl₃) δ 7.67 (d, J = 2.1 Hz, 1H), 7.50 (dd, J = 8.6, 2.1 Hz, 1H), 7.27 (d, J = 8.6 Hz, 2H), 6.93 (d, J = 8.6 Hz, 1H), 6.87 (d, J = 8.6 Hz, 2H), 6.47 (d, J = 3.2 Hz, 1H), 6.24 (d, J = 3.2 Hz, 1H), 3.92 (s, 3H), 3.82 (s, 2H), 3.80 (s, 3H), 3.78 (s, 2H). HRMS (ESI) m/z calculated for C₁₂H₁₀ClO₂⁺ [M - C₈H₁₀NO]⁺: 221.0364; found: 221.0330.

### (9) 1-(5-(4-chloro-3-methoxyphenyl)furan-2-yl)-N-(4-methoxybenzyl)methanamine; (Compound 45)

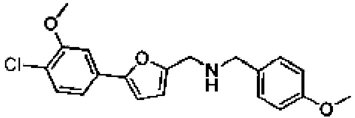

Compound 45 was prepared as a yellow solid in 60% yield using 5-(4-chloro-3-methoxyphenyl)furan-2-carbaldehyde (40) (110 mg, 0.46 mmol) and (4-methoxyphenyl)methaneamine (60 µL, 0.46 mmol) according to the same procedure as described in general synthesis method B.

*R*_{f} = 0.13 (Hexane/EtOAc = 7:3); ¹H NMR (600 MHz, CDCl₃) δ 7.34 (d, J = 8.2 Hz, 1H), 7.26 (d, J = 8.6 Hz, 2H), 7.20 (d, J = 1.8 Hz, 1H), 7.18 (dd, J = 8.2, 1.8 Hz, 1H), 6.87 (d, J = 8.6 Hz, 2H), 6.59 (d, J = 3.2 Hz, 1H), 6.28 (d, J = 3.2 Hz, 1H), 3.97 (s, 3H), 3.84 (s, 2H), 3.80 (s, 3H), 3.78 (s, 2H). HRMS (ESI) m/z calculated for C₁₂H₁₀ClO₂⁺ [M - C₈H₁₀NO]⁺: 221.0364; found: 221.0329.

### <Example 1> Establishment of stable cell line expressing target odorant receptor

### 1-1. Establishment of stable cell Line expressing Olfr164 in PinKo cell lines

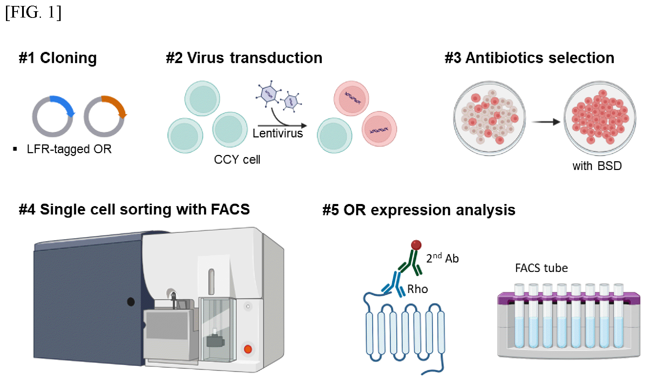

The target odorant receptor Olfr164 conjugated with the Lucy-Flag-Rho tag (LFR tag), was cloned into a lentivirus vector containing a blasticidin (BSD) resistance gene. HEK293T cells were infected with the lentivirus expressing the target odorant receptor Olfr164 through a virus packaging process. Cell lines expressing the target odorant receptor Olfr164 were primarily selected through an antibiotic screening process using BSD. Single-cell sorting was performed using FACS to select the cell line that uniformly expresses the target odorant receptor. Single cells were collected and maintained in 96-well plates, and FACS was performed according to the cell growth rate to select cells exhibiting the highest surface expression of the target odorant receptor Olfr164. The process for establishing a stable cell line for the expression of the target odorant receptor Olfr164 is shown in FIG. 1.

To select cell lines stably expressing the target odorant receptor Olfr164 and the cAMP sensor, FACS and Fold change (Fsk/DMSO) tests were conducted, and additionally, the surface expression of the target odorant receptor Olfr164 was confirmed through immunofluorescence staining.

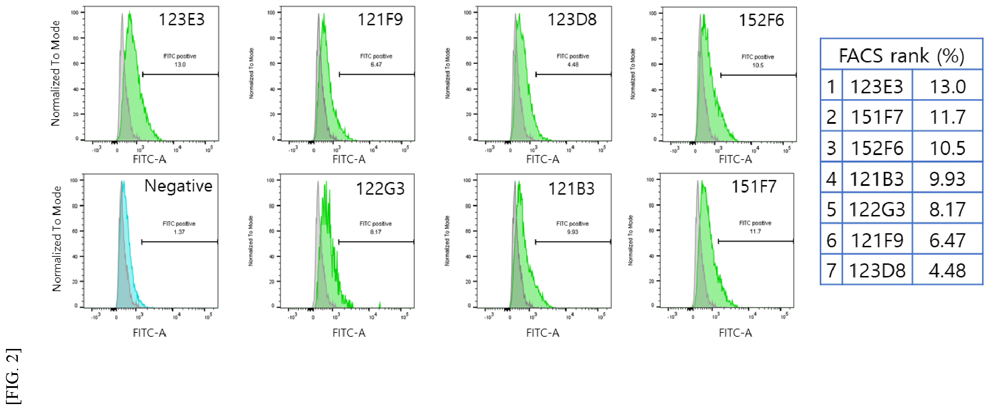

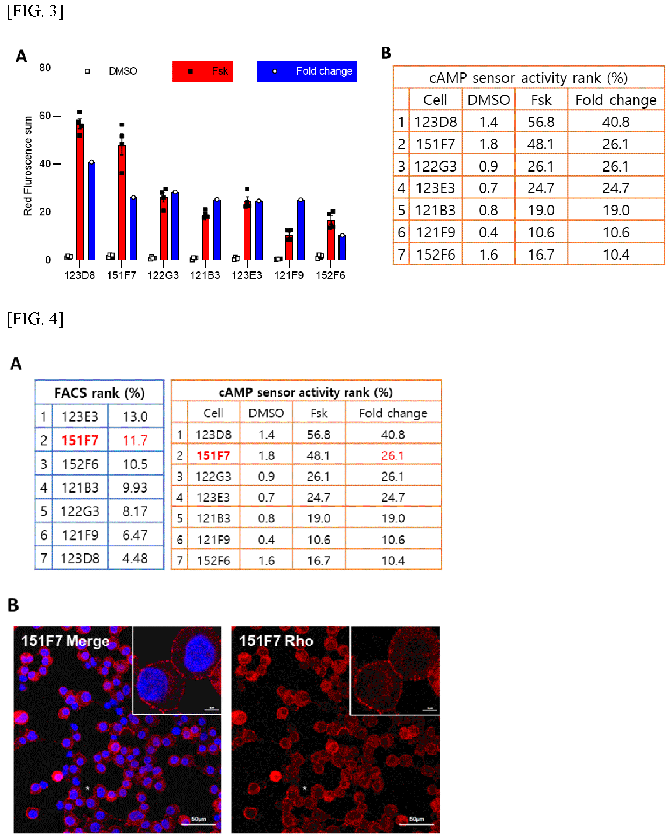

The level of target odorant receptor Olfr164 expression confirmed by FACS is shown in FIG. 2, the level of cAMP sensor expression confirmed by Fold change (Fsk/DMSO) values is shown in FIG. 3, and the level of target odorant receptor Olfr164 expression confirmed by the Rho antibody and the results of a numerical comparison are shown in FIG. 4.

### <Example 2> Ligand screening in cell lines stably expressing target odorant receptors

### 2-1. Primary screening (YFP quenching assay)

To select ligands that activate the target odorant receptor Olfr164, primary to tertiary screenings were performed using two assay systems through complementary validation.

The screening was conducted based on a GPCR library consisting of approximately 8,800 compounds from the Korean Chemical Bank, and ligands were selected using two different assay systems: the primary screening utilized the YFP quenching assay, which has higher throughput, while the secondary and tertiary screenings utilized the cAMP sensor assay.

The YFP assay was performed using CCY cells, a Chinese hamster ovary (CHO)-derived cell line co-expressing cystic fibrosis transmembrane conductance regulator (CFTR) and halide-sensitive yellow fluorescent protein (YFP). To obtain efficient and reproducible results, a stable CCY cell line expressing the target olfactory receptor, Olfr164, was established.

For the assay, the Olfr164-expressing CCY cells were seeded into a 96-well plate. After cell attachment, the culture medium was replaced with a regular solution (140 mM NaCl, 10 mM HEPES, 10 mM glucose, 5 mM KCl, and 1 mM MgCl₂ and CaCl₂), followed by treatment with the test compound. After 10 minutes of incubation, an iodide solution (140 mM NaI, 10 mM HEPES, 10 mM glucose, 5 mM KCl, and 1 mM MgCl₂ and CaCl₂) was added. Fluorescence intensity was then measured using an EnVision plate reader (PerkinElmer^{®}) at an excitation wavelength of 485 nm and an emission wavelength of 535 nm.

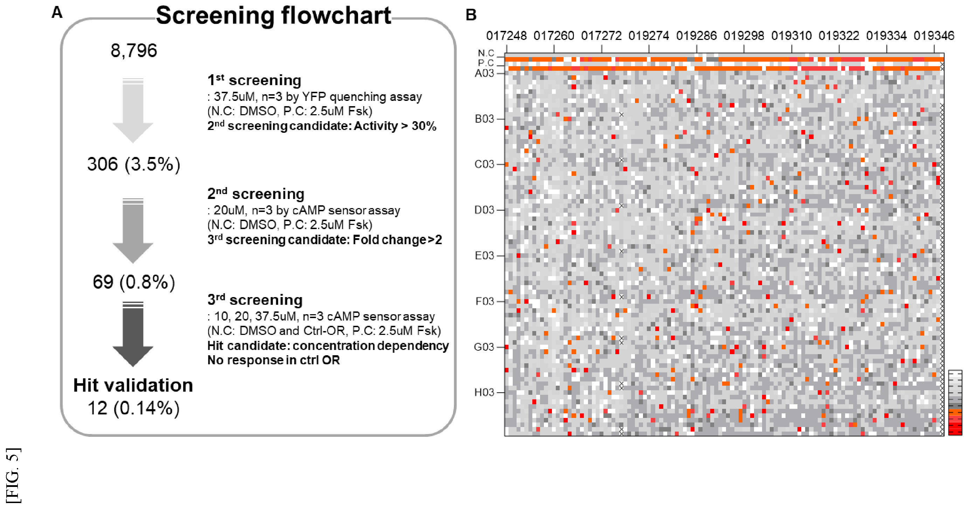

The first screening was conducted at a concentration of 37.5 µM with n=3, and compounds exhibiting higher activity than the positive control, 2.5 µM forskolin, were selected for the second screening. The heatmap of the primary screening results via the YFP quenching assay is shown in FIG. 5.

### 2-2. Secondary screening (cAMP sensor assay)

The YFP quenching assay is based on a detection principle in which the YFP fluorescence signal is inversely correlated with activation of the target odorant receptor Olfr164. In contrast, the cAMP sensor assay is based on a detection principle in which activation of the target odorant receptor is directly proportional to the RFP signal generated by the cAMP sensor. Therefore, an image based real-time monitoring through the cAMP sensor assay enables complementary validation of receptor activation and cytotoxicity.

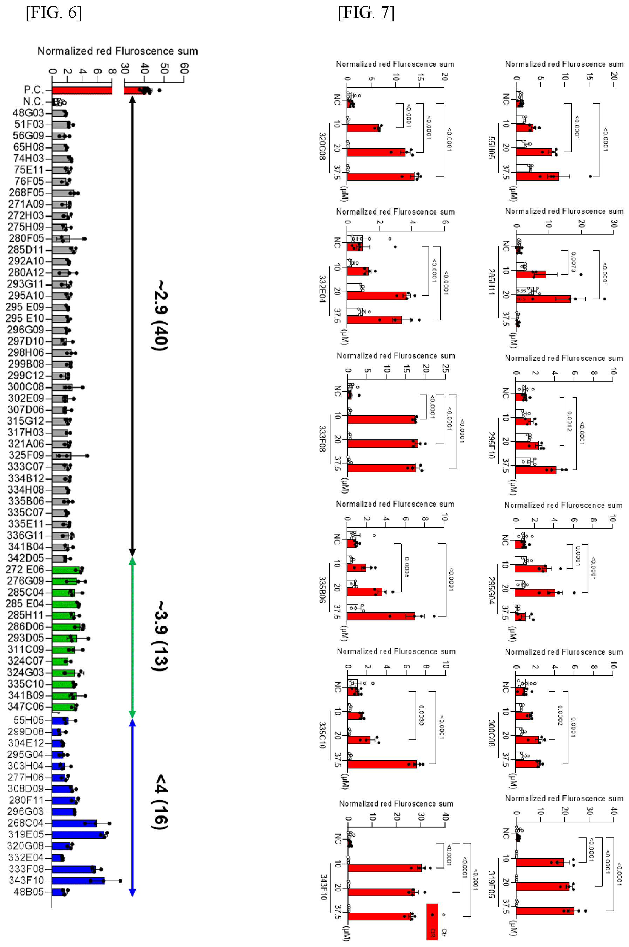

Secondary screening was performed using a cAMP sensor assay at a concentration of 10 µM with n=3. As shown in FIG. 6, the results of the second screening were selected for the third screening, based on compounds that showed a statistically significant increase compared to DMSO, the solvent of the compounds, and as a result, a total of 69 compounds were selected for the third screening, including 40 compounds with a chemical/DMSO fold change value of 2 or higher, 13 compounds with 3 or higher, and 16 compounds with 4 or higher.

### 2-3. Third screening (cAMP sensor assay)

The third screening was conducted using the cAMP sensor assay to select compounds that induce a concentration dependent response of the target odorant receptor Olfr164. Therefore, dose-dependent screening was performed using cell lines that stably express the target odorant receptor Olfr164 and other odorant receptors. Tests were conducted at concentrations of 10, 20, and 37.5 µM with n=3, and as shown in FIG. 7, it was confirmed that 12 out of 69 compounds exhibited activity specific to the target odorant receptor. Based on the list of compounds that elicit a specific response to the target odorant receptor selected through screening and the similarity of their skeletal structures, they were classified into three groups, and among them, a total of 35 KB45 derivatives were synthesized based on the group containing a furan-containing skeleton.

### <Example 3> Activity of KB45 derivatives in cell lines stably expressing target odorant receptor Olfr164

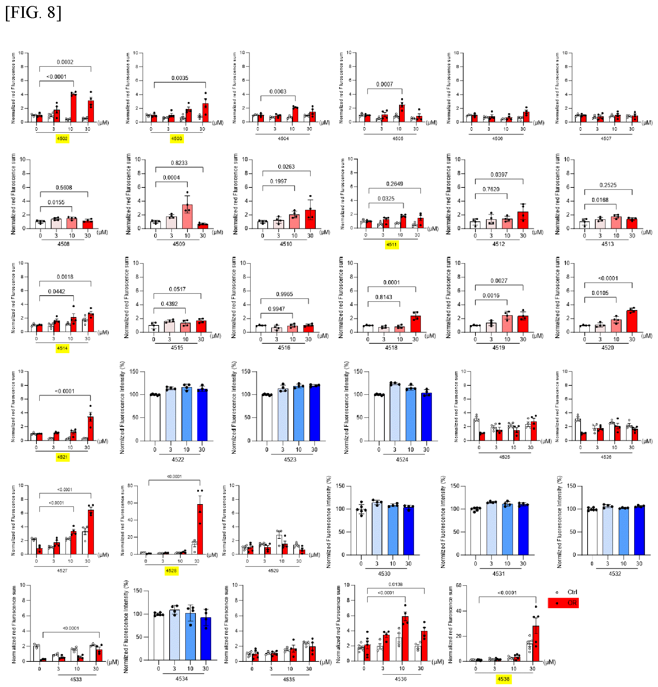

Reactivity tests were conducted for the total of 35 synthesized compounds using YFP or cAMP sensor assays at concentrations of 3, 10, and 30 µM in cell lines stably expressing the target odorant receptor Olfr164. As a result, as shown in Table 1 and FIG. 8, 7 out of a total of 35 compounds (4502, 4503, 4511, 4514, 4521, 4528, 4538) showed specific activity on the target odorant receptor Olfr164.

**TABLE 1**

| No . | Compoun d | KB code (KB45X X) | Experimental concentration (µM), Ctrl | | | Experimental concentration (µM), target odorant receptor | | | Assay metho d |
|---|---|---|---|---|---|---|---|---|---|
| | | | 3 | 10 | 30 | 3 | 10 | 30 | |
| 1 | 1a | KB4501 | X | X | X | - | 3.7±0.9 | 8.7±3.5 (37.5 µM) | cAMP |
| 2 | 27 | KB4502 | 1±0.3 | 0.4±0. 1 | 0.5±0.2 | 1.8±0.8 | 4.1±0.2 | 3.1±0.9 | cAMP |
| 3 | 28 | KB4503 | 0.6±0.1 | 0.6±0. 4 | 0.8±0.2 | 1±0.4 | 1.9±0.5 | 2.7±1.2 | cAMP |
| 4 | 2a | KB4504 | 0.7±0.1 | 0.7±0. 2 | 0.9±0.1 | 0.9±0.2 | 2.1±0.1 | 1.5±0.6 | cAMP |
| 5 | 8a | KB4505 | 0.5±0.2 | 0.8±0. 2 | 0.5±0.1 | 1.1±0.5 | 2.5±0.6 | 0.8±0.6 | cAMP |
| 6 | 3a | KB4506 | 0.7±0.1 | 0.5±0. 1 | 0.6±0.2 | 0.8±0.4 | 0.7±0.3 | 1.5±0.4 | cAMP |
| 7 | 9a | KB4507 | 0.7±0.2 | 0.9±0. 2 | 0.9±0.2 | 0.8±0.4 | 1±0.4 | 1±0.4 | cAMP |
| 8 | 4a | KB4508 | X | X | X | 1.4±0.2 | 1.5±0.2 | 1.2±0.2 | cAMP |
| 9 | 10a | KB4509 | X | X | X | 1.8±0.2 | 3.5±1.1 | 0.7±0.1 | cAMP |
| 10 | 5a | KB4510 | X | X | X | 1.2±0.4 | 2.1±0.4 | 2.7±1.3 | cAMP |
| 11 | 29 | KB4511 | 0.6±0.2 | 0.7±0. 1 | 0.5±0.2 | 1.2±0.4 | 1.8±0.2 | 1.5±0.7 | cAMP |
| 12 | 11a | KB4512 | X | X | X | 1.4±0.6 | 1.4±0.3 | 2.5±1 | cAMP |
| 13 | 6a | KB4513 | X | X | X | 1.4±0.2 | 1.8±0.2 | 1.4±0.2 | cAMP |
| 14 | 13a | KB4514 | 0.9±0.3 | 1.1±0. 1 | 1.7±0.5 | 1.6±0.4 | 2.1±1 | 2.7±0.5 | cAMP |
| 15 | 7a | KB4515 | X | X | X | 1.6±0.2 | 1.3±0.4 | 1.7±0.3 | cAMP |
| 16 | 7b | KB4516 | X | X | X | 0.7±0.3 | 1±0.2 | 1±0.2 | cAMP |
| 17 | 30 | KB4518 | X | X | X | 0.8±0.2 | 0.8±0.2 | 2.4±0.5 | cAMP |
| 18 | 31 | KB4519 | X | X | X | 1.3±0.3 | 2.5±0.5 | 2.4±0.5 | cAMP |
| 19 | 32 | KB4520 | X | X | X | 1.1±0.3 | 1.8±0.4 | 3.2±0.3 | cAMP |
| 20 | 33 | KB4521 | 0.3±0 | 0.3±0. 1 | 0.3±0 | 1.1±0.1 | 1.2±0.4 | 3.4±1.1 | cAMP |
| 21 | 12a | KB4522 | X | X | X | 113.9±2. 6 | 116.8±5.6 | 113±5 | YFP |
| 22 | 12b | KB4523 | X | X | X | 114±6.2 | 119.2±3.5 | 119.9±1. 9 | YFP |
| 23 | 14a | KB4524 | X | X | X | 124±2.2 | 115.3±4.5 | 104.1±5. 9 | cAMP |
| 24 | 15a | KB4525 | 1.9±0.4 | 2.2±0. 3 | 2.3±0.5 | 1.6±0.5 | 1.5±0.6 | 2.8±0.7 | cAMP |
| 25 | 16a | KB4526 | 1.8±0.5 | 2.6±0. 2 | 2.2±0.4 | 1.8±0.3 | 2.1±0.7 | 1.6±0.3 | cAMP |
| 26 | 17a | KB4527 | 1±0.1 | 2.3±0. 2 | 3.3±0.8 | 1.7±0.5 | 3.3±0.5 | 6.5±0.7 | cAMP |
| 27 | 41 | KB4528 | 1.3±0.1 | 1.6±0. 4 | 11.8±5. 4 | 1.6±0.2 | 2.7±1.2 | 58.6±16. 5 | cAMP |
| 28 | 42 | KB4529 | 1.5±0.2 | 2.8±0. 9 | 1.4±0.3 | 1±0.5 | 1.5±0.7 | 0.7±0.4 | cAMP |
| 29 | 43 | KB4530 | X | X | X | 114.8±4. 3 | 108.6±2.8 | 104.2±4 | YFP |
| 30 | 34 | KB4531 | X | X | X | 115.4±1. 6 | 111.6±4.4 | 110.6±3 | YFP |
| 31 | 35 | KB4532 | X | X | X | 106.6±3 | 102.4±1 | 106.4±1. 7 | YFP |
| 32 | 36 | KB4533 | 0.9±0.1 | 1.6±0. 2 | 2.2±0.2 | 0.5±0.2 | 0.5±0.2 | 1.6±0.5 | cAMP |
| 33 | 37 | KB4534 | X | X | X | 109.6±8. 2 | 102.5±15. 3 | 92.8±15. 4 | YFP |
| 34 | 44 | KB4535 | 1.1±0.2 | 1.5±0. 2 | 2.4±0.3 | 1.1±0.3 | 1.7±0.8 | 2±0.8 | cAMP |
| 35 | 45 | KB4536 | 1.9±0.6 | 3.1±1 | 1.9±0.4 | 3.5±0.5 | 5.9±1 | 4±0.9 | cAMP |
| 36 | 41a | KB4538 | 1.7±0.5 | 2.7±0. 9 | 14±4.6 | 1.6±0.6 | 3.6±1.8 | 28.4±12. 4 | cAMP |

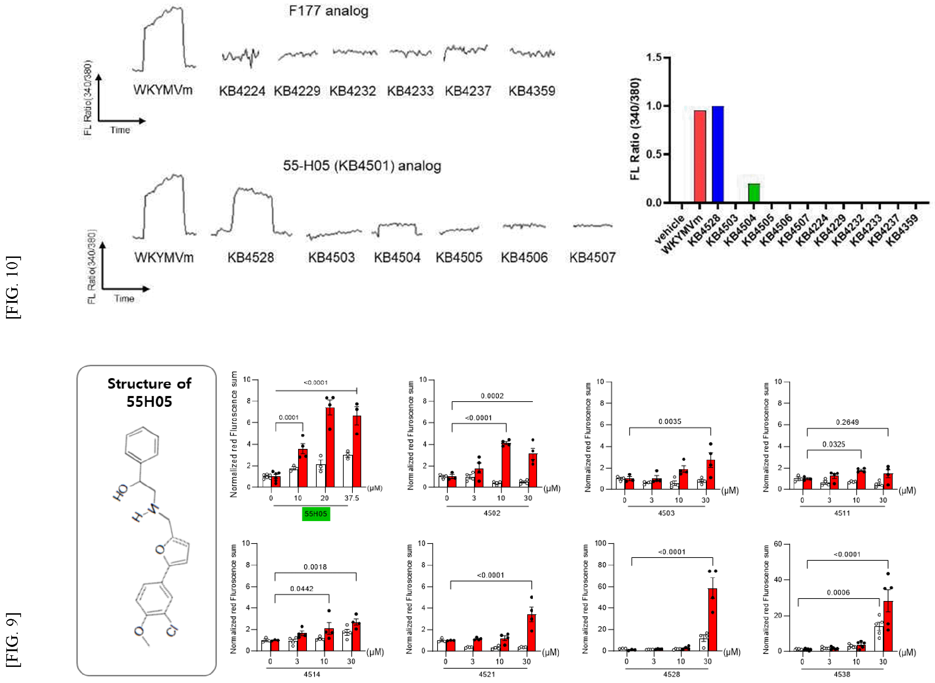

In addition, the results of comparing the activity of derivatives that exhibited a specific response to the target odorant receptor Olfr164 with that of 55H05 (KB4501), selected through screening, are shown in FIG. 9. KB4502 showed good activity at a level similar to 55H05 (KB4501), while KB4528 showed very high activity at a single concentration of 30 µM.

### <Example 4> Analysis of cell signaling and functional inhibitory effects of derivatives in neutrophils

### 4-1. Analysis of calcium ion increase activity in neutrophils

The modulation effect of phospholipase C (PLC)-mediated calcium ion increase, a key signaling molecule for neutrophil activation, was analyzed for structural analogs of the active substance 55H05 (KB4501). To measure the increase in calcium ions in neutrophils, neutrophils isolated from mouse bone marrow were stained with the calcium-sensing fluorescent dye Fura-2 AM and suspended in a buffer solution. Subsequently, stained neutrophils were treated with the active substance 55H05 (KB4501) and its structural analogs (KB4528, KB4504, etc.) or a vehicle, and changes in intracellular calcium ion concentration following stimulation were measured by changes in the fluorescence ratio (340/380 nm). Fluorescence signals were analyzed using a spectro-fluorescence spectrometer. WKYVMm was used as a positive control for inducing a PLC-mediated increase in calcium ions.

In this manner, the increase in intracellular calcium ions caused by the active substance F177 and its derivatives derived in Step 1 was measured on mouse bone marrow-derived neutrophils, and it was confirmed that these substances did not induce the calcium ion increase in neutrophils. On the other hand, as a result of analyzing the calcium ion increase in neutrophils caused by 55H05 and its structural analogs, a strong increase in intracellular calcium ions was observed by KB4528, as shown in FIG. 10, and weak activity was also observed by KB4504.

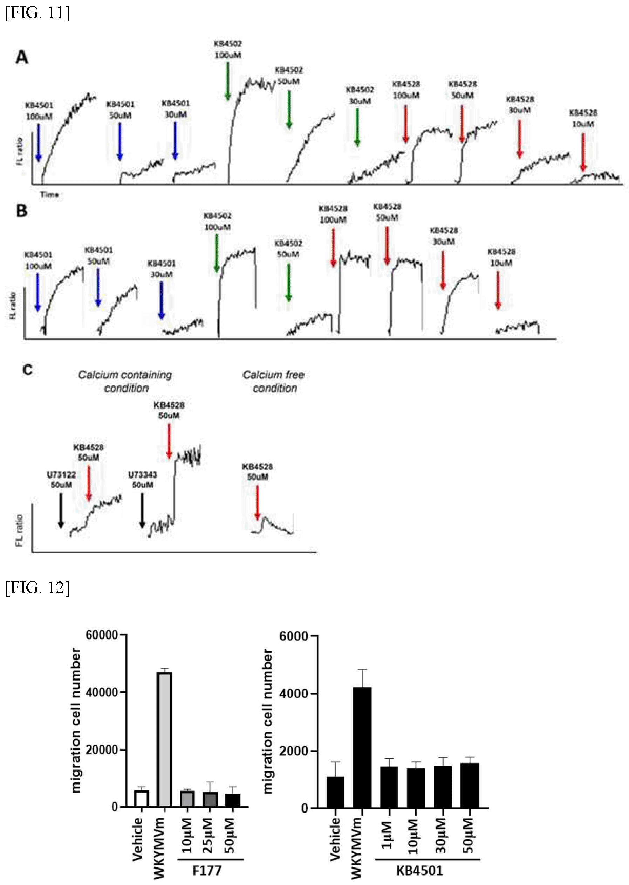

In addition, as shown in FIG. 11, calcium ion levels in neutrophils (A) and low-density myeloid cells (B, C) isolated from mouse bone marrow were measured using KB45 derivatives, and a distinct increase in calcium ions was observed in neutrophils and low-density myeloid cells upon stimulation by KB4501, KB4502, and KB4528. In particular, stimulation by KB4528 induced a very strong concentration-dependent increase in intracellular calcium ions.

To investigate whether the increase in intracellular calcium ions is mediated through PLC activity, experiments were conducted by pre-treating with U-73122, a selective PLC inhibitor, and U-73343, an inactive analog. As shown in FIG. 11C, the increase in intracellular calcium ions induced by KB4528 was effectively inhibited by pre-treatment with U-73122, but was not significantly affected by pre-treatment with U-73343.

Meanwhile, even under conditions where extracellular calcium ions were removed so that changes in intracellular calcium ions were mediated solely through the release of calcium ions from the endoplasmic reticulum (ER), stimulation by KB4528 induced an increase in intracellular calcium ions. These results confirmed that KB4528, identified as an Olfr164 agonist, induces an increase in intracellular PLC-activation mediated calcium ions through odorant receptor activation.

### 4-2. Neutrophil chemotaxis modulation experiment

The effects of the active substance F177 and the additionally identified active substance 55H05 (KB4501) on chemotaxis, an indicator of neutrophil activation, were investigated. Neutrophil chemotaxis was analyzed using a transwell migration assay. Briefly, neutrophils isolated from mouse bone marrow were dispensed into the upper chamber of the transwell. In the lower chamber, the active substance F177, 55H05 (KB4501), or the formyl peptide receptor (FPR) agonist WKYVMm as a positive control was added. After incubation for 2 hours at 37 °C, the number of neutrophils that migrated to the lower chamber was counted to quantitatively analyze chemotaxis.

As a result, as shown in FIG. 12, it was confirmed that neutrophil chemotaxis was strongly promoted by the FPR agonist WKYVMm used as a control, but neutrophil chemotaxis was not promoted by F177 and KB4501.

### 4-3. Analysis of inhibitory effect on neutrophil cytokine production

The mediation of inflammatory responses by neutrophil activation occurs through the production of various inflammatory cytokines from neutrophils. To evaluate the inflammatory response mediating effects following neutrophil activation, the effect of active substances on the production of neutrophil-derived inflammatory cytokines induced by lipopolysaccharide (LPS) stimulation was analyzed. Cytokine production levels were measured using an enzyme-linked immunosorbent assay (ELISA). Mouse neutrophils were stimulated with *Pseudomonas aeruginosa*-derived LPS (1 µg/ml) for 24 hours, and experiments were performed under conditions with or without treatment with F177 or 55H05 (KB4501). After stimulation, the culture supernatant was recovered and the concentration of extracellularly secreted cytokines was analyzed.

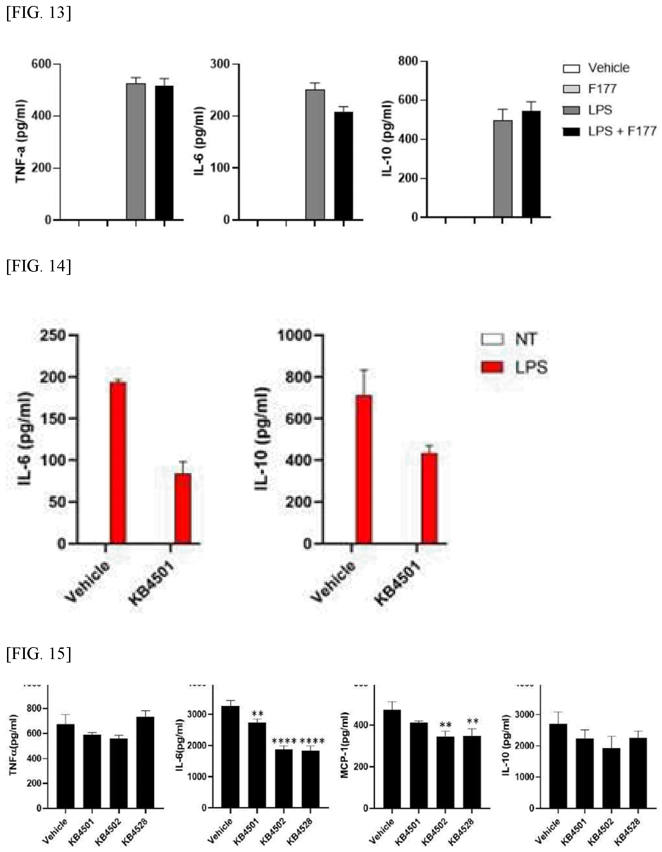

As a result of stimulation with LPS, the production of TNF-α, IL-6, and IL-10 was strongly induced, as shown in FIG. 13, however, no significant change in cytokine production was observed with F177 treatment. On the other hand, in the case of KB4501, it was confirmed that LPS-mediated inflammatory cytokine production was effectively inhibited, as shown in FIG. 14. These results confirmed that neutrophil-mediated inflammatory responses may be effectively controlled through target odorant receptor Olfr164 agonists.

### 4-4. Analysis of inhibitory effect on LPS-induced inflammatory cytokine and chemokine production

Since the pathological mediation of sepsis is mediated through the production of various inflammatory cytokines and chemokines, the modulatory effects of 32 representative derivatives on cytokine production were investigated in low-density myeloid cells. Cytokine production levels were measured using an enzyme-linked immunosorbent assay (ELISA). Mouse neutrophils were stimulated with *Pseudomonas aeruginosa*-derived LPS (1 µg/ml) for 24 hours, and experiments were conducted under conditions with or without treatment with the derivatives. After stimulation, the culture supernatant was collected, and the concentration of extracellularly secreted cytokines was analyzed.

As a result, as shown in FIG. 15, the production of TNF-α, IL-6, MCP-1, and IL-10 was strongly increased by stimulation with lipopolysaccharide (LPS, 1 µg/ml), a neutrophil activation stimulant, and IL-6 production induced by LPS stimulation was significantly reduced by treatment with three representative derivatives (KB4501, KB4502, and KB4528). In addition, it was confirmed that MCP-1 production was significantly reduced by KB4502 and KB4528. Based on these results, it was confirmed that inflammatory responses may be controlled by these derivatives.

### 4-5. Analysis of modulatory effect on pDC cytokine production

The effect of active substances on the modulation of immune cell activity in pDCs, which are immune cells expressing the target odorant receptor Olfr164 along with neutrophils, was analyzed. Stimulating pDCs with CpG-A, a TLR9 stimulator, strongly promotes the production of inflammatory cytokines IL-6 and TNF-α, as well as type I IFN (IFN-α). The cytokine production levels of pDCs were measured using an enzyme-linked immunosorbent assay (ELISA). Mouse-derived pDCs were stimulated with CpG-A, a TLR9 stimulator, and simultaneously treated with the active substance KB4501 and its derivatives (KB4502, KB4527, KB4528, KB4533, etc.). After culturing for 24 hours following CpG-A stimulation, the culture supernatant was collected, and the concentrations of cytokines secreted from the pDCs were analyzed.

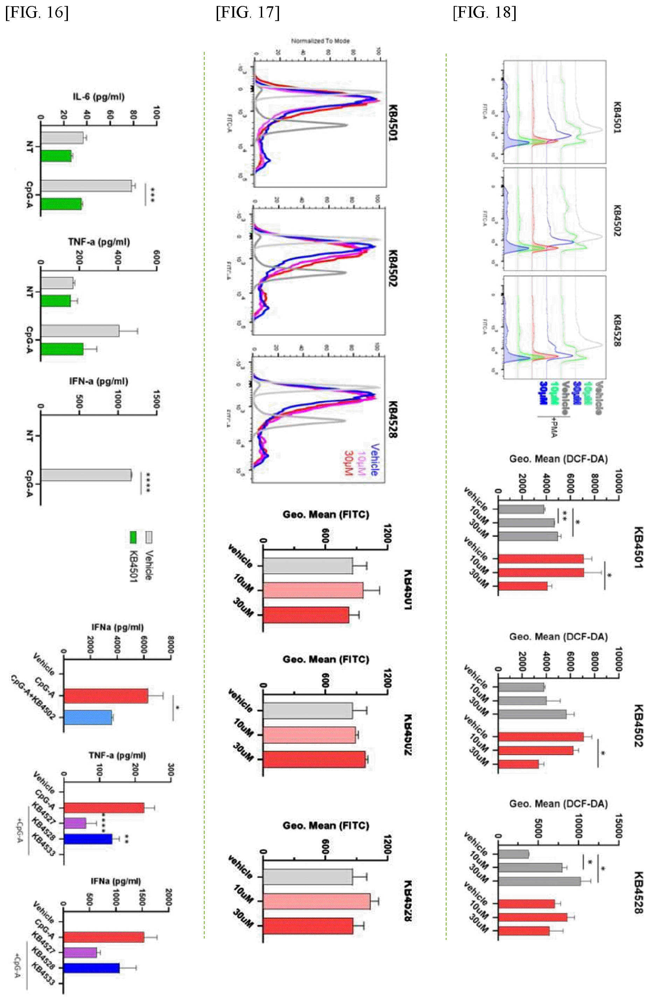

As a result of investigating the modulatory effect on CpG-A-induced cytokine production in pDCs by the derivatives KB45xx, the production of IL-6, TNF-α, and IFN-α was strongly inhibited by the active substance KB4501, as shown in FIG. 16. The production of TNF-α, IL-6, and IFN-α induced by CpG-A stimulation was also inhibited by KB4502, KB4527, KB4528, and KB4533.

### 4-6. Effects of derivatives on neutrophil antigen phagocytosis ability

Since phagocytosis plays a key role in the mechanism of neutrophil control of infectious agents, changes in neutrophil antigen phagocytosis following derivative treatment were analyzed using pHrodo^{™} BioParticles. Neutrophils isolated from mouse bone marrow were treated with three representative active derivatives (KB4501, KB4502, and KB4528). Subsequently, pHrodo^{™} BioParticles were added, and reacted for 30 minutes at 37°C. Since pHrodo fluorescence signals selectively increase within acidified phagocytic vesicles, the degree of antigen phagocytosis based on this was analyzed using flow cytometry. Antigen phagocytosis ability was quantified by the proportion of pHrodo-positive cells or the mean fluorescence intensity (MFI).

As a result, as shown in FIG. 17, the antigen phagocytosis ability of neutrophils did not show a significant change upon stimulation by the three derivatives (KB4501, KB4502, and KB4528), confirming that these derivatives do not affect the antigen phagocytic function of neutrophils.

### 4-7. Analysis of modulatory effect on neutrophil reactive oxygen species (ROS) generation

Since the generation of reactive oxygen species (ROS) plays a crucial role in the control of infectious agents by neutrophils, the modulatory effects of three representative derivatives (KB4501, KB4502, and KB4528) on neutrophil ROS generation were investigated. Neutrophils were treated with the active substance KB4528 or each derivative. Subsequently, DCF-DA (5 µM), a ROS-sensing fluorescent dye, was added to the cells, and reacted at 37°C for 30 minutes. After the reaction was completed, the intracellular ROS generation level was measured using flow cytometry, and the degree of ROS generation was quantitatively analyzed based on fluorescence intensity.

As shown in FIG. 18, the results showed that ROS generation was promoted by KB4528 alone, while KB4502 inhibited ROS generation induced by PMA (Phorbol 12-myristate 13-acetate) treatment.

### 4-8. Analysis of modulatory effect of NET formation

The formation of neutrophil extracellular traps (NETs) plays a significant role in the mechanisms of infectious disease pathology modulation by neutrophils. While NETs may be used to control infectious agents, NET formation may cause problems with blood circulation and mediate septic pathology. Neutrophils were stimulated with lipopolysaccharide (LPS, 50 µg/ml) for 4 hours. After stimulation, the cells were fixed with 4% formaldehyde at room temperature for 15 minutes, and SYTOX Orange (100 nM) was added for extracellular DNA staining, followed by reaction at room temperature for 15 minutes. NET formation was observed and quantitatively analyzed using a fluorescence microscope.

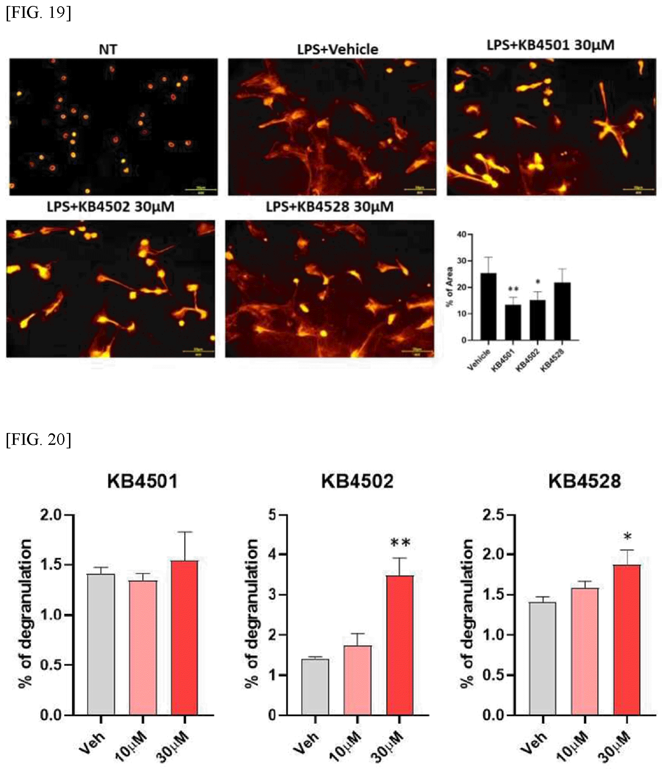

As a result of analyzing the modulatory effects of three representative derivatives with good activity (KB4501, KB4502, and KB4528) on NET formation, as shown in FIG. 19, KB45401 and KB4502 significantly inhibited LPS-induced NET formation, whereas KB4528 did not have a significant effect on LPS-induced NET formation.

### 4-9. Analysis of neutrophil degranulation effect

The activity of derivatives affecting neutrophil degranulation was analyzed. Neutrophils were cultured with the vehicle or active substances (KB4501 and derivatives) for 30 minutes. Subsequently, the culture supernatant and cell lysate were collected and reacted with a β-hexosaminidase substrate solution at 37°C for 2 hours. The absorbance of the products generated after the reaction was measured using a spectrophotometer. The degree of degranulation was quantified as the ratio of β-hexosaminidase activity released into the supernatant to total enzyme activity.

As a result of analyzing the degranulation effect using this method, as shown in FIG. 20, neutrophil degranulation was promoted by KB4502 and KB4528, while degranulation activity was not induced by KB4501.

### 4-10. Analysis of modulatory effect on pDC surface protein and transcription factor expression

To analyze the modulatory effect of pDC activation on cell surface antigen and transcription factor expression, immunophenotyping analysis using flow cytometry was performed. Mouse-derived pDCs were stimulated with the TLR9 stimulator CpG-A for 18 hours, and simultaneously treated with the active substance KB4501 and its derivatives (KB4502, KB4527, KB4528, KB4533). After stimulation, cells were collected, and the expression levels of activation markers CD86, PD-L1, and MHC class II (MHCII) were analyzed using fluorescently labeled antibodies. Additionally, the expression of IRF5 and IRF7, major transcription factors mediating cell activation, was analyzed using fluorescently labeled antibodies after the cell fixation and permeation process.

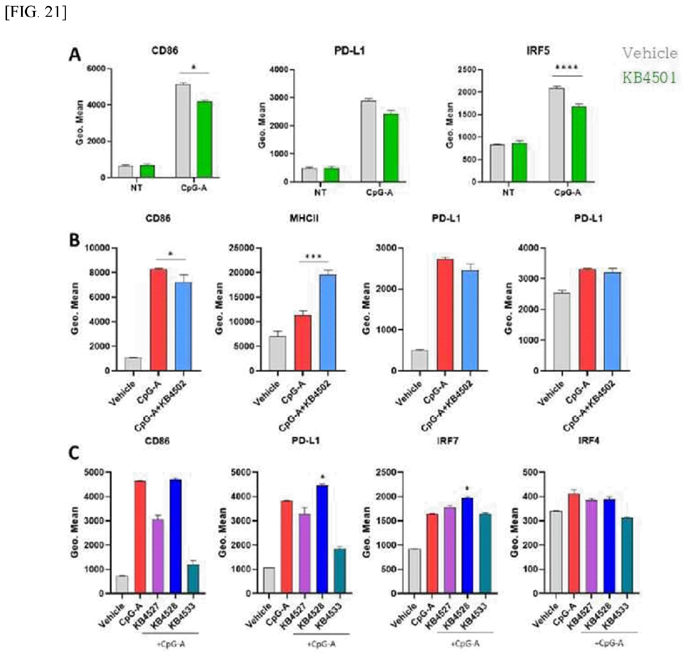

As shown in FIG. 21, CpG-A stimulation significantly increased the expression of CD86 and PD-L1 on the surface of pDCs. This increase in the expression of cell surface antigens was effectively suppressed upon treatment with KB4501. Furthermore, it was confirmed that the expression of interferon regulatory factor 5 (IRF5), a major transcription factor mediating cell activation, was induced by CpG-A stimulation and significantly decreased upon treatment with KB4501.

Meanwhile, treatment with KB4502 reduced CpG-A stimulation-induced CD86 expression, whereas increased MHC class II (MHCII) expression. In addition, a decreasing trend in CD86 expression, which was increased by CpG-A stimulation, was observed with treatments of KB4527 and KB4533. On the other hand, treatment with KB4528 confirmed an increase in the expression of PD-L1 and interferon regulatory factor 7 (IRF7).

Overall, it was determined that KB4501 and its derivatives, KB4502 and KB4528, may effectively control the activation of neutrophils and pDCs, thereby enabling the control of inflammatory diseases.

### <Example 5> Therapeutic effect of KB4528 in sepsis animal model

### 5-1. Analysis of therapeutic effect of KB4528 on sepsis

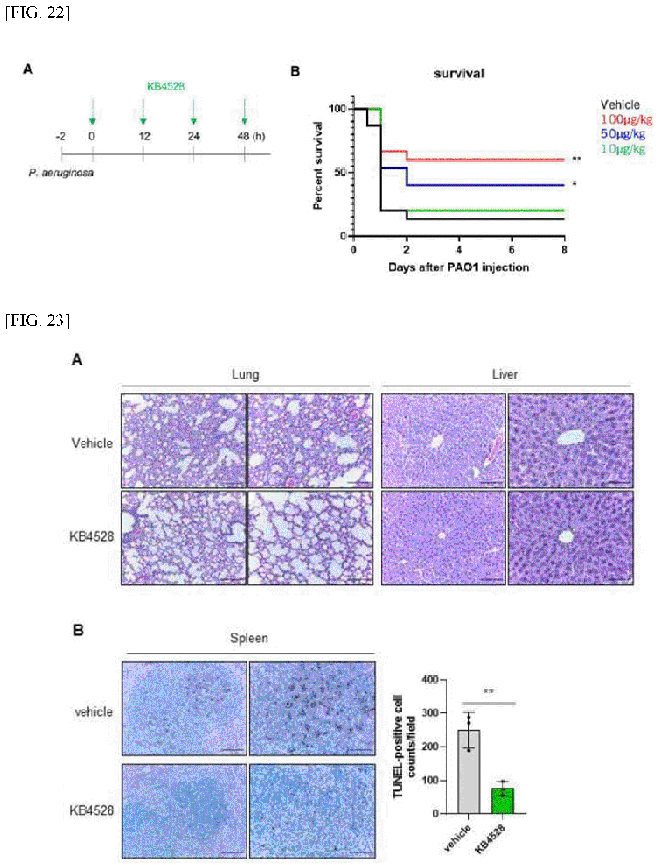

In mice, the lead compound KB4528 was administered intraperitoneally a total of four times at 12-hour intervals starting 2 hours after infection with *Pseudomonas aeruginosa* at three different doses (10, 50, and 100 µg/kg), and survival rates were compared. As shown in FIG. 22, the control group administered the vehicle showed a rapid decline in survival rate within 48 hours of infection, whereas the group administered the lead compound KB4528 showed a concentration-dependent increase in survival rate against *Pseudomonas aeruginosa-*induced sepsis, and in particular, a significant improvement in survival rate was confirmed at a concentration of 100 µg/kg. Through this, it was confirmed that sepsis induced by *Pseudomonas aeruginosa* infection is effectively modulated by the administration of KB4528.

### 5-2. Inhibitory effect of KB4528 on organ damage in sepsis animal model

Damage to major organs caused by sepsis was checked in the experimental group injected with 100 µg/kg of KB4528, which showed a significant improvement in survival rate. 24 hours after infection with *Pseudomonas aeruginosa*, the degree of tissue damage was checked using H&E and TUNEL staining after a total of two KB4528 injections. Lungs and spleens were excised for histological analysis. The excised tissues were fixed in neutral buffered formalin and then sectioned to a thickness of 4 µm using a paraffin molding process. The degree of tissue damage was observed using a light microscope through hematoxylin and eosin (H&E) staining.

As a result, lung damage (influx of immune cells, reduction in alveolar area), which is considered a major cause of death due to sepsis, was significantly reduced by the administration of KB4528, as shown in FIG. 23A. Liver damage did not show a significant difference between the vehicle control group and the KB4528 experimental group. Based on these results, it was confirmed that KB4528 was able to improve survival rates by preventing damage to the lungs, which are the most critical organ determining survival in sepsis.

In addition, since it is well known that the decline in immune activity and the decrease in lymphocyte counts, which are the key characteristics of sepsis, are mediated by immune cell death in the spleen, this was also confirmed. To evaluate the degree of cell death, terminal deoxynucleotidyl transferase dUTP nick end labeling (TUNEL) analysis was performed. Spleen paraffin sections were permeabilized with 100 mM sodium citrate solution at 70 °C for 30 minutes, followed by blocking with normal goat serum at room temperature for 30 minutes to prevent non-specific binding. Subsequently, TUNEL staining was performed using an *in situ* cell death detection kit. As a result, as shown in FIG. 23B, it was confirmed that while TUNEL-positive immune cells in the spleen significantly increased in a *Pseudomonas aeruginosa*-induced sepsis model, immune cell death in the spleen was significantly reduced upon administration of KB4528. Through this, it was confirmed that the administration of KB4528 inhibits immune cell death in septic individuals, and that this may be associated with survival rate.

### 5-3. Analysis of enhanced bactericidal activity of KB4528

In an animal model of sepsis induced by intraperitoneal injection of *Pseudomonas aeruginosa*, bacteria were collected from the peritoneal cavity 24 hours after administration of KB4528, and CFU were measured. Peritoneal lavage fluid collected from mice was serially diluted (1:100) with sterile buffer solution and plated onto LB agar plates. The plated plates were incubated at 37°C for 16 hours, and the bacterial load in the peritoneal cavity was quantified by counting the colony forming units (CFU) formed after incubation.

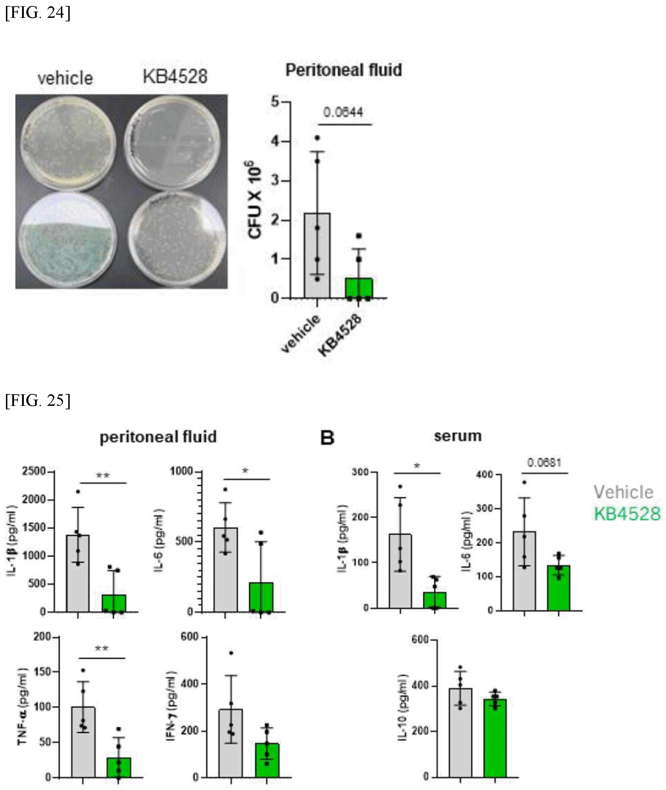

As a result, as shown in FIG. 24, the intraperitoneal bacteria in the experimental group administered KB4528 were effectively controlled compared to the control group, which was consistent with the result of increased survival rates following the injection of KB4528.

### 5-4. Analysis of modulatory effect of KB4528 on inflammatory cytokine production in sepsis animal model

The modulatory effect of KB4528 administration on inflammatory cytokine production in an animal model of sepsis induced by *Pseudomonas aeruginosa* infection was analyzed. Mice were sacrificed at 24 hours post-infection, and peritoneal lavage fluid was collected to evaluate the inflammatory response within the abdominal cavity, the site of infection. In addition, peripheral blood was collected and serum was isolated to confirm the systemic inflammatory response. Cytokine concentrations in the peritoneal lavage fluid and serum were measured using an enzyme-linked immunosorbent assay (ELISA). The concentrations of IL-1β, IL-6, TNF-α, IFN-γ, and IL-10 were quantitatively analyzed to compare local and systemic inflammatory responses.

As shown in FIG. 25, the administration of KB4528 significantly reduced the inflammatory cytokines IL-1β, IL-6, TNF-α, and IFN-γ within the peritoneal cavity, the site of infection in an animal model of sepsis caused by *Pseudomonas aeruginosa.* When examining cytokines in peripheral blood, which may confirm systemic inflammatory responses, the experimental group administered KB4528 showed a significant reduction in the inflammatory cytokines IL-1β and IL-6, but no difference was observed in the production of IL-10, known as an anti-inflammatory cytokine.

### 5-5. Analysis of mechanism of neutrophil reactive oxygen species (ROS) generation by KB4528

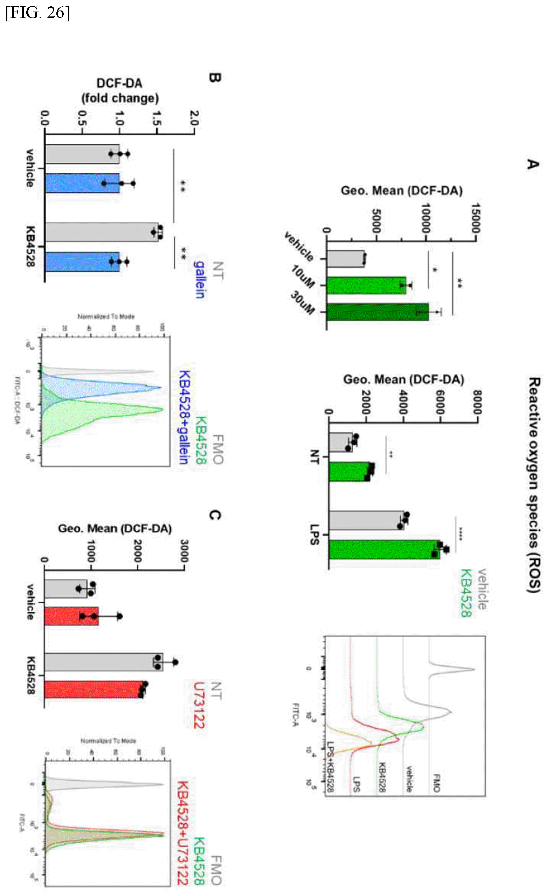

The effect of KB4528 on the generation of reactive oxygen species (ROS), which is important for the bactericidal activity of neutrophils, and the related mechanism of action were investigated. DCF-DA (5 µM), an ROS-sensing fluorescent dye, was added to neutrophils and reacted at 37°C for 30 minutes. After the reaction was completed, intracellular ROS generation levels were measured using flow cytometry, and the extent of ROS generation was quantitatively analyzed based on fluorescence intensity. As shown in FIG. 26A, ROS generation in neutrophils significantly increased upon treatment with KB4528, and this increase in ROS was similarly observed in neutrophils stimulated with LPS.

In addition, to investigate whether Gβγ is involved in ROS generation in neutrophils stimulated by KB4528, the Gβγ inhibitor gallein was used. As shown in FIG. 26B, it was confirmed that ROS generation induced by KB4528 was significantly inhibited by pretreatment with gallein. These results indicated that ROS generation in neutrophils induced by KB4528 is mediated through Gβγ.

To determine whether the activation of phospholipase C (PLC), a key signaling molecule downstream of GPCRs, affects ROS generation in neutrophils induced by KB4528, U-73122, a selective PLC inhibitor, was used. As a result, as shown in FIG. 26C, ROS generation in neutrophils induced by KB4528 was effectively inhibited by U-73122. Based on these results, it was confirmed that KB4528 may promote ROS generation in neutrophils through the Gβγ-PLC-mediated signaling pathway.

### 5-6. Analysis of modulatory effect of KB4528 on neutrophil NET formation

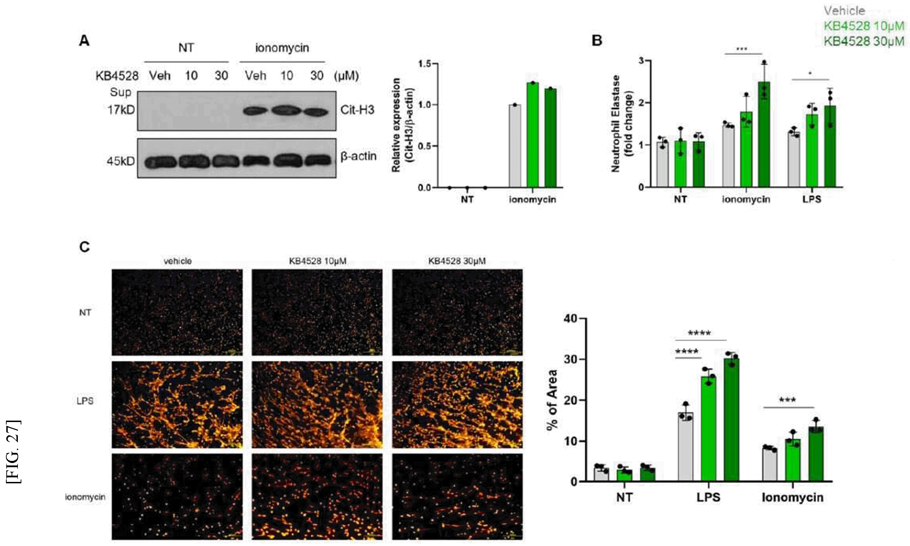

As another mechanism accompanying the increased bactericidal activity in a sepsis model induced by KB4528 administration, the modulatory effect on neutrophil extracellular trap (NET) formation in neutrophils was analyzed. Western blot analysis was performed to analyze the citrullination of histone 3, a key molecular marker for NET formation. Neutrophils isolated from mouse bone marrow were stimulated with KB4528 and ionomycin, and the cells were recovered to extract proteins, and the expression levels were analyzed using antibodies against citrullinated histone 3. As a result, it was confirmed that citrullination of histone 3 increased in neutrophils treated with ionomycin upon stimulation by KB4528, as shown in FIG. 27.

In addition, the activity of neutrophil elastase, which plays a crucial role in the NET formation process, was measured using an enzyme-linked immunosorbent assay (ELISA). Neutrophils were treated with KB4528 and then stimulated with ionomycin or phorbol 12-myristate 13-acetate (PMA), and the culture supernatant was recovered, and the level of neutrophil elastase activity was quantitatively analyzed. Neutrophil elastase activity induced by ionomycin and phorbol 12-myristate 13-acetate (PMA) stimulation was significantly increased by KB4528 treatment.

Furthermore, KB4528 stimulation strongly promoted LPS-induced NET formation, and it was confirmed that ionomycin stimulation-induced NET formation also significantly increased upon KB4528 treatment. These results suggest that KB4528 promotes NET formation by activating key mechanisms related to neutrophil NET formation, thereby effectively increasing pathogen clearance activity in sepsis models.

### 5-7. Analysis of neutrophil increase effect by KB4528 in sepsis animal model

To evaluate changes in neutrophils following the administration of KB4528 in an actual sepsis animal model, bone marrow cells were isolated 24 hours after *Pseudomonas aeruginosa* infection, and the expression levels of activation markers Ly6G, CD11b, Siglec-F, CD45, MHCII, NK1.1, CD4, CD3e, CD19, Ly6C, F4/80, CD11c, and CD8a were analyzed using fluorescently labeled antibodies. Subsequently, immune cell-specific antigens were analyzed using the spectral flow cytometry system, Cytek Aurora.

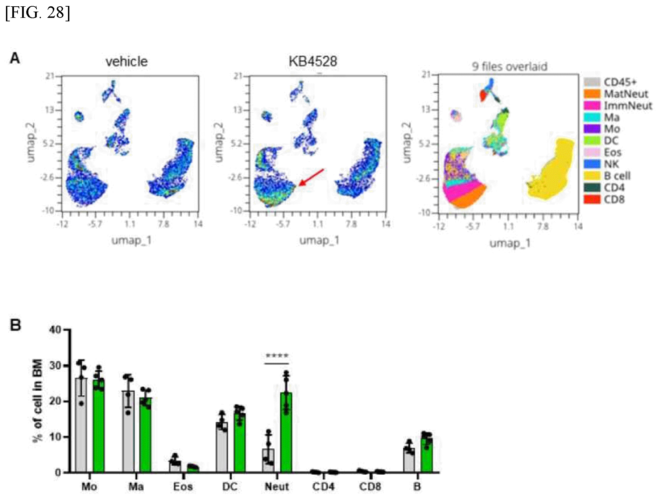

As a result of forming immune cell clusters through UMAP analysis on cells expressing various antigens, it was confirmed that the cluster corresponding to neutrophils significantly increased in the experimental group administered KB4528, as shown in FIG. 28.

Representative changes observed in neutrophils upon infection include an increase in emergency granulopoiesis, which is a response to infection and a key mechanism for pathological modulation, and an increase in immature neutrophils.

Bone marrow analysis 24 hours after *Pseudomonas aeruginosa* infection revealed that the experimental group administered KB4528 showed a significant increase in the proportion of neutrophils in both manual gating analysis and UMAP clustering analysis. In particular, it was confirmed that the increase in bone marrow neutrophils caused by KB4528 was not merely a numerical increase, but an increase in mature neutrophils.

### 5-8. Analysis of maturation increase effect of neutrophil during emergency granulopoiesis by KB4528

To analyze the effect of KB4528 on the increase in neutrophil maturation during emergency granulopoiesis, immunophenotyping analysis using flow cytometry was performed. After isolating bone marrow cells from a sepsis model, the expression levels of the activation markers c-Kit, Ly6G, CD11b, and CD101 were analyzed using fluorescently labeled antibodies.

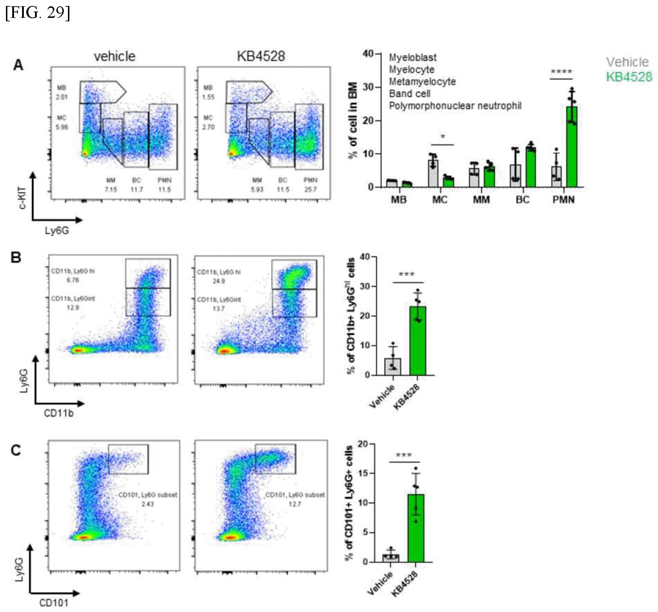

As shown in FIG. 29, when identifying neutrophil progenitor cells and cells in their developmental stages (myeloblast, myelocyte, metamyelocyte, band cell, PMN) that may be identified by c-Kit and Ly6G, administration of KB4528 induced a decrease in myelocytes, which are intermediate cells, along with a significant increase in PMNs, which are fully mature cells.

In addition, regarding the degree of neutrophil maturation confirmed by the expression levels of CD11b and Ly6G, cells with high expression levels of Ly6G, a neutrophil marker, increased, and the expression of CD101, which is expressed by fully mature neutrophils, also increased.

### 5-9. Analysis of modulatory effect on chemokine receptor expression of neutrophil by KB4528

To investigate the modulatory effect of KB4528 administration on neutrophil surface antigen expression in a sepsis animal model, immunophenotyping analysis using flow cytometry was performed. After isolating bone marrow neutrophils from the sepsis model, the expression levels of the activation markers CXCR2, CXCR4, and CD62L were analyzed using fluorescently labeled antibodies.

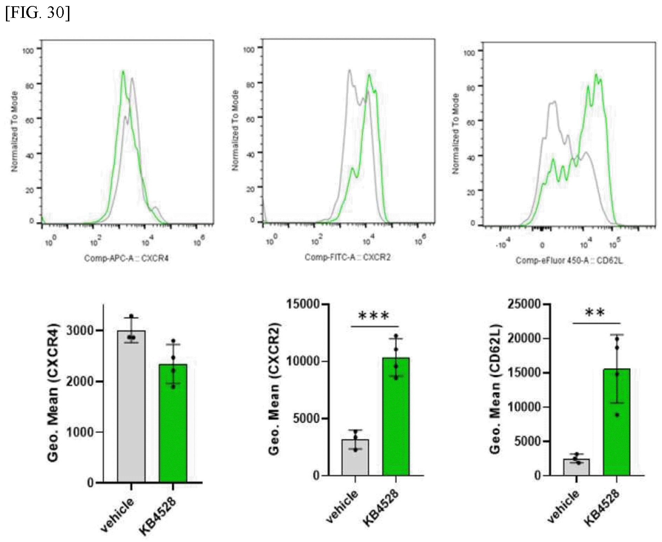

As a result, it was confirmed that KB4528 strongly increased the expression of CXCR2 in bone marrow neutrophils in the sepsis model while decreasing the expression of CXCR4, as shown in FIG. 30. This result implies that bone marrow neutrophils may migrate to peripheral blood, while confirming that the expression of CD62L was strongly increased by the administration of KB4528, it was observed that neutrophils were strongly activated.

### 5-10. Analysis of neutrophil autophagy activation by KB4528

Since it was confirmed that neutrophil maturation was enhanced and the number of mature neutrophils increased upon administration of KB4528 in a sepsis animal model, the effect of KB4528 on the activation of autophagy, which is important in the neutrophil maturation process, was investigated. Neutrophil autophagy activation was evaluated using the CYTO-ID^{®} autophagy detection kit. Neutrophils were stained with CYTO-ID^{®} reagent (1:500 dilution) added at 37°C for 30 minutes. Subsequently, the cells were fixed with 4% paraformaldehyde, and Hoechst dye was applied for 5 minutes for nuclear staining. Fluorescence signals were observed using a confocal microscope.

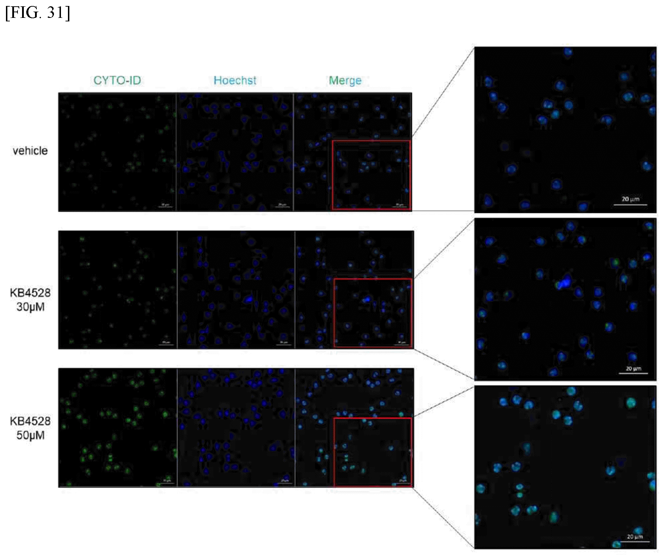

As shown in FIG. 31, an increase in CYTO-ID+ signals was observed upon stimulation by KB4528. As a result, it was confirmed that KB4528 activates the autophagy of neutrophils, and that KB4528 promotes neutrophil maturation, which is reduced in sepsis animal models, thereby increasing the number of mature neutrophils and demonstrating a therapeutic effect against sepsis, an infectious disease.

### <Example 6> Toxicity evaluation of KB4528

### 6-1. Analysis of body weight change

A single-dose toxicity test was conducted on KB4528 and two derivatives (KB4501, KB4529). The administration route was intraperitoneal, and 5- and 10-fold overdose doses were selected based on the dose (0.1 mg/kg) that showed excellent therapeutic effects in a mouse sepsis animal model. The dosage per individual was calculated as 5 ml/kg based on body weight after fasting (on the day of administration). It was administered once into the peritoneal cavity using a disposable syringe (1 ml). All animals were fasted for at least 4 hours prior to administration while being allowed free water, and were fed approximately 4 hours after administration.

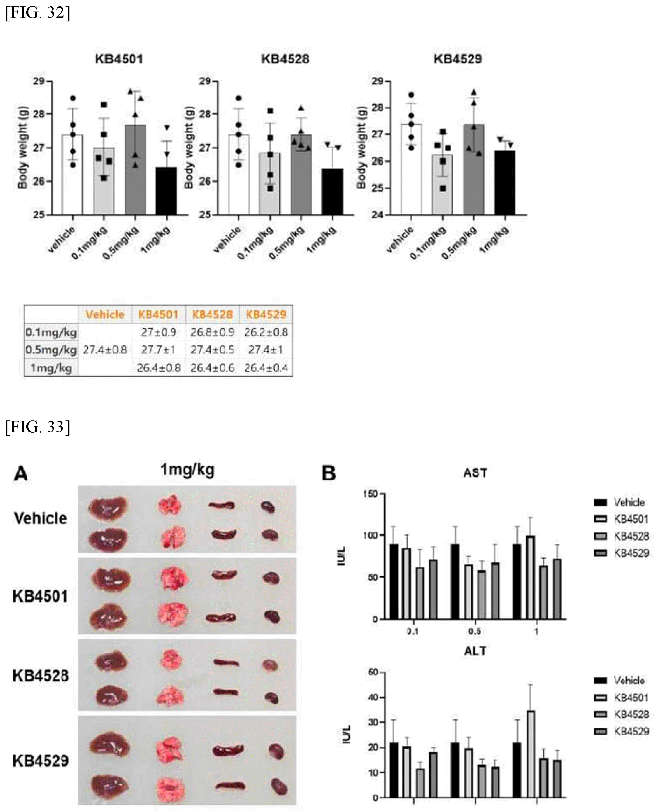

During the observation of general symptoms, motility, mortality, and the presence of injury were monitored for all animals. If abnormal symptoms were observed, the experimenter's opinion was taken into account during the experiment period. Regarding the observation of abnormal symptoms, on Day 1 of administration, symptoms were observed at 10 minutes, 1, 2, 4, and 6 hours, and thereafter, general symptoms were observed once daily until Day 14. In addition to the measurement times, observations were conducted across the entire range, including hair growth, hair loss, drooling, convulsions, drug-contaminated stool, and loose stool. As a result, as shown in FIG. 32, there were almost no changes in general symptoms observed visually with the administration of KB4528, KB4501, and KB4529 at different doses. No deaths occurred with the administration of KB4528, KB4501, and KB4529 at different doses.

Body weight was measured for all animals after the acclimatization period and before group separation, on the day of administration (before administration), and from after administration until necropsy. There was almost no change in body weight with the administration of KB4528 and KB4501 at different doses, but a slight decrease in body weight was observed with the 1 mg/kg of KB4529 among administration conditions at different doses, which was confirmed to be not significant.

### 6-2. Analysis of changes in major organs

Animals that survived during the experiment were euthanized after the experiment ended, and the major organs were visually inspected for abnormalities through necropsy. As a result of necropsy, no specific changes were observed in the different dosing groups of KB4501 and KB4529 compared to the control group, as shown in FIG. 33. For KB4528, no particular specificity was observed with the 0.1 mg/kg and 0.5 mg/kg administrations, but in the 1 mg/kg administration group, no specific changes were observed in the liver, lungs, or bone marrow compared to the control group, and changes in kidney color were observed visually in some mice. As a result of measuring AST and ALT activities, which may measure the degree of liver damage, there were no significant changes caused by KB4528 and KB4529, but when KB4501 was administered at 1 mg/kg, ALT increased, and from this result, it was confirmed that there is a possibility that KB4501 may induce liver toxicity.

Although specific parts of the present disclosure have been described in detail above, it is clear for those skilled in the art that these specific descriptions are merely preferred example embodiments and the scope of the present disclosure is not limited thereto. Accordingly, the substantial scope of the present invention will be defined by the appended claims and equivalents thereof.

## Claims

1. A pharmaceutical composition for preventing or treating sepsis comprising, as an active ingredient, a compound selected from a compound represented by Chemical Formula 1 below, a pharmaceutically acceptable salt thereof, a solvate thereof, or a stereoisomer thereof: 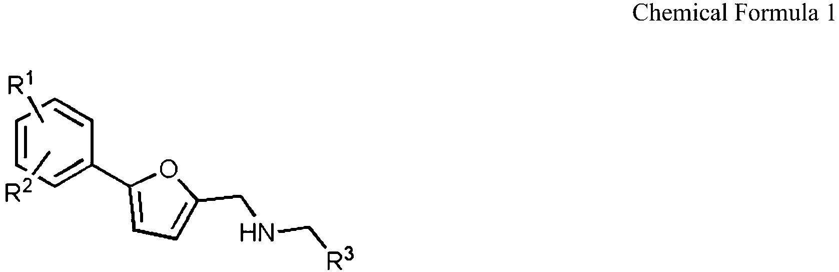 wherein, in Chemical Formula 1,
R¹ and R² are the same or different, and are each independently selected from the group consisting of hydrogen, hydroxy (OH), halogen, (C1~C4)alkyl, (C1~C4)alkoxy, acetyl, 1-hydroxyethyl (-CH(OH)CH₃), and trifluoromethyl (CF₃),
R³ is selected from the group consisting of 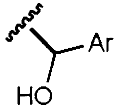 and substituted or unsubstituted phenyl, wherein the substituted phenyl is substituted with any one selected from the group consisting of halogen, hydroxy (OH), (C1~C4)alkyl, (C1~C4)alkoxy, and trifluoromethyl (CF₃), and
Ar is selected from substituted or unsubstituted phenyl, pyridinyl, 1,3-benzodioxol, or naphthyl, wherein the substituted phenyl is substituted with one or two substituents selected from the group consisting of halogen, (C1~C4)alkyl, (C1~C4)alkoxy, and trifluoromethyl.

2. The pharmaceutical composition of claim 1, wherein R¹ and R² are the same as or different from each other, and are each independently selected from the group consisting of hydrogen, fluoro (F), chloro (Cl), and methoxy, and
R³ is any one selected from the group consisting of

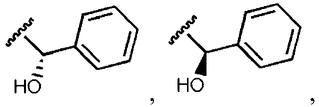

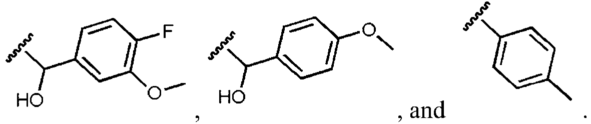

3. The pharmaceutical composition of claim 1, wherein the compound is selected from the group consisting of:
2-(((5-(4-methoxyphenyl)furan-2-yl)methyl)amino)-1-phenylethanol (Compound 2a);
4-(5-(((2-hydroxy-2-phenylethyl)amino)methyl)furan-2-yl)phenol (Compound 3a);
1-phenyl-2-(((5-(4-(trifluoromethyl)phenyl)furan-2-yl)methyl)amino)ethanol (Compound 4a);
2-(((5-(4-fluorophenyl)furan-2-yl)methyl)amino)-1-phenylethanol (Compound 5a);
1-phenyl-2-(((5-(p-tolyl)furan-2-yl)methyl)amino)ethanol (Compound 6a);
1-(4-(5-(((2-hydroxy-2-phenylethyl)amino)methyl)furan-2-yl)phenyl)ethenone (Compound 7a);
2-(((5-(4-(1-hydroxyethyl)phenyl)furan-2-yl)methyl)amino)-1-phenylethanol (Compound 7b);
2-(((5-(3-chlorophenyl)furan-2-yl)methyl)amino)-1-phenylethanol (Compound 8a);
3-(5-(((2-hydroxy-2-phenylethyl)amino)methyl)furan-2-yl)phenol (Compound 9a);
1-phenyl-2-(((5-(3-(trifluoromethyl)phenyl)furan-2-yl)methyl)amino)ethanol (Compound 10a);
2-(((5-(3-fluorophenyl)furan-2-yl)methyl)amino)-1-phenylethanol (Compound 11a);
1-(3-(5-(((2-hydroxy-2-phenylethyl)amino)methyl)furan-2-yl)phenyl)ethenone (Compound 12a);
2-(((5-(3-(1-hydroxyethyl)phenyl)furan-2-yl)methyl)amino)-1-phenylethanol (Compound 12b);
2-(((5-(3,4-difluorophenyl)furan-2-yl)methyl)amino)-1-phenylethanol (Compound 13a);
2-(((5-(3,4-dichlorophenyl)furan-2-yl)methyl)amino)-1-phenylethanol (Compound 14a);
2-(((5-(3,4-dimethoxyphenyl)furan-2-yl)methyl)amino)-1-phenylethanol (Compound 15a);
2-(((5-(3-fluoro-4-methoxyphenyl)furan-2-yl)methyl)amino)-1-phenylethanol (Compound 16a);
2-(((5-(4-methoxy-3-methylphenyl)furan-2-yl)methyl)amino)-1-phenylethanol (Compound 17a);
(R)-2-(((5-(3-chloro-4-methoxyphenyl)furan-2-yl)methyl)amino)-1-phenylethanol (Compound 27);
(S)-2-(((5-(3-chloro-4-methoxyphenyl)furan-2-yl)methyl)amino)-1-phenylethanol (Compound 28);
2-(((5-(3-chloro-4-methoxyphenyl)furan-2-yl)methyl)amino)-1-(4-methoxyphenyl)ethanol (Compound 29);
2-(((5-(3-chloro-4-methoxyphenyl)furan-2-yl)methyl)amino)-1-(pyridin-3-yl)ethanol (Compound 30);
2-(((5-(3-chloro-4-methoxyphenyl)furan-2-yl)methyl)amino)-1-(p-tolyl)ethanol (Compound 31);
2-(((5-(3-chloro-4-methoxyphenyl)furan-2-yl)methyl)amino)-1-(4-(trifluoromethyl)phenyl)ethanol (Compound 32);
2-(((5-(3-chloro-4-methoxyphenyl)furan-2-yl)methyl)amino)-1-(4-fluoro-3-methoxyphenyl)ethanol (Compound 33);
2-(((5-(3-chloro-4-methoxyphenyl)furan-2-yl)methyl)amino)-1-(3,4-dimethoxyphenyl)ethanol (Compound 34);
1-(benzo[d][1,3]dioxol-5-yl)-2-(((5-(3-chloro-4-methoxyphenyl)furan-2-yl)methyl)amino)ethanol (Compound 35);
2-(((5-(3-chloro-4-methoxyphenyl)furan-2-yl)methyl)amino)-1-(3,4-difluorophenyl)ethanol (Compound 36);
2-(((5-(3-chloro-4-methoxyphenyl)furan-2-yl)methyl)amino)-1-(naphthalen-2-yl)ethanol (Compound 37);
1-(5-(2-fluorophenyl)furan-2-yl)-N-(4-methylbenzyl)methanamine (Compound 41);
1-(5-(2-fluorophenyl)furan-2-yl)-N-(4-methylbenzyl)methanamine hydrochloride (Compound 41a);
1-(5-(4-chlorophenyl)furan-2-yl)-N-(4-methoxybenzyl)methanamine (Compound 42);
N-(3-chloro-4-methoxybenzyl)-1-(5-(4-chlorophenyl)furan-2-yl)methanamine (Compound 43);
1-(5-(3-chloro-4-methoxyphenyl)furan-2-yl)-N-(4-methoxybenzyl)methanamine (Compound 44); and
1-(5-(4-chloro-3-methoxyphenyl)furan-2-yl)-N-(4-methoxybenzyl)methanamine (Compound 45).

4. The pharmaceutical composition of claim 1, wherein the compound binds to an odorant receptor 164 (Olfr164).

5. The pharmaceutical composition of claim 1, wherein the sepsis is induced by infection with *Pseudomonas aeruginosa.*

6. The pharmaceutical composition of claim 1, wherein the composition modulates production of one or more inflammatory cytokines selected from the group consisting of IL-1β, IL-6, TNF-α, and IFN-γ.

7. A health functional food composition for preventing or improving sepsis comprising, as an active ingredient, a compound selected from a compound represented by Chemical Formula 1 below, a pharmaceutically acceptable salt thereof, a solvate thereof, or a stereoisomer thereof: 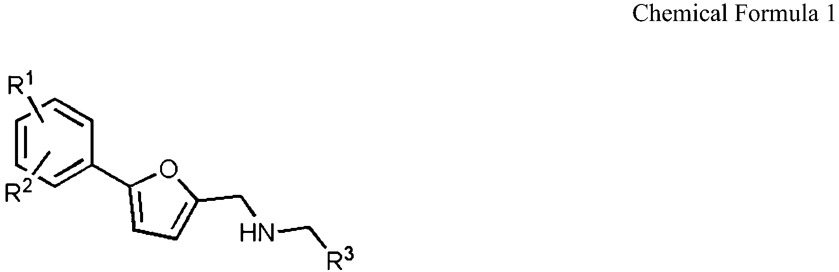 wherein, in Chemical Formula 1,
R¹ and R² are the same or different, and are each independently selected from the group consisting of hydrogen, hydroxy (OH), halogen, (C1~C4)alkyl, (C1~C4)alkoxy, acetyl, 1-hydroxyethyl (-CH(OH)CH₃), and trifluoromethyl (CF₃),
R³ is selected from the group consisting of 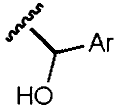
and substituted or unsubstituted phenyl, wherein the substituted phenyl is substituted with any one selected from the group consisting of halogen, hydroxy (OH), (C1~C4)alkyl, (C1~C4)alkoxy, and trifluoromethyl (CF₃), and
Ar is selected from substituted or unsubstituted phenyl, pyridinyl, 1,3-benzodioxol, or naphthyl, wherein the substituted phenyl is substituted with one or two substituents selected from the group consisting of halogen, (C1~C4)alkyl, (C1~C4)alkoxy, and trifluoromethyl.

8. The health functional food composition of claim 7, wherein the compound binds to an odorant receptor 164 (Olfr164).

9. The health functional food composition of claim 7, wherein the sepsis is induced by infection with *Pseudomonas aeruginosa*.

10. A reagent composition for modulating an odorant receptor Olfr164 comprising, as an active ingredient, a compound selected from a compound represented by Chemical Formula 1 below, a pharmaceutically acceptable salt thereof, a solvate thereof, or a stereoisomer thereof: 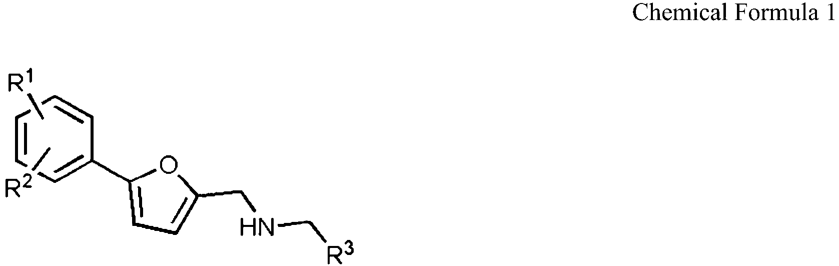 wherein, in Chemical Formula 1,
R¹ and R² are the same or different, and are each independently selected from the group consisting of hydrogen, hydroxy (OH), halogen, (C1~C4)alkyl, (C1~C4)alkoxy, acetyl, 1-hydroxyethyl (-CH(OH)CH₃), and trifluoromethyl (CF₃),
R³ is selected from the group consisting of 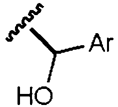
and substituted or unsubstituted phenyl, wherein the substituted phenyl is substituted with any one selected from the group consisting of halogen, hydroxy (OH), (C1~C4)alkyl, (C1~C4)alkoxy, and trifluoromethyl (CF₃), and
Ar is selected from substituted or unsubstituted phenyl, pyridinyl, 1,3-benzodioxol, or naphthyl, wherein the substituted phenyl is substituted with one or two substituents selected from the group consisting of halogen, (C1~C4)alkyl, (C1~C4)alkoxy, and trifluoromethyl.
